(19) Europäisches Patentamt
European Patent Office
Office européen des brevets

(11) EP 4 063 377 A1

(12) EUROPEAN PATENT APPLICATION
published in accordance with Art. 153(4) EPC

(43) Date of publication:
**28.09.2022 Bulletin 2022/39**

(21) Application number: **20889968.2**

(22) Date of filing: **19.11.2020**

(51) International Patent Classification (IPC):
***C07K 1/00*** (2006.01) ***C07K 7/00*** (2006.01)
***C40B 40/08*** (2006.01) ***C40B 40/10*** (2006.01)
***C12M 1/00*** (2006.01) ***C12N 15/11*** (2006.01)
***C12P 21/02*** (2006.01)

(52) Cooperative Patent Classification (CPC):
**C07K 1/00; C07K 7/00; C12M 1/00; C12N 15/11; C12P 21/02; C40B 40/08; C40B 40/10**

(86) International application number:
**PCT/JP2020/043289**

(87) International publication number:
**WO 2021/100833 (27.05.2021 Gazette 2021/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.11.2019 JP 2019209114**

(71) Applicant: **The University of Tokyo
Bunkyo-ku, Tokyo 113-8654 (JP)**

(72) Inventors:
- **SUGA, Hiroaki
Tokyo 113-8654 (JP)**
- **KATOH, Takayuki
Tokyo 113-8654 (JP)**
- **IWANE, Yoshihiko
Tokyo 113-8654 (JP)**

(74) Representative: **Mewburn Ellis LLP
Aurora Building
Counterslip
Bristol BS1 6BX (GB)**

# (54) MODIFICATION OF TRNA T-STEM FOR ENHANCING N-METHYL AMINO ACID INCORPORATION

(57) The problem to be solved by the present invention is to develop a novel peptide synthesizing method which enables the discovery of a highly N-methylated peptide having improved membrane permeability and improved stability against proteolysis and enables the simultaneous incorporation of a plurality of respectively different N-methyl amino acids. The present invention provides a tRNA having a T-stem in which $N_1N_2GGN_3$ and $N_4CCN_5U$ form a pair and encoding an N-methyl amino acid ($N_1$ is G or A, one of $N_2$ and $N_4$ is G and the other one is C, and $N_3$ and $N_4$ are arbitrary amino acids forming a pair).

EP 4 063 377 A1

[Fig.3]

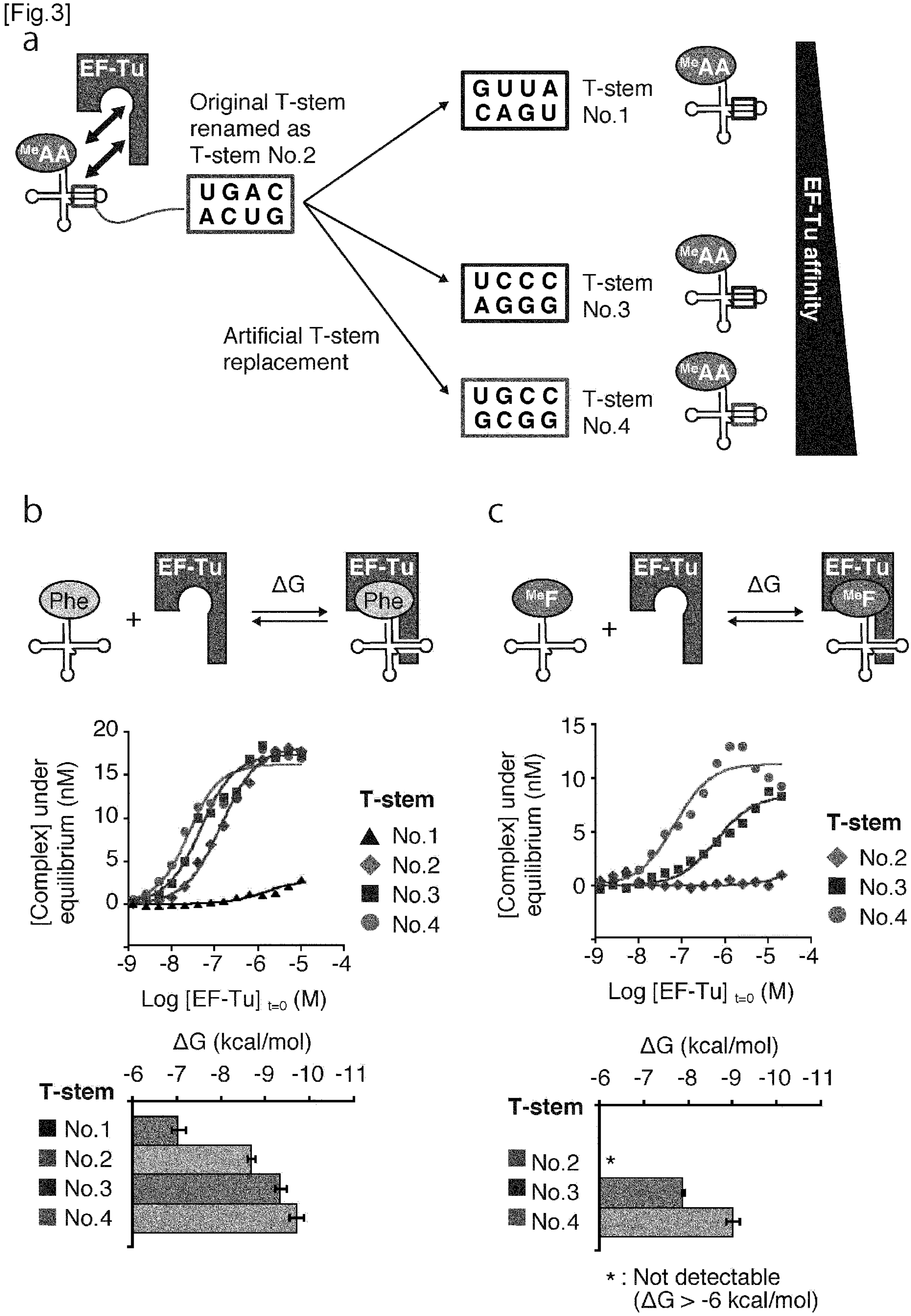
a
EF-Tu
MeAA
Original T-stem renamed as T-stem No.2
UGAC
ACUG
Artificial T-stem replacement
GUUA
CAGU
T-stem No.1
UCCC
AGGG
T-stem No.3
UGCC
GCGG
T-stem No.4
EF-Tu affinity
b
Phe
EF-Tu
ΔG
[Complex] under equilibrium (nM)
Log [EF-Tu] t=0 (M)
T-stem
No.1
No.2
No.3
No.4
ΔG (kcal/mol)
c
MeF
*
* : Not detectable (ΔG > -6 kcal/mol)

## Description

## Technical Field

The present invention relates to the modification of the T-stem of tRNA for enhancing the incorporation of an N-methyl amino acid.

## Background Art

Peptides as middle molecules have high affinity and selectivity for target proteins having large surface recognition properties (Non-Patent Documents 1 to 3), so that they have attracted great attention as a new drug scaffold. It has been revealed that among them, naturally-occurring physiologically active peptides often have a plurality of backbone N-methyl modifications and such N-methylation enhances membrane permeability and protease resistance on peptide molecules (Non-Patent Documents 1 to 11).

In order to find novel peptide-based drugs having such ideal drug pharmacokinetic properties, it is ideal to develop a non-standard peptide library containing abundant, multiple, and respectively-different N-methyl amino acids ($^{Me}$AA) and thereby further improve the drug pharmacokinetic properties.

However, the $^{Me}$AA is not the most suited substrate for a translation mechanism and this becomes a practical obstacle. It may cause early termination of the translation or incorporation of an inaccurate amino acid in place of a desired $^{Me}$AA and deteriorate the incorporation efficiency and accuracy of N-methylated peptide synthesis (Non-Patent Documents 12 to 14). Non-Patent Document 14 discloses that only 5 $^{Me}$AAs, out of 19 $^{Me}$AAs, show an efficient incorporation efficiency (>80% to a proteinogenic amino acid (pAA) controls); 6 $^{Me}$AAs are moderate (10 to 80%); and the remaining 8 $^{Me}$AAs are poor and undetectable (<10%).

Some measures for improving the incorporation efficiency of a single $^{Me}$AA in a model peptide are reported (Non-Patent Documents 12, 15, and 16), but it is still difficult to simultaneously incorporate a plurality of different $^{Me}$AAs in one peptide.

Citation List

Non-Patent Documents

Non-Patent Document 1: Passioura, T., Katoh, T., Goto, Y. & Suga, H. Selection-based discovery of druglike macrocyclic peptides. Annu. Rev. Biochem. 83, 727-752, doi:10.1146/annurev-biochem-060713-035456 (2014).

Non-Patent Document 2: Driggers, E. M., Hale, S. P., Lee, J. & Terrett, N. K. The exploration of macrocycles for drug discovery-an underexploited structural class. Nat. Rev. Drug Discov. 7, 608-624, doi:10.1038/nrd2590 (2008).

Non-Patent Document 3: Koehn, F. E. & Carter, G. T. The evolving role of natural products in drug discovery. Nat. Rev. Drug Discov. 4, 206-220, doi:10.1038/nrd1657 (2005).

Non-Patent Document 4: Handschumacher, R. E., Harding, M. W., Rice, J., Drugge, R. J. & Speicher, D. W. Cyclophilin: a specific cytosolic binding protein for cyclosporin A. Science 226, 544-547 (1984).

Non-Patent Document 5: Takahashi, N., Hayano, T. & Suzuki, M. Peptidyl-Prolyl Cis-Trans Isomerase Is the Cyclosporin-a-Binding Protein Cyclophilin. Nature 337, 473-475, doi:DOI 10.1038/337473a0 (1989).

Non-Patent Document 6: Walsh, C. T., Zydowsky, L. D. & McKeon, F. D. Cyclosporin A, the cyclophilin class of peptidylprolyl isomerases, and blockade of T cell signal transduction. J. Biol. Chem. 267, 13115-13118 (1992).

Non-Patent Document 7: Altschuh, D., Vix, O., Rees, B. & Thierry, J. C. A conformation of cyclosporin A in aqueous environment revealed by the X-ray structure of a cyclosporin-Fab complex. Science 256, 92-94 (1992).

Non-Patent Document 8: Conradi, R. A., Hilgers, A. R., Ho, N. F. & Burton, P. S. The influence of peptide structure on transport across Caco-2 cells. II. Peptide bond modification which results in improved permeability. Pharm. Res. 9, 435-439 (1992).

Non-Patent Document 9: Haviv, F. et al. Effect of N-methyl substitution of the peptide bonds in luteinizing hormone-releasing hormone agonists. J. Med. Chem. 36, 363-369 (1993).

Non-Patent Document 10: Chikhale, E. G., Ng, K. Y., Burton, P. S. & Borchardt, R. T. Hydrogen bonding potential as a determinant of the in vitro and in situ blood-brain barrier permeability of peptides. Pharm. Res. 11, 412-419 (1994).

Non-Patent Document 11: Miller, S. M. et al. Comparison of the Proteolytic Susceptibilities of Homologous L-Amino-Acid, D-Amino-Acid, and N-Substituted Glycine Peptide and Peptoid Oligomers. Drug Dev. Res. 35, 20-32, doi:DOI 10.1002/ddr.430350105 (1995).

Non-Patent Document 12: Roberts, R. W. & Szostak, J. W. RNA-peptide fusions for the in vitro selection of peptides

and proteins. Proceedings of the National Academy of Sciences of the United States of America 94, 12297-12302 (1997).

Non-Patent Document 13: Murakami, H., Ohta, A., Ashigai, H. & Suga, H. A highly flexible tRNA acylation method for non-natural polypeptide synthesis. Nat. Methods 3, 357-359, doi:10.1038/nmeth877 (2006).

Non-Patent Document 14: Ohta, A., Murakami, H., Higashimura, E. & Suga, H. Synthesis of polyester by means of genetic code reprogramming. Chem. Biol. 14, 1315-1322, doi:10.1016/j.chembiol.2007.10.015 (2007).

Non-Patent Document 15: Goto, Y. et al. Reprogramming the translation initiation for the synthesis of physiologically stable cyclic peptides. ACS chemical biology 3, 120-129, doi:10.1021/cb700233t (2008).

Non-Patent Document 16: Goto, Y., Murakami, H. & Suga, H. Initiating translation with D-amino acids. RNA 14, 1390-1398, doi:10.1261/rna.1020708 (2008).

## Summary

### Technical Problem

The technical problem to be dissolved by the present invention is to develop a novel peptide synthesis method that enables the discovery of a highly N-methylated peptide having improved membrane permeability and improved stability to proteolysis and the simultaneous incorporation of a plurality of different N-methyl amino acids.

### Solution to Problem

As a result of keen researches, the present inventors have found that the aforesaid technical problem can be dissolved by paying attention to the T-stem of an aminoacyl-tRNA and completed the present invention.

The present invention is as follows.

(1) A tRNA having a T-stem in which $N_1N_2GGN_3$ and $N_4CCN_5U$ form a pair and encoding an N-methyl amino acid. ($N_1$ is G or A; one of $N_2$ and $N_5$ is G and the other one is C; and $N_3$ and $N_4$ are any amino acids forming a pair).

(2) A translation system containing the tRNA as described in (1) and an EF-Tu protein.

(3) A method of producing a peptide library, including a step of translating with a cell-free translation system by using the tRNA as described in (1) or the translation system as described in (2).

(4) A method of producing a complex library of a peptide and an mRNA encoding the peptide, including a step of translating with a cell-free translation system by using the tRNA as described in (1) or the translation system as described in (3).

(5) The production method as described in (3) or (4), including a step of preparing the tRNA as described in (1) or the translation system as described in (2), wherein in the preparation step, which tRNA is charged, a tRNA having a T-stem in which $N_1$ $N_2GGN_3$ and $N_4CCN_5U$ form a pair or a tRNA other than the tRNA having a T-stem in which $N_1N_2GGN_3$ and $N_4CCN_5U$ form a pair is determined prior to charging with an N-methyl amino acid.

(6) The production method as described in (5) including a step of preparing the tRNA described in (1) or the translation system described in (2), wherein in the preparation step, charging of a tRNA having a T-stem in which $AGGGN_3$ and $N_4CCCU$ form a pair and a tRNA having a T-stem in which $GCGGN_3$ and $N_4CCGU$ form a pair, or a tRNA other than the said tRNAs is determined prior to charging with an N-methyl amino acid.

(7) The production method described in (5) further including a step of preparing the tRNA as described in (1) or the translation system as described in (2), wherein in the preparation step, charging of a tRNA having $AGGGG(N_4)_mC$-CCCU (SEQ ID NO: 1) and a tRNA having $GCGGG(N_4)_mCCCGU$ (SEQ ID NO: 2), or a tRNA other than the said tRNAs is determined prior to charging with an N-methyl amino acid.

(8) The production method as described in any of (3) to (8), wherein a tRNA having, as an affinity between a tRNA charged with an N-methyl amino acid and EF-Tu, $\Delta G$ (kcal/mol) of -7.5 to -10 is selected and the tRNA thus selected is charged with the N-methyl amino acid.

(9) A peptide library produced by the method as described in any of (3) and (5) to (8) or a peptide-mRNA complex library produced by the method as described in any of (4) to (8).

(10) The peptide library or peptide-mRNA complex library as described in (9), including a peptide containing two or more N-methyl amino acids in one peptide structure.

### Advantageous Effects of Invention

The present invention makes it possible to provide a novel peptide synthesis method that enables the discovery of a highly N-methylated peptide and the simultaneous incorporation of a plurality of different N-methyl amino acids.

## Brief Description of Drawings

Fig. 1 is a schematic view showing the interaction between EF-Tu and aminoacyl-tRNA. Fig. 1a shows the EF-Tu-mediated delivery, to a ribosome, of protein-constituting amino acid (proteinogenic amino acid: pAA)-tRNA. Fig. 1b schematically shows a hypothetical hindrance to the N-methyl amino acid ($^{Me}$AA)-tRNA interaction. It means insufficient EF-Tu affinity under competition with pAA-tRNA. Fig. 1c is a schematic view of the concept in the present invention. Fig. 1d shows used N-methyl amino acids, and they are as follows: $^{Me}$G: N-methylglycine, $^{Me}$S: N-methylserine, $^{Me}$A: N-methylalanine, $^{Me}$F: N-methylphenylalanine, $^{Me}$L: N-methylleucine, $^{Me}$M: N-methylmethionine, $^{Me}$T: N-methylthreonine, $^{Me}$Y: N-methyltyrosine, $^{Me}$D: N-methylaspartic acid, $^{Me}$V: N-methylvaline, $^{Me}$Nl: N-methylnorleucine, $^{Me}$Nv:N-methylnorvaline, $^{Me}$Ym: N-methyl-p-methoxyphenylalanine, and $^{Ac}$K: ε-N-acetyllysine.

Fig. 2 shows the correlation between weak EF-Tu affinity of $^{Me}$AA-tRNA and inaccurate synthesis of $^{Me}$AA-containing peptides. Fig. 2a shows the EF-Tu affinity of a standard pAA-tRNA. The error bars represent the standard deviation. Fig. 2b shows weak EF-Tu affinity of many $^{Me}$AA-tRNA$^{AsnE2}_{GAC}$s and $^{Ac}$K-tRNA$^{AsnE2}_{GAC}$. Fig. 2c shows a cause of the weak EF-Tu affinity of $^{Me}$AA-tRNA. This drawing was made from the co-crystal structure (PDB ID:1TTT) of EF-Tu and Phe-tRNA$^{Phe}$ (Reference Literature 1). Fig. 2d shows the results of the translation accuracy of peptides containing $^{Me}$AA or $^{Ac}$K. In each experiment, mRNA12 containing a GUC codon was translated using a FIT system containing a npAA-tRNA$^{AsnE2}_{GAC}$ of interest. The † peak corresponds to a byproduct containing Ile in place of a nonproteinogenic amino acid (non-proteinogenic amino acid: npAA).

Fig. 3 shows the reinforcement of EF-Tu affinity of an aminoacyl-tRNA by a T-stem substitution strategy. Fig. 3a shows a strategy for reinforcing the weak EF-Tu affinity of $^{Me}$AA-tRNA. The T-stem sequence of $^{Me}$AA-tRNA was replaced by three different sequences having respectively-different EF-Tu affinities. The original T-stem was renamed as T-stem No. 2 and the newly prepared T-stems were named as T-stems Nos. 1, 3, and 4. Their EF-Tu affinities are supposed to increase with an increase in the number. Figs. 3b and 3c show the quantification of the EF-Tu affinity of Phe-tRNA$^{AsnE2}_{GAC}$ (b) and $^{Me}$F-tRNA$^{AsnE2}_{GAC}$ (c) having different T-stem sequences, respectively. The asterisk shows that the affinity is at a not-detectable level ($\Delta G > -6$ kcal/mol). The error bars represent the standard deviation. In Fig. 3c, an uncharged tRNA was used as a control.

Fig. 4 shows that the translation accuracy and efficiency are improved by selecting an appropriate T-stem. Figs. 4a and 4b show the MALDI-TOF-MS of peptide products after a translation reaction for 15 minutes. The GUC codon was translated by Phe-tRNA$^{AsnE2}_{GAC}$ (a) and $^{Me}$F-tRNA$^{AsnE2}_{GAC}$ (b) having different T-stem sequences, respectively. The † peak corresponds to byproducts containing Ile in place of Phe (a) and $^{Me}$F (b), respectively. Figs. 4c and 4d respectively show time course of the peptide expression levels in the FIT systems containing Phe-tRNA$^{AsnE2}_{GAC}$ (c) and $^{Me}$F-tRNA$^{AsnE2}_{GAC}$ (d).

Fig. 5 shows accurate and efficient synthesis of model peptides containing 6 respectively-different $^{Me}$AAs under the optimum conditions. Fig. 5a shows the outline of EF-Tu affinity tuning. Fig. 5b shows the adjustment of the concentration of each of $^{Me}$AA-tRNAs based on the flexizyme-catalyzed acylation efficiency. Fig. 5c shows the respective sequences of mRNA13 and its resultant P13 peptide. The arrow shows the relation between sequences. Fig. 5d shows a reprogrammed gene encoding 15 pAAs and 6 $^{Me}$AAs. Figs. 5e and 5f show a comparison between the conventional system and the optimized system based on the analysis of a peptide product by tricine-SDS-PAGE (e) and MALDI-TOF-MS (f).

Fig. 6 shows the expression of a highly N-methylated peptide containing both 15 pAAs and 9 respectively-different $^{Me}$AAs. Fig. 6a shows the respective sequences of mRNA14 and its resultant P14 peptide. The CUC, UUG, GUC, CUG, AUC, GCG, UUC, GUG, and GCC codons were translated by $^{Me}$S-tRNA$^{AsnE2TS5}_{GAG}$, $^{Me}$G-tRNA$^{AsnE2TS5}_{CAA}$, $^{Me}$V-tRNA$^{AsnE2TS1}_{GAC}$, $^{Me}$Nl-tRNA$^{AsnE2TS1}_{CAG}$, $^{Me}$Y-tRNA$^{AsnE2TS3}_{GAU}$, $^{Me}$A-tRNA$^{AsnE2TS3}_{CGC}$, $^{Me}$Ym-tRNA$^{AsnE2TS1}_{GAA}$, $^{Me}$Nv-tRNA$^{AsnE2TS3}_{CAC}$, and $^{Me}$F-tRNA$^{AsnE2TS3}_{GGC}$, respectively. Fig. 6b shows a reprogrammed gene encoding 9 $^{Me}$AAs and 15 pAAs. Fig. 6c shows the outline of EF-Tu affinity tuning. Fig. 6d and Fig. 6e show the expression of P14 peptides containing 9 different $^{Me}$AAs. The mRNA was translated by either a FIT system containing 20 pAAs (as a control) or a FIT system containing 15 pAAs (except Phe, Leu, Ile, Val, and Ala) and 9 $^{Me}$AA-tRNAs. The peptide products were analyzed by MALDI-TOF-MS (d) and tricine-SDS-PAGE (e).

Fig. 7 shows the confirmation results of the byproduct in Fig. 2d.

Fig. 8 shows the results of a control experiment for confirming that an uncharged tRNA does not bind to EF-Tu.

Fig. 9 shows the translation accuracy improved by reinforcing the EF-Tu affinity of $^{Me}$AA-tRNA and $^{Ac}$K-tRNA. Fig. 9a shows the respective sequences of mRNA12 and its resultant P12-npAA peptide. The GUC codon was translated by the intended npAA-tRNA$^{AsnE2}_{GAC}$. Figs. 9b to 9f are the results of studying the effect of $^{Me}$G-tRNA$^{AsnE2}_{GAC}$ (b), $^{Me}$S-tRNA$^{AsnE2}_{GAC}$ (c), $^{Me}$A-tRNA$^{AsnE2}_{GAC}$ (d), $^{Me}$L-tRNA$^{AsnE2}_{GAC}$ (e), and $^{Ac}$K-tRNA$^{AsnE2}_{GAC}$ (f) having respectively-different T-stem sequences on EF-Tu affinity and translation accuracy. The asterisk shows that the affinity is at a not-detectable level ($\Delta G > -6$ kcal/mol). The error bars represent the standard deviation. The † peak corresponds

to a byproduct containing Ile in place of npAA.

Fig. 10 shows the reinforcement of the EF-Tu affinity for other $^{Me}$AA-tRNAs.

Fig. 10a to Fig. 10h respectively show the EF-Tu affinity of $^{Me}$M-tRNA$^{AsnE2}_{GAC}$ (a), $^{Me}$T-tRNA$^{AsnE2}_{GAC}$ (b), $^{Me}$Y-tRNA$^{AsnE2}_{GAC}$ (c), $^{Me}$D-tRNA$^{AsnE2}_{GAC}$ (d), $^{Me}$V-tRNA$^{AsnE2}_{GAC}$ (e), $^{Me}$Nv-tRNA$^{AsnE2}_{GAC}$ (f), $^{Me}$Nl-tRNA$^{AsnE2}_{GAC}$ (g), and $^{Me}$Ym-tRNA$^{AsnE2}_{GAC}$ (h) containing different T-stem sequences. The asterisk shows that the affinity is at a not-detectable level ($\Delta$G > -6 kcal/mol). The error bars represent the standard deviation.

**Description of Embodiments**

The tRNA of the present invention is a tRNA having a T-stem in which $N_1N_2GGN_3$ and $N_4CCN_5U$ form a pair and encoding an N-methyl amino acid.

In the $N_1N_2GGN_3$ and $N_4CCN_5U$ forming the T-stem, $N_1$ is G or A; one of $N_2$ and $N_5$ is G and the other one is C; and $N_3$ and $N_4$ are any amino acids forming a pair.

The present inventors assumed that the inefficient incorporation of an N-methyl amino acid occurs because of the following reasons. A ribosome incorporates an aminoacyl-tRNA, which is a tRNA charged with an amino acid, into a ribosome A site by the catalytic action of EF-Tu (Elongation Factor Thermo unstable) (Fig. 1a). The EF-Tu binds to the aminoacyl-tRNA at two different sites, that is, an amino acid site and a T-stem site. When only the interaction of the former one at the amino acid site is considered, the binding affinity differs largely depending on the kind of the amino acid. In order to complement the difference in affinity, the T-stem structure has evolved in nature so that the EF-Tu and each of 20 aminoacyl-tRNAs have equivalent affinities. Due to these equivalent affinities, EF-Tu can deliver all the 20 proteinogenic aminoacyl-tRNAs (pAA-tRNAs) to a ribosome at a similar efficiency (Fig. 1b). In contrast, if the affinity between EF-Tu and N-methylaminoacyl-tRNA ($^{Me}$AA-tRNA) is weak, the $^{Me}$AA-tRNA cannot bind to EF-Tu under competition with the pAA-tRNA, leading to a reduction in synthesis efficiency of a $^{Me}$AA-containing peptide. The present inventors have thought that if this assumption is an actual cause for the observed inefficiency, the tuning of EF-Tu affinity of $^{Me}$AA-tRNA may improve the translation efficiency and various $^{Me}$AAs can be incorporated in one peptide simultaneously.

In the present invention, it was experimentally confirmed that most of the $^{Me}$AA-tRNAs had affinity smaller than the average affinity of pAA-tRNAs and therefore, a thought that the $^{Me}$AA-tRNA cannot bind to EF-Tu with sufficient intensity was supported (Fig. 1c). Next, a method of tuning the weak EF-Tu affinity of $^{Me}$AA-tRNAs to the average affinity of pAA-tRNAs was developed (Fig. 1c). The optimized FIT system thus obtained showed improvement in the synthesis efficiency and accuracy of N-methylated peptides. Based on the aforesaid results, the new method of the present invention enables the synthesis of a library of highly N-methylated peptides and is expected to promote the future discovery of practical and non-standard peptide drugs having improved pharmacokinetic properties.

The other structures in the tRNA of the present invention, that is, acceptor stem, anticodon stem, anticodon loop, variable loop and D-arm may have any base sequence. Of these, the anticodon loop should have a base sequence corresponding to a codon to which an N-methyl amino acid is assigned.

The tRNA has a CCA sequence at the 3' end thereof and binds to an arbitrary amino acid. The tRNA of the present invention binds to an N-methyl amino acid.

The term "tRNA encodes an N-methyl amino acid" as used herein means that the tRNA binds to the N-methyl amino acid at the CCA-end of the tRNA.

In the T-stem in the tRNA of the present invention, any base may be selected as $N_1$ to $N_5$ insofar as $N_1N_2GGN_3$ and $N_4CCN_5U$ form a base pair, but particularly in the present invention, $N_1$ is G or A, one of $N_2$ and $N_5$ is G and the other one is C, and $N_3$ and $N_4$ are any amino acids forming a pair.

The term "one of $N_2$ and $N_5$ is G and the other one is C" as used herein means that when $N_2$ is G, $N_5$ is C and when $N_2$ is C, $N_5$ is G.

The term "$N_3$ and $N_4$ are any amino acids forming a pair" means that $N_3$ and $N_4$ may independently be any base and $N_3$ and $N_4$ are required to be a base pair.

The $N_1N_2GGN_3$ and $N_4CCN_5U$ forming a T-stem are preferably $AN_2GGN_3$ and $N_4CCN_5U$ or $GN_2GGN_3$ and $N_4CCN_5U$, more preferably $AGGGN_3$ and $N_4CCCU$ or $GCGGN_3$ and $N_4CCGU$, respectively.

The T-stem forms base pairs to form a T-loop and a T-arm.

The $N_3$ and $N_4$ are a base pair and a preferred embodiment of the T-stem in the present invention, that is, a base pair on the T-stem on which AGGG forms a base pair with CCCU and GCGG forms a base pair with CCGU, is shown as Fig. 3a.

The base sequence of the T-loop is not particularly limited insofar as it forms a loop and examples include the base sequence represented by SEQ ID NO: 3. UUCGAAU (SEQ ID NO: 3)

The base sequence represented by SEQ ID NO: 3 forms a T-loop in tRNA and binds to $N_1N_2GGN_3$ and $N_4CCN_5U$ forming a T-stem.

When the base sequence represented by SEQ ID NO: 3 binds to the T-stem, it may bind thereto via another base.

In the base sequence represented by SEQ ID NO: 3, one or more than one bases may be substituted, deleted, or inserted.

The term "more than one bases may be substituted" means that in the 7 bases forming the T-loop of the base sequence of SEQ ID NO: 1, 2, 3, 4, 5, 6, or 7 bases may be substituted. In the 7 bases forming the T-loop of the base sequence of SEQ ID NO: 1, 1 to 4 bases may be substituted, 1 to 3 bases may be substituted, 1 to 2 bases may be substituted, or 1 base may be substituted.

The term "more than one bases may be deleted" means that in the 7 bases forming the T-loop of the base sequence of SEQ ID NO: 1, 2, 3, 4, 5, 6, or 7 bases may be deleted. In the 7 bases forming the T-loop of the base sequence of SEQ ID NO: 1, 1 to 4 bases may be deleted, 1 to 3 bases may be deleted, 1 to 2 bases may be deleted, or 1 base may be deleted.

The term "more than one bases may be inserted" means that in the 7 bases forming the T-loop of the base sequence of SEQ ID NO: 1, 1 to 4 bases may be inserted, 1 to 3 bases may be inserted, 1 to 2 bases may be inserted, or 1 base may be inserted.

The base sequence represented by SEQ ID NO: 3 may be a base sequence having 70% or more, 80% or more, 85% or more, 90% or more, or 95% or more homology with this base sequence.

The tRNA of the present invention has a T-stem in which $N_1N_2GGN_3$ and $N_4CCN_5U$ form a pair and preferably, it further has a T-loop having a base sequence represented by SEQ ID NO: 3. It is to be noted that in the base sequence represented by SEQ ID NO: 3, one or more than one bases may be substituted, deleted, or inserted and it may be a base sequence having 70% or more, 80% or more, 85% or more, 90% or more, or 95% or more homology with this base sequence.

The tRNA of the present invention preferably has a T-arm having a base sequence represented by SEQ ID NO: 4, more preferably has a T-arm having a base sequence represented by SEQ ID NO: 1 or SEQ ID NO: 2, still more preferably has a T-arm having a base sequence represented by SEQ ID NO: 5 or SEQ ID NO: 6.

$N_1N_2GGN_3(N_6)_mN_4CCN_5U$ (SEQ ID NO: 4)
AGGGG(N6)mCCCCU (SEQ ID NO: 1)
GCGGG(N6)mCCCGU (SEQ ID NO: 2)
AGGGGUUCGAAUCCCCU (SEQ ID NO: 5)
GCGGGUUCGAAUCCCGU (SEQ ID NO: 6)

In SEQ ID NOS: 1, 2, and 4, $N_1$ to $N_5$ have the same meanings as described above, $N_6$ is an arbitrary base, and m is an integer of 1 or more. It is to be noted that $(N_6)_m$ is preferably a base sequence constituting a T-loop.

In SEQ ID NO: 4, $N_1N_2GGN_3$ and $N_4CCN_5U$ may be the aforesaid preferable base sequences, respectively.

In the base sequence represented by SEQ ID NOS: 1, 2, and 4 to 6, one or more than one bases may be substituted, deleted, or inserted. Preferably, in the base sequence constituting the T-loop, one or more than one bases may be substituted, deleted, or inserted.

The term "in the base sequence represented by SEQ ID NOS: 1, 2, and 4 to 6, one or more than one bases may be substituted" means that in the base sequence forming the T-arm and T-loop having the base sequence represented by SEQ ID NOS: 1, 2, and 4 to 6, 1, 2, 3, 4, 5, 6, 7, 8, 9, or 10 bases may be deleted, 1 to 10, 1 to 9, 1 to 8, 1 to 7, 1 to 6, 1 to 5, 1 to 4, 1 to 3, or 1 to 2 bases may be substituted, or 1 base may be substituted.

The term "in the base sequence represented by SEQ ID NOS: 1, 2, and 4 to 6, one or more than one bases may be deleted" means that in the bases forming the T-arm and T-loop of the base sequence represented by SEQ ID NOS: 1, 2, and 4 to 6, 1, 2, 3, 4, 5, 6, 7, 8, 9, or 10 bases may be deleted, 1 to 10, 1 to 9, 1 to 8, 1 to 7, 1 to 6, 1 to 5, 1 to 4, 1 to 3, or 1 to 2 bases may be deleted, or 1 base may be deleted.

The term "in the base sequence represented by SEQ ID NOS: 1, 2, and 4 to 6, one or more than one bases may be inserted" means that in the bases forming the T-arm and T-loop of the base sequence represented by SEQ ID NOS: 1, 2, and 4 to 6, 1, 2, 3, 4, 5, 6, 7, 8, 9, or 10 bases may be inserted, 1 to 10, 1 to 9, 1 to 8, 1 to 7, 1 to 6, 1 to 5, 1 to 4, 1 to 3, or 1 to 2 bases may be inserted, or 1 base may be inserted.

However, when one or more than one bases are substituted, deleted, or inserted in the base sequence represented by SEQ ID NOS: 1, 2, and 4 to 6, it is preferred that no base is substituted, deleted, or inserted in the base sequence $N_1N_2GGN_3$ and $N_4CCN_3U$ of the T-arm. In other words, the base sequence of the T-loop portion preferably has substitution, deletion, or insertion.

In SEQ ID NOS: 1, 2, and 4, m is not particularly limited insofar as $(N_6)_m$ can form a T-loop and it may be an integer of 2, 3, 4, 5, 6, 7, 8, 9, or 10, preferably an integer of 5 to 9, more preferably an integer of 6 to 8. It is still more preferred that the $(N_6)_m$ is a base sequence represented by SEQ ID NO: 3. The $(N_6)_m$ may be a sequence containing the base sequence represented by SEQ ID NO: 3. For example, the $(N_6)_m$ may be $(N_7)_{m1}UUCGAAU(N_8)_{m2}$, in which m1 and m2 may be independently an integer of 0 to 5 or may be an integer of 0 to 4, 0 to 3, 0 to 2, 0 to 1, 1, or 0. The $N_7$ and $N_8$ may be any base insofar as they do not hinder the T-loop structure formed by the base sequence represented

by SEQ ID NO: 3 and may form a T-loop with the base sequence represented by SEQ ID NO: 3. The base sequence represented by SEQ ID NO: 3 may form a T-loop, independently of $(N_7)_{m1}$ and $(N_8)_{m2}$, or with some bases.

In the tRNA used in the present invention, the base sequence other than that of the T-arm may be derived from a wild type tRNA, may be derived from an Escherichia coli-derived wild type tRNA, or may be an artificial tRNA prepared by in vitro transcription.

The tRNA in the present invention may not have a D-arm which $tRNA^{Pro1E1}$ or $tRNA^{Pro1E2}$ has. Specifically, the tRNA in the present invention may have a D-arm having a base sequence different from the base sequence of the D-arm of a tRNA known as $tRNA^{Pro1E2(CGG)}$, $tRNA^{Pro1E2(GAU)}$, $tRNA^{Pro1E2(GGU)}$, or $tRNA^{Pro1E2(GUG)}$. More specifically, it is presumed that the tRNA of the present invention does not have a D-stem in which GCGC and CGCG form a pair. Generally, it may not have a D-arm in which RNAs include a base sequence represented by SEQ ID NO: 134. $N_9N_{10}GCN_{11}N_{12}N_{13}N_{14}N_{15}N_{16}N_{17}N_{18}N_{19}GCN_{20}N_{21}$ (SEQ ID NO: 134) (in SEQ ID NO: 134, $N_9$ to $N_{21}$ are each an arbitrary base, $N_{11}$ to $N_{19}$ form a D-loop, and $N_9N_{10}GC$ forms a base pair with $N_{21}N_{20}CG$).

The tRNA of the present invention may not have a D-arm which $tRNA^{GluE2(CGG)}$ has. Specifically, the tRNA of the present invention may have a D-arm having a base sequence different from the base sequence of the D-arm of a tRNA known as $tRNA^{GluE2(CGG)}$, $tRNA^{Pro1E2(GAU)}$, $tRNA^{Pro1E2(GGU)}$, or $tRNA^{Pro1E2(GUG)}$. More specifically, it is presumed that the tRNA of the present invention may not have a D-stem in which GCGC and CGCG form a pair. Generally, it may not have a D-arm in which RNAs include a base sequence represented by SEQ ID NO: 134. The base sequence represented by SEQ ID NO: 134 may alternatively be $N_9N_{10}GCGCAGCCUGGUAGCGCN_{20}N_{21}$ (SEQ ID NO: 135) (in SEQ ID NO: 135, $N_9$, $N_{10}$, $N_{29}$, and $N_{21}$ have the same meanings as described above and it is a D-stem in which $N_9N_{10}GC$ and $N_{21}N_{20}CG$ form a base pair) or GCGCGCAGCCUGGUAGCGCGC (SEQ ID NO: 136).

The tRNA of the present invention may not have the base sequence which $tRNA^{Pro1E2(CGG)}$, $tRNA^{Pro1E2(GAU)}$, $tRNA^{Pro1E2(GGU)}$, $tRNA^{Pro1E2(GUG)}$ and $tRNA^{GluE2(CGG)}$ have.

The base sequences of these tRNAs are disclosed in WO2019/077887.

In the present invention, the translation system contains the tRNA of the present invention and the translation system including the tRNA enables the synthesis of a peptide having consecutive N-methyl amino acids which generally have difficulty in consecutive introduction. More specifically, it becomes a translation system capable of synthesizing a peptide having 2 or more consecutive N-methyl amino acids. It is also possible to prepare a peptide library with a translation system using a codon table to which 2 or more, preferably 3 or more, 4 or more, 5 or more, 6 or more, 7 or more, 8 or more, or 9 or more N-methyl amino acids are simultaneously assigned.

The translation system is not particularly limited insofar as it contains the tRNA of the present invention and it contains constituents used in a cell-free translation system.

The translation system preferably contains an EF-Tu protein. In addition, it preferably contains other constituents used in a conventionally known cell-free translation system.

The translation system of the present invention contains the tRNA of the present invention and preferably contains a tRNA to be charged with a proteinogenic amino acid. The tRNA to be charged with a proteinogenic amino acid is more preferably a naturally occurring tRNA and has, as a T-arm, the T-arm of a naturally occurring tRNA present in nature. Particularly, the tRNA charged with a proteinogenic amino acid is preferably a naturally occurring tRNA charged therewith. The naturally occurring tRNA charged therewith may be a tRNA present in a natural translation system. This means that when a tRNA not subjected to artificial alteration before charging is charged with an amino acid, the tRNA charged with the amino acid has the T-arm of a naturally occurring tRNA.

When a proteinogenic amino acid is not reconstituted in the codon table, a tRNA present in a natural translation system and charged with the proteinogenic amino acid may be used. When a proteinogenic amino acid is reconstituted in the codon table, a tRNA present in a natural translation system and corresponding to an assigned codon may be charged with the proteinogenic amino acid.

The term "charge" or "charged" as used herein has the same meaning as "bind" or "bound" or "combine" or "combined", respectively. Therefore, "a tRNA charged with a proteinogenic amino acid" means a tRNA acylated with a proteinogenic amino acid and has the same meaning as a tRNA having an "aminoacylated" proteinogenic amino acid.

The translation system of the present invention may contain a plurality of the tRNAs of the present invention. It may contain a plurality of the tRNAs of the present invention different in N-methyl amino acids to be charged (in this case, corresponding to codons assigned to N-methyl amino acids) or may contain a plurality of the tRNAs different in T-arm.

Described specifically, the translation system may contain a plurality of tRNA selected from tRNAs having a T-arm having a base sequence represented by SEQ ID NO: 4. When the translation system contains a plurality of tRNAs having a T-arm having a base sequence represented by SEQ ID NO: 4, they may be two or more selected from the aforesaid preferable base sequences. For example, when two tRNAs having a combination of the base sequences of a T-arm, that is, $N_1N_2GGN_3$ and $N_4CCN_5U$ as the base sequence represented by SEQ ID NO: 4 are used, examples include a combination of $AN_2GGN_3$ and $N_4CCN_5U$ with $GN_2GGN_3$ and $N_4CCN_5U$ and a combination of $AGGGN_3$ and $N_4CCCU$ with $GCGGN_3$ and $N_4CCGU$.

More specifically, the translation system of the present invention may contain a tRNA having a T-arm having a base sequence represented by SEQ ID NO: 1 and/or a tRNA having a T-arm having a base sequence represented by SEQ ID NO: 2; or may contain a tRNA having a T-arm having a base sequence represented by SEQ ID NO: 5 and/or a tRNA having a T-arm having a base sequence represented by SEQ ID NO: 6.

In the present invention, the translation system may contain a tRNA having a T-arm having a base sequence represented by SEQ ID NO: 1 and a tRNA having a T-arm having a base sequence represented by SEQ ID NO: 2; or may contain a tRNA having a T-arm having a base sequence represented by SEQ ID NO: 5 and a tRNA having a T-arm having a base sequence represented by SEQ ID NO: 6. In the translation system of the present invention, when the tRNA having a T-arm having a base sequence represented by SEQ ID NO: 1 is compared with the tRNA having a T-arm having a base sequence represented by SEQ ID NO: 2, the tRNA having a T-arm having a base sequence represented by SEQ ID NO: 2 has higher EF-Tu affinity, so that it is preferred to charge the tRNA having a T-arm having a base sequence represented by SEQ ID NO: 2 with an N-methyl amino acid, which has difficulty in introduction into a peptide.

As the translation system of the present invention, the tRNA having a T-arm having a base sequence represented by SEQ ID NO: 1 and the tRNA having a T-arm having a base sequence represented by SEQ ID NO: 2 may be charged with an N-methyl amino acid as needed, in consideration of the EF-Tu affinity.

When a tRNA aminoacylated with an N-methyl amino acid has EF-Tu affinity without using the tRNA of the present invention having a T-stem, the naturally occurring tRNA charged with an amino acid corresponding to the N-methyl amino acid (for example, an amino acid corresponding to N-methylglycine is glycine) may be charged. Alternatively, a naturally occurring tRNA corresponding to a codon to which N-methyl glycine is assigned may be charged. Examples of such an N-methyl amino acid include N-methylglycine and N-methylserine. The tRNA of the present invention may be charged with N-methylglycine or N-methylserine, but in the translation system of the present invention, N-methylglycine or N-methylserine is not necessarily charged to the tRNA of the present invention.

In the present invention, to obtain the translation system of the present invention, a tRNA having a T-stem in which $N_1N_2GGN_3$ and $N_4CCN_5U$ form a pair is charged with an N-methyl amino acid to prepare the tRNA of the present invention charged with an N-methyl amino acid.

Also in the present invention, in the translation in the present invention, that is, a method of producing a peptide library or a method of producing a library of a complex between a peptide and an mRNA encoding the peptide, a translation system including the tRNA of the present invention is prepared. The translation system preferably contains an EF-Tu protein.

The present invention includes a step of translating in a cell-free translation system using the tRNA or translation system of the present invention and it preferably includes a step of preparing the translation system of the present invention before translating in the cell-free translation system.

In the step of preparing the translation system of the present invention, a translation system for translating in a cell-free translation system is prepared. In the present invention, in the preparation step, it is preferred to determine, prior to charging with an N-methyl amino acid, which tRNA is charged: a tRNA having a T-stem in which $AGGGN_3$ and $N_4CCCU$ form a pair, a tRNA having a T-stem in which $GCGGN_3$ and $N_4CCGU$ form a pair, or a tRNA other than the aforesaid ones.

The N-methyl amino acid with which is charged to a tRNA is incorporated in an amino acid sequence of a peptide by translating the charged product in a cell-free translation system.

For efficient translation, it is preferred that the EF-Tu affinity of the tRNA charged with an N-methyl amino acid is equivalent to the EF-Tu affinity of a tRNA charged with another amino acid (including an N-methyl amino acid).

In the preparation step, therefore, it is preferred to determine, prior to charging with an N-methyl amino acid, which tRNA is charged, a tRNA having a T-stem in which $AGGGN_3$ and $N_4CCCU$ form a pair, a tRNA having a T-stem in which $GCGGN_3$ and $N_4CCGU$ form a pair, or a tRNA other than the aforesaid ones, in consideration of the EF-Tu affinity.

The tRNAs charged with an amino acid (including an N-methyl amino acid) prepared in consideration of the EF-Tu affinity preferably have equivalent incorporation efficiencies when they are incorporated in an amino acid sequence of a peptide.

In the preparation step, it is preferred to select, as a tRNA to be charged with an N-methyl amino acid, a tRNA having $AGGGG(N_4)_mCCCCU$ (SEQ ID NO: 1), a tRNA having $GCGGG(N_4)_mCCCGU$ (SEQ ID NO: 2), or a tRNA other than the aforesaid ones.

The selection from the tRNA having a T-arm having a base sequence represented by SEQ ID NO: 1, the tRNA having a T-arm having a base sequence represented by SEQ ID NO: 2 and a naturally occurring tRNA is preferably performed so that the EF-Tu affinity falls within a specific range.

As an index of the EF-Tu affinity of a tRNA, $\Delta G$ (kcal/mol) is preferably in a range of -7.5 to -10 and within this range, the upper limit is preferably -8 or less and the lower limit is preferably -9.5 or more, more preferably -9 or more.

The $\Delta G$ (kcal/mol) is preferably in a range of -7.5 to -10 and it may be -8 to -10, -7.5 to -9.5, -7.5 to -9, -8 to -9.5 or -8 to -9.

In the translation system of the present invention, reassignment of a codon using a flexizyme is performed by assigning an N-methyl amino acid to a codon selected as needed as NNN.

In the translation system of the present invention, a natural translation system may be used. In the natural translation system, tRNAs having an anticodon corresponding to each amino acid exist and these tRNAs have inherent sequences in a region other than an anticodon loop, respectively.

In the present invention, if the base sequence of the T-stem of the present invention is introduced, the tRNA may apply sequences inherent to tRNAs in the natural translation system at the other sequences, so that a base sequence of an acceptor stem, an anticodon stem, an anticodon loop and a variable loop may be arbitrary.

In the translation system of the present invention, a flexizyme is used to reassign an arbitrary amino acid to all the NNNs (N is an arbitrary base) and in this case, all the tRNAs may be artificial or a combination of naturally occurring and artificial ones. In this case, 85% or more or 90% or more of the full length of respective elongator tRNAs corresponding to the NNNs to be added to the translation system may be composed of the same base sequence; or an elongator tRNA group having the same sequence as most of the sequences except an anticodon may be used.

The term "anticodon loop" as used herein means an anticodon-containing single-stranded loop portion of a tRNA. The sequence of the anticodon loop can be determined as needed by those skilled in the art to complement the codon-anticodon interaction.

The present invention provides a method of producing a peptide library including:

a step of preparing an mRNA library containing mRNAs encoding the respective peptides of the peptide library, the mRNAs each containing NNNs encoding one or more than one, preferably more than one N-methyl amino acids; and
a step of translating the mRNAs of the mRNA library in a cell-free translation system which contains a tRNA having an anticodon to any codon of the NNNs and charged with an N-methyl amino acid corresponding to the codon.

The translation system is preferably a translation system for which the tRNA having a T-stem or T-arm according to the present invention is selected, as described above.

Described specifically, the cell-free translation system is preferably obtained by charging, with an N-methyl amino acid, any of the naturally occurring tRNA, the tRNA having a T-arm having a base sequence represented by SEQ ID NO: 1 and the tRNA having a T-arm having a base sequence represented by SEQ ID NO: 2. The naturally occurring tRNA may be charged with a proteinogenic amino acid and the translation system preferably contains the tRNA charged with an N-methyl amino acid and having a T-arm having a base sequence represented by SEQ ID NO: 1 and/or the tRNA charged with an N-methyl amino acid and having a T-arm having a base sequence represented by SEQ ID NO: 2, more preferably contains both of these tRNAs.

In the present invention, N-methylalanine, N-methylglycine, and N-methylserine are preferably charged to the naturally occurring tRNA; N-methylalanine may be charged to the tRNA of the present invention. It may be charged to the tRNA having a T-arm having a base sequence represented by SEQ ID NO: 1 or may be charged to the tRNA having a T-arm having a base sequence represented by SEQ ID NO: 5.

Examples of the N-methyl amino acid which may be charged to the tRNA having a T-arm having a base sequence represented by SEQ ID NO: 1 or the tRNA having a T-arm having a base sequence represented by SEQ ID NO: 5 include N-methyl amino acids other than N-methylglycine and N-methylserine.

An N-methylated unnatural amino acid may be charged to the tRNA of the present invention.

Examples of N-methyl amino acids which may be charged to the tRNA having a T-arm having a base sequence represented by SEQ ID NO: 2 or the tRNA having a T-arm having a base sequence represented by SEQ ID NO: 6 include N-methyl amino acids which are not charged to tRNAs other than the tRNA of the present invention and may be charged to the tRNA having a T-arm having a base sequence represented by SEQ ID NO: 1 or the tRNA having a T-arm having a base sequence represented by SEQ ID NO: 5. Although not particularly limited, N-methylvaline may be charged among them.

N-methyl amino acids other than N-methylvaline, N-methylglycine and N-methylserine may be charged to the tRNA having a T-arm having a base sequence represented by SEQ ID NO: 1, 2, 5 or 6. Examples of such an N-methyl amino acid include N-methyl amino acids listed in Fig. 1d. The tRNA charged with $^{Ac}$K in place of the N-methyl amino acid may be used in the translation system of the present invention.

In the present invention, the method of producing a peptide-mRNA complex library is performed by, in preparing an mRNA library by the aforesaid method of producing a peptide library, binding puromycin to the downstream region of the ORF (Open reading frame) of each mRNA. The puromycin may be bound to the mRNA via a linker composed of a peptide or a nucleic acid. When puromycin is bound to the ORF downstream region of the mRNA, a ribosome which has translated the ORF of the mRNA incorporates puromycin therein to form an mRNA-peptide complex. Such a peptide-mRNA complex can associate a genotype with a phenotype and can be applied to the in vitro display.

In the present invention, first, an NNN to which an N-methyl amino acid is assigned is selected and then, a tRNA having an anticodon of the NNN in an anticodon loop and encoding an N-methyl amino acid is prepared with a

flexizyme or the like.

Then, by preparing an mRNA library of mRNAs each containing an NNN encoding one or more than one N-methyl amino acids and performing translation, peptides containing the assigned N-methyl amino acid can be expressed.

The term "NNN" as used herein means a codon designating an amino acid and three Ns forming the codon are independently selected from adenine (A), guanine (G), cytosine (C) and uracil (U). One mRNA may contain a plurality of NNNs encoding an N-methyl amino acid.

In the present invention, an arbitrary amino acid may be reassigned to an NNN not encoding an N-methyl amino acid, and a relation between a codon and an amino acid based on a natural genetic code may be applied.

In the reassignment, an amino acid different from the relation between a codon and an amino acid in the natural genetic code table may be assigned or the same one may be assigned.

The term "natural genetic code table" is a table showing amino acids assigned to the triplet (codon) of an mRNA in a living body. The table is shown below in Table 1.

[Table 1]

| Base of second letter → | | | | | | | | | | | |
|---|---|---|---|---|---|---|---|---|---|---|---|
| | | U | | C | | A | | G | | | |
| | | Codon | Amino acid | Codon | Amino acid | Codon | Amino acid | Codon | Amino acid | | |
| | U | UUU | Phenylalanine | UCU | Serine | UAU | Tyrosine | UGU | Cysteine | U | |
| | | UUC | Phenylalanine | UCC | Serine | UAC | Tyrosine | UGC | Cysteine | C | |
| | | UUA | Leucine | UCA | Serine | UAA | Stop | UGA | Stop | A | |
| Base | | UUG | Leucine | UCG | Serine | UAG | Stop | UGG | Tryptophan | G | Base |
| of | C | CUU | Leucine | ccu | Proline | CAU | Histidine | CGU | Arginine | | of |
| first | | cuc | Leucine | CCC | Proline | CAC | Histidine | CGC | Arginine | C | third |
| letter | | CUA | Leucine | CCA | Proline | CAA | Glutamine | CGA | Arginine | A | letter |
| ↓ | | CUG | Leucine | CCC | Proline | CAG | Glutamine | CGG | Arginine | | ↓ |
| | A | AUU | Isoleucine | ACU | Threonine | AAU | Asparagine | AGU | Serine | U | |
| | | AUC | Isoleucine | ACC | Threonine | AAC | Asparagine | AGC | Serine | C | |
| | | AUA | Isoleucine | ACA | Threonine | AAA | Lysine | AGA | Arginine | A | |
| | | AUG | Methionine | ACG | Threonine | AAG | Lysine | AGG | Arginine | G | |
| | G | GUU | Valine | GCU | Alanine | GAU | Aspartic acid | GGU | Glycine | U | |
| | | GUC | Valine | GCC | Alanine | GAC | Aspartic acid | GGC | Glycine | | |
| | | GUA | Valine | GCA | Alanine | GAA | Glutamic acid | GGA | Glycine | A | |
| | | GUG | Valine | GCG | Alanine | GAG | Glutamic acid | GGG | Glycine | G | |

Assignment, to each codon, of an amino acid different from that in the natural genetic code table is achieved, for example, by codon reassignment making use of an artificially amino acylation RNA catalyst, Flexizyme. Flexizyme can make a desired amino acid bound to a tRNA having an arbitrary anticodon, so that an arbitrary amino acid can be assigned to an arbitrary codon.

The term "amino acid" as used herein includes an artificial amino acid variant or derivative as well as a proteinogenic amino acid and examples include proteinogenic L-amino acids and chemically synthesized compounds having properties known in the art as characteristics of an amino acid.

The "proteinogenic amino acids" (proteinogenic amino acids) when expressed by a three-letter code known in the art are Arg, His, Lys, Asp, Glu, Ser, Thr, Asn, Gln, Cys, Gly, Pro, Ala, Ile, Leu, Met, Phe, Trp, Tyr and Val.

Non-proteinogenic amino acids (non-proteinogenic amino acids) other than an N-methyl amino acid may be used and the term "non-proteinogenic amino acids" means a natural or unnatural amino acid other than the proteinogenic amino acid.

Examples of the unnatural amino acid include α,α-disubstituted amino acids (such as α-methylalanine), N-alkyl-α-amino acids, D-amino acids, β-amino acids and α-hydroxy acids, each having a main chain structure different from that of natural amino acids; amino acids (such as norleucine and homohistidine) having a side-chain structure

different from that of natural amino acids; amino acids (such as "homo"amino acids, homophenylalanine and homohistidine) having extra methylene in the side chain thereof; and amino acids (such as cysteic acid) obtained by substituting a carboxylic acid functional group in the side chain thereof by a sulfonic acid group. Specific examples of the unnatural amino acid include amino acids described in WO2015/030014.

The non-proteinogenic amino acid is preferably a D-amino acid, a β-amino acid or an α,α-disubstituted amino acid, more preferably a D-amino acid or a β-amino acid.

In the present invention, a peptide library containing $1\times10^6$ or more peptides can be produced.

The number of amino acids contained in each peptide and encoded by NNN is not particularly limited insofar as an N-methyl amino acid is contained and examples of the number may be 1, 2, 3, 4, 5, 6, 7, 8, 9, 10, 15, 20 and 30. It may be 1 to 10. The position of the NNN encoding a non-proteinogenic amino acid in each mRNA is not particularly limited. By using the tRNA of the present invention, a peptide having consecutive non-proteinogenic amino acids is translated also from an mRNA in which NNNs encoding a non-proteinogenic amino acid are consecutively placed, in the same level as the library synthesis of proteinogenic amino acids.

For codon reassignment, a translation system obtained by arbitrarily removing constituent factors of the translation system according to purpose and reconstituting only necessary components may be used. For example, when a translation system from which a specific amino acid has been removed is reconstituted, a codon corresponding to the amino acid becomes an empty codon that does not encode any amino acid. When an arbitrary amino acid is combined with a tRNA having an anticodon complementary to the empty codon by making use of a flexizyme or the like and translation is performed by adding it, the arbitrary amino acid is encoded by the codon and a peptide having the arbitrary amino acid introduced instead of the removed amino acid is translated.

The term "cell-free translation system" as used herein means a translation system not containing cells. As the cell-free translation system, for example, an Escherichia coli extract, a wheat germ extract, a rabbit reticulocyte extract or an insect cell extract may be used. Alternatively, a reconstituted cell-free translation system may be used, which is obtained by reconstituting a purified ribosome protein, aminoacyl-tRNA synthase (aaRS), ribosomal RNA, amino acid, rRNA, GTP, ATP, translation initiation factor (IF), elongation factor (EF), release factor (RF) and ribosome regeneration factor (RRF), and another factor necessary for translation.

The system may contain RNA polymerase for simultaneously performing transcription from DNA. Examples of a commercially available cell-free translation system include Escherichia-coli derived systems such as "RTS-100" (trade mark) of Roche Diagnostics, reconstituted translation systems such as "PURESYSTEM" (trade mark) of PGI and "PURExpressR In Vitro Protein Synthesis Kit" of New England BioLabs and systems using a wheat germ extract such as those of ZOEGENE Corporation or CellFree Sciences.

As a system using a ribosome of Escherichia coli, for example, a technology described in the following documents is known: H. F. Kung et al., 1977. The Journal of Biological Chemistry Vol. 252, No. 19, 6889-6894; M. C. Gonza et al., 1985, Proceeding of National Academy of Sciences of the United States of America Vol. 82, 1648-1652; M. Y. Pavlov and M. Ehrenberg, 1996, Archives of Biochemistry and Biophysics Vol. 328, No. 1, 9-16; Y. Shimizu et al., 2001, Nature Biotechnology Vol. 19, No. 8, 751-755; and H. Ohashi et al., 2007, Biochemical and Biophysical Research Communications Vol. 352, No. 1, 270-276.

By using the cell-free translation system, a high-purity expression product can be obtained without purifying.

The cell-free translation system of the present invention may be used not only for translation, but also for transcription after adding a factor necessary for transcription.

The peptide obtained in the present invention may be a cyclic peptide and it may be, for example, a cyclic peptide in which an amino acid having Functional group 1 and an amino acid having Functional group 2 corresponding thereto shown below in Table 2 are cyclized.

Either Functional group 1 or Functional group 2 may be placed on the N-terminal side; they may be placed at the N terminal and C terminal sides, respectively; one may be a terminal amino acid and the other one may be a non-terminal amino acid; or both groups may be a non-terminal amino acid.

The bond formed by Functional group 1 and Functional group 2 may be a chemically crosslinked structure for forming a molecular ring structure in a cyclic peptide.

[Table 2]

| | Functional group1 | Functional group2 |
|---|---|---|
| (A) | 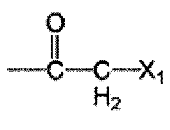 (A-1) | HS- (A-2) |
| (B) | -C≡C-H (B-1) | $N_3$- (B-2) |

(continued)

| | Functional group1 | Functional group2 |
|---|---|---|
| (C) | $-Ar-CH_2NH_2$ (C-1) | 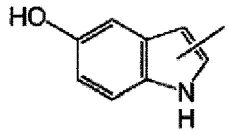 (C-2) |
| (D) | $-C{\equiv}C-CH_2-X_1$ (D-1) | HS- (D-2) |
| (E) | $-Ar-CH_2-X_1$ (E-1) | HS- (E-2) |

In the formula, $X_1$ is a leaving group, for example, the leaving group is a halogen atom such as Cl, Br, or I; and Ar is an aromatic ring which may have a substituent.

As an amino acid having Functional group (A-1), for example, a chloroacetylated amino acid can be used. Examples of the chloroacetylated amino acid include N-chloroacetyl-L-alanine, N-chloroacetyl-L-phenylalanine, N-chloroacetyl-L-tyrosine, N-chloroacetyl-L-tryptophan, N-3-(2-chloroacetamido)benzoyl-L-phenylalanine, N-3-(2-chloroacetamido)benzoyl-L-tyrosine, N-3-(2-chloroacetamido)benzoyl-L-tryptophan, β-N-chloroacetyl-L-diaminopropanoic acid, γ-N-chloroacetyl-L-diaminobutyric acid, δ-N-chloroacetyl-L-ornithine, and ε-N-chloroacetyl-L-lysine, and D-amino acid derivatives corresponding thereto.

As the amino acid having Functional group (A-1), N-chloroacetyl-L-tryptophan and N-chloroacetyl-L-tyrosine are preferred, with D-forms being more preferred.

The present specification sometimes clearly describes that the amino acid used is an L-form, but it may be either an L-form or a D-form, or it may be a mixture of an L-form and a D-form mixed at any ratio. Even when the present specification does not clearly describe that the amino acid used is an L-form or a D-form, it means that it may be either an L-form or a D-form, or it may be a mixture of an L-form and a D-form at any ratio.

Examples of an amino acid having Functional group (A-2) include cysteine, homocysteine, mercaptonorvaline, mercaptonorleucine, 2-amino-7-mercaptoheptanoic acid, and 2-amino-8-mercaptooctanoic acid.

As the amino acid having Functional group (A-2), cysteine is preferred.

Examples of a cyclization method with the amino acid having Functional group (A-1) and the amino acid having Functional group (A-2) include methods described in Kawakami, T. et al., Nature Chemical Biology 5, 888-890 (2009); Yamagishi, Y. et al., ChemBioChem 10, 1469-1472 (2009); Sako, Y. et al., Journal of American Chemical Society 130, 7932-7934 (2008); Goto, Y. et al., ACS Chemical Biology 3, 120-129 (2008); Kawakami T. et al., Chemistry & Biology 15, 32-42 (2008); and WO2008/117833.

Examples of an amino acid having Functional group (B-1) include propargylglycine, homopropargylglycine, 2-amino-6-heptynoic acid, 2-amino-7-octynoic acid, and 2-amino-8-nonynoic acid.

A 4-pentynoylated or 5-hexynoylated amino acid may also be used.

Examples of the 4-pentynoylated amino acid include N-(4-pentenoyl)-L-alanine, N-(4-pentenoyl)-L-phenylalanine, N-(4-pentenoyl)-L-tyrosine, N-(4-pentenoyl)-L-tryptophan, N-3-(4-pentynoylamido)benzoyl-L-phenylalanine, N-3-(4-pentynoylamido)benzoyl-L-tyrosine, N-3-(4-pentynoylamido)benzoyl-L-tryptophan, β-N-(4-pentenoyl)-L-diaminopropanoic acid, γ-N-(4-pentenoyl)-L-diaminobutyric acid, σ-N-(4-pentenoyl)-L-ornithine and ε-N-(4-pentenoyl)-L-lysine, and D-amino acid derivatives corresponding thereto.

Examples of the 5-hexynoylated amino acid include amino acids obtained by substituting the 4-pentynoyl group of the compounds mentioned above as the 4-pentynoylated amino acids by a 5-hexynoyl group.

Examples of an amino acid having Functional group (B-2) include azidoalanine, 2-amino-4-azidobutanoic acid, azidoptonorvaline, azidonorleucine, 2-amino-7-azidoheptanoic acid, and 2-amino-8-azidooctanoic acid.

In addition, azidoacetylated or 3-azidopentanoylated amino acids can also be used.

Examples of the azidoacetylated amino acids include N-azidoacetyl-L-alanine, N-azidoacetyl-L-phenylalanine, N-azidoacetyl-L-tyrosine, N-azidoacetyl-L-tryptophan, N-3-(4-pentynoylamido)benzoyl-L-phenylalanine, N-3-(4-pentynoylamido)benzoyl-L-tyrosine, N-3-(4-pentynoylamido)benzoyl-L-tryptophan, β-N-azidoacetyl-L-diaminopropanoic acid, γ-N-azidoacetyl-L-diaminobutyric acid, σ-N-azidoacetyl-L-ornithine and ε-N-azidoacetyl-L-lysine, and D-amino acid derivatives corresponding thereto.

Examples of the 3-azidopentanoylated amino acids include amino acids obtained by substituting the azidoacetyl group of the compounds mentioned above as the azidoacetylated amino acids by a 3-azidopentanoyl group.

Examples of a cyclization method with the amino acid having Functional group (B-1) and the amino acid having Functional group (B-2) include those described in Sako, Y. et al., Journal of American Chemical Society 130, 7932-7934 (2008) and WO2008/117833.

Examples of an amino acid having Functional group (C-1) include N-(4-aminomethyl-benzoyl)-phenylalanine (AMBF) and 3-aminomethyltyrosine.

Examples of an amino acid having Functional group (C-2) include 5-hydroxytryptophan (WOH).

Examples of a cyclization method with the amino acid having Functional group (C-1) and the amino acid having Functional group (C-2) include those described in Yamagishi, Y. et al., ChemBioChem 10, 1469-1472 (2009) and WO2008/117833.

Examples of an amino acid having Functional group (D-1) include 2-amino-6-chloro-hexynoic acid, 2-amino-7-chloro-heptynoic acid and 2-amino-8-chloro-octynoic acid.

Examples of an amino acid having Functional group (D-2) include cysteine, homocysteine, mercaptonorvaline, mercaptonorleucine, 2-amino-7-mercaptoheptanoic acid and 2-amino-8- mercaptooctanoic acid.

Examples of a cyclization method with the amino acid having Functional group (D-1) and the amino acid having Functional group (D-2) include that described in WO2012/074129.

Examples of an amino acid (E-1) include
N-3-chloromethylbenzoyl-L-phenylalanine, N-3-chloromethylbenzoyl-L-tyrosine and N-3-chloromethylbenzoyl-L-tryptophane, and D-amino acid derivatives corresponding thereto.

Examples of an amino acid (E-2) include cysteine, homocysteine, mercaptonorvaline, mercaptonorleucine, 2-amino-7-mercaptoheptanoic acid and 2-amino-8- mercaptooctanoic acid.

A cyclization method with the amino acid having Functional group (E-1) and the amino acid having Functional group (E-2) can be carried out with reference to, for example, the cyclization method with (A-1) and (A-2) or the cyclization method with (D-1) and (D-2).

As a ring-forming amino acid, a combination of the amino acid having Functional group (A-1) with the amino acid having Functional group (A-2) is preferred; a combination of N-acetyltryptophan whose H has been substituted by a leaving group with cysteine is more preferred; and a combination of an N-haloacetyl-D-tyrosine or an N-haloacetyl-D-tryptophan, preferably N-chloroacetyl-D-tyrosine or N-chloroacetyl-D-tryptophan with cysteine (Cys) is still more preferred.

The present invention also provides a peptide library or a peptide-mRNA complex library.

Producing a peptide library with the tRNA of the present invention enables the synthesis of a peptide containing an N-methyl amino acid at an expression level equal to that of a peptide composed of an L-amino acid, so that a peptide library more abundant in variety in the N-methyl amino acid introduction in one peptide than a conventional peptide library can be formed.

Particularly, a library containing a peptide containing two or more N-methyl amino acids can be formed.

Producing a peptide-mRNA complex library with the tRNA of the present invention enables the synthesis of a peptide containing an N-methyl amino acid at an expression level equal to that of a peptide composed of an L-amino acid, so that a peptide-mRNA complex library more abundant in variety in the N-methyl amino acid introduction than a conventional peptide-mRNA complex library can be formed.

The present invention also provides a screening method for identifying a peptide which binds to a target substance by using the peptide library produced with the tRNA of the present invention.

The screening method includes a step of bringing the peptide library produced with the tRNA of the present invention into contact with a target substance and incubating the resulting mixture.

The target substance is not particularly limited and examples include low molecular compounds, high molecular compounds, nucleic acids, peptides, proteins, sugars and lipids.

The screening method further includes a step of selecting a peptide bound to the target substance. The selection of a peptide bound to the target substance is performed, for example, by labeling the peptide to be detectable by a known method, washing the surface of a solid phase carrier with a buffer after the aforesaid contact step, and then detecting a compound bound to the target substance.

Examples of the detectable label include enzymes such as peroxidase and alkaline phosphatase, radioactive substances such as 125I, 131I, 35S and 3H, fluorescent substances such as fluorescein isothiocyanate, rhodamine, dansyl chloride, phycoerythrin, tetramethyl rhodamine isothiocyanate and near infrared fluorescent materials, light-emitting substances such as luciferase, luciferin and aequorin, and nanoparticles such as gold colloid and quantum dot. When the enzyme is used, detection can be achieved by adding a substrate of the enzyme to develop a color. Detection can also be achieved by binding biotin to a peptide and then binding avidin or streptavidin labeled with the enzyme or the like to the biotin-bound peptide.

Screening using the peptide-mRNA complex library can be carried out by applying the TRAP display method.

In this case, after the peptide-mRNA complex library is subjected to a reverse transcription reaction, the resulting library is brought into contact with a target substance. A complex that binds to the target substance is selected and its DNA is amplified by PCR. By adding the resulting DNA to a TRAP reaction system, a peptide-mRNA complex library is formed again. Then, a similar operation is repeated.

Since a peptide-mRNA complex having high affinity for the target substance is concentrated by the aforesaid

operation, a peptide that binds to the target substance can be identified efficiently by analyzing the DNA sequence of the concentrated complex.

Disclosure of all the Non-Patent Documents and Reference Literatures cited herein is incorporated herein by reference in their entirety.

## Examples

The present invention will hereinafter be described specifically based on Examples but the present invention is not limited to them. The present invention can be changed into various modes by those skilled in the art without departing from the significance of the present invention and such a change is also embraced within the scope of the present invention.

### Preparation of tRNA, flexizyme, mDNA and mRNA

The oligonucleotides listed in Table 3 were all purchased from Eurofins Genomics (Japan). In Table 3, G(Me) means 2'-O-methylguanosine. The sequence of a tRNA transcript prepared in the present Example are shown in Table 4. Double-stranded DNA templates encoding tRNAs were prepared by primer extension and then, PCR in the following manner: appropriate Forward primer and Reverse primer (each, 1 μM, refer to Table 5 as to the primers used) were mixed in 100 μL of a PCR mixture (10 mM Tris-HCl (pH 9.0), 50 mM KCl, 2.5 mM $MgCl_2$, each dNTP, 0.1% (v/v) Triton X-100, and 45 nM Taq DNA polymerase). The primer extension was performed by denaturation (at 95°C for 1 minute), 5 cycles of annealing (at 50°C for 1 minute), and extension (at 72°C for 1 minute). After 1 μL of the reaction mixture thus obtained was diluted 200-fold with 199 μL of a PCR mixture containing appropriate Forward and Reverse primers (each, 0.5 μM, refer to Table 5 as to the primers used) and PCR was performed by 12 cycles of denaturation (at 95°C for 40 seconds), annealing (at 50°C for 40 seconds), and extension (at 72°C for 40 seconds). The amplification of the resulting PCR product was confirmed by 3% agarose gel electrophoresis and ethidium bromide staining. The DNA thus obtained was purified by phenol/chloroform extraction and ethanol precipitation and then, the purified product was dissolved in 20 μL of water. The concentration of the tRNA was determined using $A_{260}$ of a 10-fold diluted solution.

The transcription reaction was performed by incubating 200 μL of a transcription mixture (40 mM Tris-HCl (pH 8.0), 1 mM spermidine, 0.01% (v/v) Triton X-100, 10 mM DTT, 22.5 mM $MgCl_2$, each NTP, 5 mM GMP, 22.5 mM KOH, 10% (v/v) DNA template, and 120 nM T7 RNA polymerase) overnight at 37°C. The transcription mixture was mixed with $MnCl_2$ (100 mM, 4 μL) and RQ1 RNase-free DNase (1 U/μL, 1 μL, Promega) and the resulting mixture was incubated at 37°C for 30 minutes. The tRNA transcript thus obtained was precipitated in isopropanol and then, dissolved in water. The tRNA transcript was purified by 8% denaturing PAGE and ethanol precipitation and was then dissolved in 10 μL of water.

Flexizymes (dFx and eFx) were prepared in accordance with the method described in Non-Patent Document 13 and Reference Literature 2. ADDIN EN.

CITE ADDIN EN.CITE.DATA

Double stranded DNA templates encoding mRNAs (refer to Table 6 as to the mDNA and mRNA sequences) were prepared by primer extension and PCR (refer to Table 7 as to the primers used). The mDNAs thus obtained were purified by phenol/chloroform extraction and ethanol precipitation and the purified product was then dissolved in 10 μL of water. The concentration was determined by 8% native PAGE and ethidium bromide staining with 100 bp Quick-Load DNA Ladders (New England BioLabs) as a control.

An mRNA was prepared by in vitro transcription as follows. A transcription mixture (200 μL) (40 mM Tris-HCl (pH 8.0), 1 mM spermidine, 0.01% (v/v) Triton X-100, 10 mM DTT, 30 mM $MgCl_2$, 5 mM each NTPs, 30 mM KOH, 10% (v/v) DNA template, and 120 nM T7 RNA polymerase) was incubated overnight at 37°C. The resulting transcription mixture was mixed with $MnCl_2$ (100 mM, 4 μL) and RQ1 RNase-free DNase (1 U/μL, 1 μL, Promega) and the resulting mixture was incubated at 37°C for 30 minutes. The resulting mRNA was precipitated by isopropanol and then dissolved in water. The tRNA transcript was purified by 8% denaturing PAGE and ethanol precipitation and then dissolved in 10 μL of water. The concentration of the mRNA was determined using $A_{260}$ of a 10-fold diluted solution.

[Table 3]

| Name | Sequence |
|---|---|
| O-197 | 5'-TAATACGACTCACTATAGCCCGGATAGCTCAGTCGGTAGAGCAGGGGACTGAAAATCCCCGTGTCC-3' |
| O-198 | 5'-GTGCCCGGACTCGGAATCGAACCAAGGACACGGGGATTTTCA-3' |
| O-199 | 5'-GGCGTAATACGACTCACTATAG-3' |
| O-200 | 5'-GG(Me)TGCCCGGACTCGG-3' |
| O-201 | 5'-TG(Me)GTGCCCGGACTCGG-3' |
| O-202 | 5'-GGCGTAATACGACTCACTATAGGTGGGGTTCCCGAGCGGCCAAAGGGAGCAGACTGTAAATCTGCCG-3' |
| O-203 | 5'-GGTGGTGGGGGAAGGATTCGAACCTTCGAAGTCTGTGACGGCAGATTTACAGTCTG-3' |
| O-204 | 5'-GG(Me)TGGTGGGGGAAGGAT-3' |
| O-205 | 5'-TG(Me)GTGGTGGGGGAAGGAT-3' |
| O-206 | 5'-GGCGTAATACGACTCACTATAGGAGAGATGCCGGAGCGGCTGAACGGACCGGTCTCGAAAACCGGAG-3' |
| O-207 | 5'-GGCGGAGAGAGGGGGATTTGAACCCCCGGTAGAGTTGCCCCTACTCCGGTTTTCGAGACC-3' |
| O-208 | 5'-GG(Me)CGGAGAGAGGGGGA-3' |
| O-209 | 5'-TG(Me)GCGGAGAGAGGGGGA-3' |
| O-210 | 5'-GTAATACGACTCACTATAGGCTCTGTAGTTCAGTCGGTAGAACGGCGG-3' |
| O-211 | 5'-GCGGCTCTGCAATGACTCGAACACTGGACATACGGATTGTCAGTCCGCCGTTCTACCG-3' |
| O-212 | 5'-GG(Me)CGGCTCTGCAATG-3' |
| O-213 | 5'-GCGGCTCTGACTGGACTCGAACCAGTGACATACGGATTGTCAGTCCGCCGTTCTACCG-3' |
| O-214 | 5'-GG(Me)CGGCTCTGACTGG-3' |
| O-215 | 5'-GCGGCTCTGAGGGGACTCGAACCCCTGACATACGGATTGTCAGTCCGCCGTTCTACCG-3' |
| O-216 | 5'-GG(Me)CGGCTCTGAGGGG-3' |
| O-217 | 5'-GCGGCTCTGACGGGACTCGAACCCGCGACATACGGATTGTCAGTCCGCCGTTCTACCG-3' |
| O-218 | 5'-GG(Me)CGGCTCTGACGGG-3' |
| O-219 | 5'-TG(Me)GCGGCTCTGCAATG-3' |
| O-220 | 5'-TG(Me)GCGGCTCTGACTGG-3' |
| O-221 | 5'-TG(Me)GCGGCTCTGAGGGG-3' |
| O-222 | 5'-TG(Me)GCGGCTCTGACGGG-3' |
| O-223 | 5'-GTAATACGACTCACTATAGGCTCTGTAGTTCAGTCGGAAGAACGGCGG-3' |
| O-224 | 5'-GCGGCTCTGCAATGACTCGAACACTGGACATACGGATTTTCAGTCCGCCGTTCTTCCG-3' |
| O-225 | 5'-GCGGCTCTGACTGGACTCGAACCAGTGACATACGGATTTTCAGTCCGCCGTTCTTCCG-3' |
| O-226 | 5'-GCGGCTCTGAGGGGACTCGAACCCCTGACATACGGATTTTCAGTCCGCCGTTCTTCCG-3' |
| O-227 | 5'-GCGGCTCTGACGGGACTCGAACCCGCGACATACGGATTTTCAGTCCGCCGTTCTACCG-3' |
| O-228 | 5'-GCGGCTCTGCAATGACTCGAACACTGGACATACGGATTTTGAGTCCGCCGTTCTACCG-3' |
| O-229 | 5'-GCGGCTCTGACTGGACTCGAACCAGTGACATACGGATTTTGAGTCCGCCGTTCTACCG-3' |
| O-230 | 5'-GCGGCTCTGAGGGGACTCGAACCCCTGACATACGGATTTTGAGTCCGCCGTTCTACCG-3' |
| O-231 | 5'-GCGGCTCTGACGGGACTCGAACCCGCGACATACGGATTTTGAGTCCGCCGTTCTACCG-3' |
| O-232 | 5'-GCGGCTCTGCAATGACTCGAACACTGGACATACGGAACCTCAATCCGCCGTTCTACCG-3' |
| O-233 | 5'-GCGGCTCTGACTGGACTCGAACCAGTGACATACGGAACCTCAATCCGCCGTTCTACCG-3' |
| O-234 | 5'-GCGGCTCTGAGGGGACTCGAACCCCTGACATACGGAACCTCAATCCGCCGTTCTACCG-3' |
| O-235 | 5'-GCGGCTCTGACGGGACTCGAACCCGCGACATACGGAACCTCAATCCGCCGTTCTACCG-3' |
| O-236 | 5'-GCGGCTCTGCAATGACTCGAACACTGGACATACGGAACCTGAATCCGCCGTTCTACCG-3' |
| O-237 | 5'-GCGGCTCTGACTGGACTCGAACCAGTGACATACGGAACCTGAATCCGCCGTTCTACCG-3' |
| O-238 | 5'-GCGGCTCTGAGGGGACTCGAACCCCTGACATACGGAACCTGAATCCGCCGTTCTACCG-3' |
| O-239 | 5'-GCGGCTCTGACGGGACTCGAACCCGCGACATACGGAACCTGAATCCGCCGTTCTACCG-3' |
| O-240 | 5'-GCGGCTCTGCAATGACTCGAACACTGGACATACGGATTATCAGTCCGCCGTTCTACCG-3' |
| O-241 | 5'-GCGGCTCTGACTGGACTCGAACCAGTGACATACGGATTATCAGTCCGCCGTTCTACCG-3' |
| O-242 | 5'-GCGGCTCTGAGGGGACTCGAACCCCTGACATACGGATTATCAGTCCGCCGTTCTACCG-3' |
| O-243 | 5'-GCGGCTCTGACGGGACTCGAACCCGCGACATACGGATTATCAGTCCGCCGTTCTACCG-3' |
| O-244 | 5'-GCGGCTCTGCAATGACTCGAACACTGGACATACGGAGTGTGAGTCCGCCGTTCTACCG-3' |
| O-245 | 5'-GCGGCTCTGACTGGACTCGAACCAGTGACATACGGAGTGTGAGTCCGCCGTTCTACCG-3' |
| O-246 | 5'-GCGGCTCTGAGGGGACTCGAACCCCTGACATACGGAGTGTGAGTCCGCCGTTCTACCG-3' |
| O-247 | 5'-GCGGCTCTGACGGGACTCGAACCCGCGACATACGGAGTGTGAGTCCGCCGTTCTACCG-3' |
| O-248 | 5'-GCGGCTCTGCAATGACTCGAACACTGGACATACGGACTGCCAGTCCGCCGTTCTACCG-3' |
| O-249 | 5'-GCGGCTCTGACTGGACTCGAACCAGTGACATACGGACTGCCAGTCCGCCGTTCTACCG-3' |
| O-250 | 5'-GCGGCTCTGAGGGGACTCGAACCCCTGACATACGGACTGCCAGTCCGCCGTTCTACCG-3' |
| O-251 | 5'-GCGGCTCTGACGGGACTCGAACCCGCGACATACGGACTGCCAGTCCGCCGTTCTACCG-3' |
| O-252 | 5'-GCGGCTCTGCAATGACTCGAACACTGGACATACGGATTGCGAATCCGCCGTTCTACCG-3' |
| O-253 | 5'-GCGGCTCTGACTGGACTCGAACCAGTGACATACGGATTGCGAATCCGCCGTTCTACCG-3' |
| O-254 | 5'-GCGGCTCTGAGGGGACTCGAACCCCTGACATACGGATTGCGAATCCGCCGTTCTACCG-3' |
| O-255 | 5'-GCGGCTCTGACGGGACTCGAACCCGCGACATACGGATTGCGAATCCGCCGTTCTACCG-3' |
| O-256 | 5'-GCGGCTCTGCAATGACTCGAACACCGGACATACGGATTGTCAGTCCGCCGTTCTACCG-3' |
| O-257 | 5'-GCGGCTCTGCAATGACTCGAACACCGGACATACGGATTTAGAGTCCGCCGTTCTACCG-3' |
| O-258 | 5'-TAATACGACTCACTATAGGGTTAACTTTAACAAGGAGAAAAACATGAAGAAGAAG-3' |
| O-259 | 5'-CGAAGCTTACTTGTCGTCGTCGTCCTTGTAGTCGTAGACCTTCTTCTTCATGTTTTTCTC-3' |
| O-260 | 5'-CGAAGCTTACTTGTCGTC-3' |
| O-261 | 5'-GAGAAAAACATGAAGAAGAAGACAAATTTCGGTATCCGTGCCTCTGTCATGGA-3' |
| O-262 | 5'-GTCGTCCTTGTAGTCGTATGGATGGAGCAGTTGCCATTCCATGACAGAGGCACG-3' |
| O-263 | 5'-CGAAGCTTACTTGTCGTCGTCGTCCTTGTAGTC-3' |
| O-264 | 5'-GAGAAAAACATGAAGAAGAAGCTCCATATGTTGGTCACAGAACTGTCTATCGCGCAAAA-3' |
| O-265 | 5'-GTCGTCCTTGTAGTCTGGGGCACGCACCCAACCGAAATTTTGCGCGATAGACAGT-3' |
| O-266 | 5'-TAATACGACTCACTATAGGGTTAACTTTAACAAGGAGAAAAACATGGACTACAAGGACGACGACGACAAG-3' |
| O-267 | 5'-CGAAGCTTACTTCTTCTTGATGAAGGCGACCAGGAGCTTGTCGTCGTCGTCCT-3' |
| O-268 | 5'-CGAAGCTTACTTCTTCTT-3' |

[Table 4]

| tRNA | Anticodon | T-stem | tRNA sequence |
|---|---|---|---|
| Phe without 3'-A | GAA | - | 5'-GCCCGGAUAGCUCAGUCGGUAGAGCAGGGGACUGAAAAUCCCCGUGUCCUUGGUUCGAUUCCGAGUCCGGGCACC-3' |
| Tyr without 3'-A | GUA | - | 5'-GGUGGGGUUCCCGAGCGGCCAAAGGGAGCAGACUGUAAAUCUGCCGUCACAGACUUCGAAGGUUCGAAUCCUUCCCCCACCACC-3' |
| Ser without 3'-A | CGA | - | 5'-GGAGAGAUGCCGGAGCGGCUGAACGGACCGGUCUCGAAAACCGGAGUAGGGGCAACUCUACCGGGGGUUCAAAUCCCCCUCUCUCCGCC-3' |
| AsnE2 without 3'-A | GAC | No.1 | 5'-GGCUCUGUAGUUCAGUCGGUAGAACGGCGGAUU GAC AUUCCGUAUGUCCAGUGUUCGAGUCAUUGCAGAGCCGCC-3' |
| AsnE2 without 3'-A | GAC | No.2 | 5'-GGCUCUGUAGUUCAGUCGGUAGAACGGCGGAUU GAC AUUCCGUAUGUCACUGGUUCGAGUCCAGUCAGAGCCGCC-3' |
| AsnE2 without 3'-A | GAC | No.3 | 5'-GGCUCUGUAGUUCAGUCGGUAGAACGGCGGAUU GAC AUUCCGUAUGUCAGGGGUUCGAGUCCCCUCAGAGCCGCC-3' |
| AsnE2 without 3'-A | GAC | No.4 | 5'-GGCUCUGUAGUUCAGUCGGUAGAACGGCGGAUU GAC AUUCCGUAUGUCGCGGGUUCGAGUCCCGUCAGAGCCGCC-3' |
| AsnE2 | GAC | No.1 | 5'-GGCUCUGUAGUUCAGUCGGUAGAACGGCGGAUU GAC AUUCCGUAUGUCCAGUGUUCGAGUCAUUGCAGAGCCGCCA-3' |
| AsnE2 | GAC | No.2 | 5'-GGCUCUGUAGUUCAGUCGGUAGAACGGCGGAUU GAC AUUCCGUAUGUCACUGGUUCGAGUCCAGUCAGAGCCGCCA-3' |
| AsnE2 | GAC | No.3 | 5'-GGCUCUGUAGUUCAGUCGGUAGAACGGCGGAUU GAC AUUCCGUAUGUCAGGGGUUCGAGUCCCCUCAGAGCCGCCA-3' |
| AsnE2 | GAC | No.4 | 5'-GGCUCUGUAGUUCAGUCGGUAGAACGGCGGAUU GAC AUUCCGUAUGUCGCGGGUUCGAGUCCCGUCAGAGCCGCCA-3' |
| AsnE2 | GAA | No.1 | 5'-GGCUCUGUAGUUCAGUCGGAAGAACGGCGGACU GAA AAUCCGUAUGUCCAGUGUUCGAGUCAUUGCAGAGCCGCCA-3' |
| AsnE2 | GAA | No.2 | 5'-GGCUCUGUAGUUCAGUCGGAAGAACGGCGGACU GAA AAUCCGUAUGUCACUGGUUCGAGUCCAGUCAGAGCCGCCA-3' |
| AsnE2 | GAA | No.3 | 5'-GGCUCUGUAGUUCAGUCGGAAGAACGGCGGACU GAA AAUCCGUAUGUCAGGGGUUCGAGUCCCCUCAGAGCCGCCA-3' |
| AsnE2 | GAA | No.4 | 5'-GGCUCUGUAGUUCAGUCGGAAGAACGGCGGACU GAA AAUCCGUAUGUCGCGGGUUCGAGUCCCGUCAGAGCCGCCA-3' |
| AsnE2 | CAA | No.1 | 5'-GGCUCUGUAGUUCAGUCGGUAGAACGGCGGACU CAA AAUCCGUAUGUCCAGUGUUCGAGUCAUUGCAGAGCCGCCA-3' |
| AsnE2 | CAA | No.2 | 5'-GGCUCUGUAGUUCAGUCGGUAGAACGGCGGACU CAA AAUCCGUAUGUCACUGGUUCGAGUCCAGUCAGAGCCGCCA-3' |
| AsnE2 | CAA | No.3 | 5'-GGCUCUGUAGUUCAGUCGGUAGAACGGCGGACU CAA AAUCCGUAUGUCAGGGGUUCGAGUCCCCUCAGAGCCGCCA-3' |
| AsnE2 | CAA | No.4 | 5'-GGCUCUGUAGUUCAGUCGGUAGAACGGCGGACU CAA AAUCCGUAUGUCGCGGGUUCGAGUCCCGUCAGAGCCGCCA-3' |
| AsnE2 | GAG | No.1 | 5'-GGCUCUGUAGUUCAGUCGGUAGAACGGCGGAUU GAG GUUCCGUAUGUCCAGUGUUCGAGUCAUUGCAGAGCCGCCA-3' |
| AsnE2 | GAG | No.2 | 5'-GGCUCUGUAGUUCAGUCGGUAGAACGGCGGAUU GAG GUUCCGUAUGUCACUGGUUCGAGUCCAGUCAGAGCCGCCA-3' |
| AsnE2 | GAG | No.3 | 5'-GGCUCUGUAGUUCAGUCGGUAGAACGGCGGAUU GAG GUUCCGUAUGUCAGGGGUUCGAGUCCCCUCAGAGCCGCCA-3' |
| AsnE2 | GAG | No.4 | 5'-GGCUCUGUAGUUCAGUCGGUAGAACGGCGGAUU GAG GUUCCGUAUGUCGCGGGUUCGAGUCCCGUCAGAGCCGCCA-3' |
| AsnE2 | CAG | No.1 | 5'-GGCUCUGUAGUUCAGUCGGUAGAACGGCGGAUU CAG GUUCCGUAUGUCCAGUGUUCGAGUCAUUGCAGAGCCGCCA-3' |
| AsnE2 | CAG | No.2 | 5'-GGCUCUGUAGUUCAGUCGGUAGAACGGCGGAUU CAG GUUCCGUAUGUCACUGGUUCGAGUCCAGUCAGAGCCGCCA-3' |
| AsnE2 | CAG | No.3 | 5'-GGCUCUGUAGUUCAGUCGGUAGAACGGCGGAUU CAG GUUCCGUAUGUCAGGGGUUCGAGUCCCCUCAGAGCCGCCA-3' |
| AsnE2 | CAG | No.4 | 5'-GGCUCUGUAGUUCAGUCGGUAGAACGGCGGAUU CAG GUUCCGUAUGUCGCGGGUUCGAGUCCCGUCAGAGCCGCCA-3' |
| AsnE2 | GAU | No.1 | 5'-GGCUCUGUAGUUCAGUCGGUAGAACGGCGGACU CAU AAUCCGUAUGUCCAGUGUUCGAGUCAUUGCAGAGCCGCCA-3' |
| AsnE2 | GAU | No.2 | 5'-GGCUCUGUAGUUCAGUCGGUAGAACGGCGGACU CAU AAUCCGUAUGUCACUGGUUCGAGUCCAGUCAGAGCCGCCA-3' |
| AsnE2 | GAU | No.3 | 5'-GGCUCUGUAGUUCAGUCGGUAGAACGGCGGACU CAU AAUCCGUAUGUCAGGGGUUCGAGUCCCCUCAGAGCCGCCA-3' |
| AsnE2 | GAU | No.4 | 5'-GGCUCUGUAGUUCAGUCGGUAGAACGGCGGACU CAU AAUCCGUAUGUCGCGGGUUCGAGUCCCGUCAGAGCCGCCA-3' |
| AsnE2 | CAC | No.1 | 5'-GGCUCUGUAGUUCAGUCGGUAGAACGGCGGACU CAC ACUCCGUAUGUCCAGUGUUCGAGUCAUUGCAGAGCCGCCA-3' |
| AsnE2 | CAC | No.2 | 5'-GGCUCUGUAGUUCAGUCGGUAGAACGGCGGACU CAC ACUCCGUAUGUCACUGGUUCGAGUCCAGUCAGAGCCGCCA-3' |
| AsnE2 | CAC | No.3 | 5'-GGCUCUGUAGUUCAGUCGGUAGAACGGCGGACU CAC ACUCCGUAUGUCAGGGGUUCGAGUCCCCUCAGAGCCGCCA-3' |
| AsnE2 | CAC | No.4 | 5'-GGCUCUGUAGUUCAGUCGGUAGAACGGCGGACU CAC ACUCCGUAUGUCGCGGGUUCGAGUCCCGUCAGAGCCGCCA-3' |
| AsnE2 | GGC | No.1 | 5'-GGCUCUGUAGUUCAGUCGGUAGAACGGCGGACU GGC AGUCCGUAUGUCCAGUGUUCGAGUCAUUGCAGAGCCGCCA-3' |
| AsnE2 | GGC | No.2 | 5'-GGCUCUGUAGUUCAGUCGGUAGAACGGCGGACU GGC AGUCCGUAUGUCACUGGUUCGAGUCCAGUCAGAGCCGCCA-3' |
| AsnE2 | GGC | No.3 | 5'-GGCUCUGUAGUUCAGUCGGUAGAACGGCGGACU GGC AGUCCGUAUGUCAGGGGUUCGAGUCCCCUCAGAGCCGCCA-3' |
| AsnE2 | GGC | No.4 | 5'-GGCUCUGUAGUUCAGUCGGUAGAACGGCGGACU GGC AGUCCGUAUGUCGCGGGUUCGAGUCCCGUCAGAGCCGCCA-3' |
| AsnE2 | CGC | No.1 | 5'-GGCUCUGUAGUUCAGUCGGUAGAACGGCGGAUU CGC AAUCCGUAUGUCCAGUGUUCGAGUCAUUGCAGAGCCGCCA-3' |
| AsnE2 | CGC | No.2 | 5'-GGCUCUGUAGUUCAGUCGGUAGAACGGCGGAUU CGC AAUCCGUAUGUCACUGGUUCGAGUCCAGUCAGAGCCGCCA-3' |
| AsnE2 | CGC | No.3 | 5'-GGCUCUGUAGUUCAGUCGGUAGAACGGCGGAUU CGC AAUCCGUAUGUCAGGGGUUCGAGUCCCCUCAGAGCCGCCA-3' |
| AsnE2 | CGC | No.4 | 5'-GGCUCUGUAGUUCAGUCGGUAGAACGGCGGAUU CGC AAUCCGUAUGUCGCGGGUUCGAGUCCCGUCAGAGCCGCCA-3' |
| AsnE2 | GAC | No.0 | 5'-GGCUCUGUAGUUCAGUCGGUAGAACGGCGGAUU GAC AUUCCGUAUGUCCGGUGUUCGAGUCAUUGCAGAGCCGCCA-3' |

[Table 5]

| tRNA | Anticodon | T-stem | Extension | | PCR | |
|---|---|---|---|---|---|---|
| | | | Forward primer | Reverse primer | Forward primer | Reverse primer |
| Phe without 3'-A | GAA | Native | O-197 | O-198 | O-199 | O-200 |
| Phe | GAA | Native | O-197 | O-198 | O-199 | O-201 |
| Tyr without 3'-A | GUA | Native | O-202 | O-203 | O-199 | O-204 |
| Tyr | GUA | Native | O-202 | O-203 | O-199 | O-205 |
| Ser without 3'-A | CGA | Native | O-206 | O-207 | O-199 | O-208 |
| Ser | CGA | Native | O-206 | O-207 | O-199 | O-209 |
| AsnE2 without 3'-A | GAC | No.1 | O-210 | O-211 | O-199 | O-212 |
| AsnE2 without 3'-A | GAC | No.2 | O-210 | O-213 | O-199 | O-214 |
| AsnE2 without 3'-A | GAC | No.3 | O-210 | O-215 | O-199 | O-216 |
| AsnE2 without 3'-A | GAC | No.4 | O-210 | O-217 | O-199 | O-218 |
| AsnE2 | GAC | No.1 | O-210 | O-211 | O-199 | O-219 |
| AsnE2 | GAC | No.2 | O-210 | O-210 | O-199 | O-220 |
| AsnE2 | GAC | No.3 | O-210 | O-212 | O-199 | O-221 |
| AsnE2 | GAC | No.4 | O-210 | O-214 | O-199 | O-222 |
| AsnE2 | GAA | No.1 | O-223 | O-224 | O-199 | O-219 |
| AsnE2 | GAA | No.2 | O-223 | O-225 | O-199 | O-220 |
| AsnE2 | GAA | No.3 | O-223 | O-226 | O-199 | O-221 |
| AsnE2 | GAA | No.4 | O-223 | O-227 | O-199 | O-222 |
| AsnE2 | CAA | No.1 | O-210 | O-228 | O-199 | O-219 |
| AsnE2 | CAA | No.2 | O-210 | O-229 | O-199 | O-220 |
| AsnE2 | CAA | No.3 | O-210 | O-230 | O-199 | O-221 |
| AsnE2 | CAA | No.4 | O-210 | O-231 | O-199 | O-222 |
| AsnE2 | GAG | No.1 | O-210 | O-232 | O-199 | O-219 |
| AsnE2 | GAG | No.2 | O-210 | O-233 | O-199 | O-220 |
| AsnE2 | GAG | No.3 | O-210 | O-234 | O-199 | O-221 |
| AsnE2 | GAG | No.4 | O-210 | O-235 | O-199 | O-222 |
| AsnE2 | CAG | No.1 | O-210 | O-236 | O-199 | O-219 |
| AsnE2 | CAG | No.2 | O-210 | O-237 | O-199 | O-220 |
| AsnE2 | CAG | No.3 | O-210 | O-238 | O-199 | O-221 |
| AsnE2 | CAG | No.4 | O-210 | O-239 | O-199 | O-222 |
| AsnE2 | GAU | No.1 | O-210 | O-240 | O-199 | O-219 |
| AsnE2 | GAU | No.2 | O-210 | O-241 | O-199 | O-220 |
| AsnE2 | GAU | No.3 | O-210 | O-242 | O-199 | O-221 |
| AsnE2 | GAU | No.4 | O-210 | O-243 | O-199 | O-222 |
| AsnE2 | CAC | No.1 | O-210 | O-244 | O-199 | O-219 |
| AsnE2 | CAC | No.2 | O-210 | O-245 | O-199 | O-220 |
| AsnE2 | CAC | No.3 | O-210 | O-246 | O-199 | O-221 |
| AsnE2 | CAC | No.4 | O-210 | O-247 | O-199 | O-222 |
| AsnE2 | GGC | No.1 | O-210 | O-248 | O-199 | O-219 |
| AsnE2 | GGC | No.2 | O-210 | O-249 | O-199 | O-220 |
| AsnE2 | GGC | No.3 | O-210 | O-250 | O-199 | O-221 |
| AsnE2 | GGC | No.4 | O-210 | O-251 | O-199 | O-222 |
| AsnE2 | CGC | No.1 | O-210 | O-252 | O-199 | O-219 |
| AsnE2 | CGC | No.2 | O-210 | O-253 | O-199 | O-220 |
| AsnE2 | CGC | No.3 | O-210 | O-254 | O-199 | O-221 |
| AsnE2 | CGC | No.4 | O-210 | O-255 | O-199 | O-222 |
| AsnE2 | GAC | No.0 | O-210 | O-256 | O-199 | O-219 |
| Non-functional | CUA | No.0 | O-210 | O-257 | O-199 | O-219 |

[Table 6]

| Name | Corresponding mDNA | mRNA sequence |
|---|---|---|
| mRNA12 | mDNA12 | 5'-GGGCUUUAAUAAGGAGAAAAACAUGAAGAAGAAGGUCUACGACUACAAGGACGACGACGACAAGUAAGCUUCG-3' |
| mRNA13 | mDNA13 | 5'-GGGCUUUAAUAAGGAGAAAAACAUGAAGAAGAAGACAAAUUUCGGUAUCCGUGCCUCUGUCAUGGAAUGGCAACUGCUCCAUCCAUACGACUACAAGGACGACGACGACAAGUAAGCUUCG-3' |
| mRNA14 | mDNA14 | 5'-GGGCUUUAAUAAGGAGAAAAACAUGAAGAAGAAGCUCCAUAUGUUGGUCACAGAACUGUCUAUCGCGCAAAAUUUCGGUUGGGUGCGUGCCCCAGACUACAAGGACGACGACGACAAGUAAGCUUCG-3' |

[Table 7]

| Name | Corresponding mRNA | Extension | | 1st PCR | | 2nd PCR | |
|---|---|---|---|---|---|---|---|
| | | Forward primer | Reverse primer | Forward primer | Reverse primer | Forward primer | Reverse primer |
| mRNA12 | mDNA12 | O-258 | O-259 | O-199 | O-260 | - | - |
| mRNA13 | mDNA13 | O-261 | O-262 | O-258 | O-263 | O-199 | O-260 |
| mRNA14 | mDNA14 | O-264 | O-265 | O-258 | O-263 | O-199 | O-260 |

The base sequences listed in Table 3 correspond to SEQ ID NOS: 8 to 78 in the order of mention, respectively. The base sequences listed in Table 4 correspond to SEQ ID NOS: 79 to 122 in the order of mention. The base sequence of the tRNA of the present invention may have a base sequence listed as No. 3 or No. 4 in Table 4.

Synthesis of aminoacyl-tRNA by flexizymes

Amino acids activated with an appropriate ester group (Phe-CME, Tyr-CME, Ser-DBE, $^{Me}$G-DBE, Me S-DBE, $^{Me}$A-DBE, $^{Me}$F-CME, $^{Me}$L-DBE, $^{Me}$M-DBE, $^{Me}$T-DBE, $^{Me}$Y-CME, $^{Me}$D-DBE, $^{Me}$V-DBE, $^{Me}$NI-DBE, $^{Me}$Ym-CME, $^{Me}$Nv-DBE, and $^{Ac}$K-DBE in which CME means a cyanomethyl ester and DBE means a 3,5-dinitrobenzyl ester) were synthesized in accordance with the method described in Non-Patent Documents 13 and 15, and Reference Literature 3.

An aminoacyl-tRNA was prepared in the following manner. ADDIN EN. CITE ADDIN EN. CITE. DATA. After 12 μL of a HEPES-KOH buffer (pH 7.5, 83 mM) containing 42 μM tRNA and 42 μM flexizyme (eFx for a CME-activated amino acid and dFx for a DBE activated amino acid) was heated at 95°C for 2 minutes, the reaction mixture was cooled to 25°C for 5 minutes. Then, 3 M $MgCl_2$ (4 μL) was added, and the resulting mixture was incubated at 25°C for 5 minutes.

The reaction was started by addition of an activated amino acid substrate in 25 mM DMSO (4 μL), followed by incubation on ice for acylation (incubation time: 2 hours for Phe-CME, Tyr-CME, $^{Me}$G-DBE, $^{Me}$A-DBE, and $^{Ac}$K-DBE; 6 hours for Ser-DBE, $^{Me}$S-DBE, $^{Me}$F-CME, $^{Me}$L-DBE, $^{Me}$M-DBE, $^{Me}$M-DBE, and $^{Me}$Ym-CME; and 10 hours for $^{Me}$T-DBE, $^{Me}$D-DBE, $^{Me}$V-DBE, $^{Me}$V-DBE$^{Me}$NI-DBE, and $^{Me}$Nv-DBE). After the acylation, the reaction was terminated by the addition of 80 μL of 0.3 M sodium acetate (pH 5.2) and the RNA was precipitated by ethanol. The pellet was washed twice with 70% ethanol containing 0.1 M sodium acetate (pH 5.2) and once with 70% ethanol. The aminoacyl-tRNA thus obtained was dissolved in 1 mM sodium acetate (pH 5.2) immediately before addition to a translation solution.

Measurement of the efficiency of flexizyme-catalyzed aminoacylation

The pellet of ethanol-precipitated 50 pmol tRNA precharged with pAA or npAA was dissolved in 0.52 μL of 10 mM sodium acetate (pH 5.2) and mixed with 5.0 μL of acid PAGE loading buffer (83% (v/v) formamide, 150 mM sodium acetate (pH 5.2), and 10 mM EDTA). The resulting solution was loaded on an acid denaturing polyacrylamide gel (12% (w/v) acrylamide/bisacrylamide (19:1), 8 M urea, and 50 mM sodium acetate (pH 5.2)) and electrophoresis was performed at 300 V (approximately 10 V/cm) for 20 hours. The resulting gel was stained with ethidium bromide and analyzed using a Typhoon FLA 7000 (GE Healthcare). The aminoacylation efficiency was calculated based on the band intensity of the aminoacyl-tRNA (A) and free tRNA (T) and presented as (A)/[(A)+(T)].

Radiolabeling of 5'-end or 3'-end of tRNA

The 5'-end of the tRNA was radiolabeled in the following manner. A dephosphorylated reaction mixture (4 μL) (1 μM tRNA, 50 mM Bis-Tris-Propane-HCl (pH 6.0), 1 mM $MgCl_2$, 0.1 mM $ZnCl_2$, and 0.5 U Antarctic Phosphatase (New England BioLabs)) was incubated at 37°C for 30 minutes and then incubated at 70°C for 5 minutes. The solution (4 μL) was mixed with 9.1 μL of water, 2.0 μL of a 10x buffer (700 mM Tris-HCl (pH 7.6), 100 mM $MgCl_2$, and 50 mM DTT), 2.9 μL of 1.7 μM [γ-$^{32}$P]-ATP, and 2.0 μL of 10 U/μL T4 Polynucleotide Kinase (New England BioLabs). The resulting 20 μL Kinase reaction mixture was incubated at 37°C for 60 minutes. The $^{32}$P labeled tRNA (4 pmol) was mixed with an unlabeled tRNA (121 pmol). The tRNA was purified by phenol/chloroform extraction and ethanol precipitation and then, dissolved in 10 μL of water. The concentration of the tRNA was determined using $A_{260}$ of a 50-fold diluted solution.

The 3'-end of the tRNA was radiolabeled in the following manner. After 7.5 μL of 16.7 μM tRNA lacking a 3'-end adenosine was incubated at 80°C for 3 minutes, it was placed on ice for 10 minutes. Then, 25 μL of a CCA-added reaction mixture (120 mM Gly-NaOH (pH 9.0), 75 mM $MgCl_2$, 30 mM DTT, 5 μM of the tRNA lacking the 3'-end adenosine prepared above, 10 mM non-radiolabeled ATP, 0.67 μM [α-$^{32}$P]-ATP and 200 nM CCA-adding enzyme) was incubated at 37°C for 20 minutes. The tRNA was purified by phenol/chloroform extraction, Micro Bio-Spin 30 column (Bio-Rad), and ethanol precipitation and then, dissolved in 5 μL of water. The concentration of the tRNA was determined using $A_{260}$ of a 100-fold diluted solution.

Quantification of affinity between EF-Tu and aminoacyl-tRNA

The aminoacyl-tRNA was prepared as described above except that the acylation reaction was performed using a mixture of the 3'-radiolabeled tRNA (10%) and a non-radiolabeled tRNA (90%). The aminoacyl-tRNA pellets thus obtained were dissolved in 3.2 μL of 10 mM sodium acetate (pH 5.2). The concentration of the aminoacyl-tRNA was adjusted as follows. The aminoacyl-tRNA solution (0.7 μL) was diluted 100-fold with 69.3 μL of 10 mM sodium acetate (pH 5.2) and the concentration of the tRNA and flexizyme was determined using $A_{260}$ of the diluted solution. The concentration of the aminoacyl-tRNA was calculated based on the tRNA concentration and the flexizyme-catalyzed acylation efficiency. The concentration of the aminoacyl-tRNA was adjusted to 2.0 μM by adding an adequate amount of 10 mM sodium acetate (pH 5.2). Immediately before use, the concentration of the aminoacyl-tRNA was adjusted to 100 nM by mixing the 2.0 μM aminoacyl-tRNA solution with a buffer A (50 mM HEPES-KOH (pH 7.6), 100 mM KOAc, 12 mM $Mg(OAc)_2$, 1 mM GTP, 1 mM DTT, 20 mM creatine phosphate, 2 mM spermidine, 3 mM phosphoenolpyruvic

acid, and 0.1 μg/μL of pyruvate kinase from rabbit muscle (Sigma-Aldrich)).

The EF-Tu stored in a GDP-bound form was converted into a GTP-bound form by incubating an EF-Tu solution in the buffer A at 37°C for 30 minutes. Samples usually containing EF-Tu at concentrations different in a range of 1.5 nM to 25 μM were prepared on ice by twofold serial dilution. The EF-Tu solution (9.6 μL) was mixed with 2.4 μL of 100 nM aminoacyl-tRNA and the resulting mixture was incubated on ice for 20 minutes. Under equilibrium binding conditions, 10 μL of each of the solutions was mixed on ice with 1 μL of 1 mg/mL RNase A (Sigma-Aldrich). Twenty seconds later, 50 μL of 10% (v/v) trichloroacetic acid (TCA) containing 0.1 mg/mL of an unfractionated yeast tRNA was added onto ice to quench the reaction. The precipitate was filtered through a nitrocellulose membrane which had a pore size of 0.45 μM and was assembled in a Bio-Dot microfiltration apparatus (Bio-Rad Laboratories), followed by washing 6 times with 200 μL of 5% (v/v) TCA. The membrane was soaked in 95% (v/v) ethanol for 5 minutes and the dried membrane was analyzed by autoradiography using Typhoon FLA 7000 (GE Healthcare). To correct for the background signal derived from an aminoacyl-tRNA that would remain after 20-second treatment with RNase A, the membrane was analyzed concurrently with one without EF-Tu, and the radioactivity of it was subtracted from the experiment data. The resulting radioactivity was converted into a concentration of a ternary complex by using a conversion coefficient determined using a calibration aminoacyl-tRNA sample in respective buffers A containing 20, 4, 0.8 and 0.16 nM tRNA. The equilibrium dissociation constant was determined by fitting the binding data to the following formula (3) by using KaleidaGraph Program (Hulinks Inc.).

The $K_D$ formula is defined as follows and in the formula, aatRNA, EFTu, and aatRNA-EFTu represent aminoacyl-tRNA, EF-Tu and complexes thereof, respectively.

Formula (1)

[Equation 1]

$$K_D = \frac{[aatRNA]_{eq} \times [EFTu]_{eq}}{[aatRNA \bullet EFTu]_{eq}}$$

Formula (2)

[Equation 2]

$$K_D = \frac{([aatRNA]_{input} - [aatRNA \bullet EFTu]_{eq}) \times ([EFTu]_{input} - [aatRNA \bullet EFTu]_{eq})}{[aatRNA \bullet EFTu]_{eq}}$$

Formula (3)

[Equation 3]

$$[aatRNA \bullet EFTu]_{eq} = \frac{\left([aatRNA]_{input} + [EFTu]_{input} + K_D - \sqrt{([aatRNA]_{input} + [EFTu]_{input} + K_D)^2 - 4 \times [aatRNA]_{input} \times [EFTu]_{input}}\right)}{2}$$

The concentration of the aminoacyl-tRNA varied slightly in every experiment due to its multistep preparation, so that both $K_D$ and the aminoacyl-tRNA concentration were set as variables in the fitting analysis.

In Vitro translation

The reconstituted cell-free translation system (Reference Literature 4) contained all the components necessary for translation except for RF1. The concentrations of the translation components are optimized as follows (Reference Literature 2) insofar as each drawing does not include a particular description: 50 mM HEPES-KOH (pH 7.6), 100 mM KOAc, 2 mM GTP, 2 mM ATP, 1 mM CTP, 1 mM UTP, 20 mM creatine phosphate, 12 to 20 mM $Mg(OAc)_2$, 2 mM spermidine, 1 mM DTT, 100 μM 10-formyl-5,6,7,8-tetrahydrofolate, 1.2 μM ribosome, 2.7 μM IF1, 0.4 μM IF2, 1.5 μM IF3, 10 μM EF-Tu, 10 μM EF-Ts, 0.26 μM EF-G, 0.25 μM RF2, 0.17 μM RF3, 0.5 μM RRF, 0.6 μM MTF, 4 μg/mL creatine kinase, 3 μg/mL myokinase, 0.1 μM pyrophosphatase, 0.1 μM nucleoside diphosphate kinase, 0.1 μM T7 RNA polymerase, 0.73 μM AlaRS, 0.03 μM ArgRS, 0.38 μM AsnRS, 0.13 μM AspRS, 0.02 μM CysRS, 0.06 μM GlnRS, 0.23

μM GluRS, 0.09 μM GlyRS, 0.02 μM HisRS, 0.4 μM IleRS, 0.04 μM LeuRS, 0.11 μM LysRS, 0.03 μM MetRS, 0.68 μM PheRS, 0.16 μM ProRS, 0.04 μM SerRS, 0.09 μM ThrRS, 0.03 μM TrpRS, 0.02 μM TyrRS, 0.02 μM ValRS, 1.5 mg/mL native E. coli tRNA mixture (Roche), 200 μM each 3 to 20 pAA, 10-50 μM $tRNA^{AsnE2}$s charged with each npAA and either 0.25 μM mDNA or 6.0 μM mRNA.

The translation products were analyzed by MALDI-TOF-MS analysis and/or autoradiography after tricine-SDS-PAGE. In MALDI-TOF MS analysis, 1.0 to 10 μL of the translation reaction mixture was incubated at 37°C for 3 to 120 minutes. After the reaction, the mixture was diluted with the same volume of a FLAG purification buffer (100 mM Tris-HCl (pH 7.6) and 300 mM NaCl). The expressed peptide was immobilized on anti-FLAG M2 agarose beads (Sigma-Aldrich) by incubating at 25°C for 1 hour. After washing the beads with 25 μL of wash buffer (50 mM Tris-HCl (pH 7.6) and 150 mM NaCl), the immobilized peptide was eluted with 15 μL of 0.2% TFA. After the purification, the peptide was desalted with SPE C-TIP (Nikkyo Technos) and eluted with 1 μL of 80% acetonitrile, 0.5% acetic acid solution 50% saturated with the matrix (R)-cyano-4-hydroxycinnamic acid (Bruker Daltonics). The MALDI-TOF MS measurement was performed using a ultraflextreme (Bruker Daltonics) under reflector/positive-mode and externally calibrated with peptide calibration standard II (Bruker Daltonics) and/or protein calibration standard I (Bruker Daltonics). For the autoradiographic analysis, the translation reaction was performed in the presence of 50 μM [$^{14}$C]-Asp instead of 200 μM nonradioactive Asp. The translation product was analyzed by 15% tricine-SDS-PAGE and autoradiography using Typhoon FLA\7000 (GE Healthcare) without FLAG purification. The amount of the peptide product was quantified based on the relative band intensity of the peptide product to the sum intensities present in the lane (that is, the sum intensities of unreacted free Asp and peptide product).

The method of preparing a $^{Me}$AA-tRNA was changed as follows for the translation of mRNAs 12 to 14 (Figs. 2, 4 to 7, and 9). First, the ethanol precipitate after flexizyme-catalyzed acylation was dissolved in 300 μL of 0.3 M sodium acetate (pH 5.2) and precipitated by second ethanol precipitation. This precipitation was performed to wash away a coprecipitated Mg salt. The pellet was washed twice with 70% ethanol containing 0.1 M sodium acetate (pH 5.2) and once with 70% ethanol. The resulting aminoacyl-tRNA was dissolved in 1 mM sodium acetate (pH 5.2) immediately before addition to a translation solution. The concentration of the aminoacyl-tRNA was adjusted as follows: 0.25 μL of the aminoacyl-tRNA solution was diluted 5000-fold with 1250 μL of 1 mM sodium acetate (pH 5.2) and the concentration of the tRNA and the flexizyme was determined using $A_{260}$ of the diluted solution. The concentration of the aminoacyl-tRNA was calculated based on the tRNA concentration and the flexizyme-catalyzed acylation efficiency.

Experimental evidence showing that many $^{Me}$AA-tRNAs do not bind to EF-Tu with sufficient intensity

First, EF-Tu affinities of standard pAA-tRNA and those of $^{Me}$AA-tRNA were compared. As a control, three standard pAA-tRNAs (Phe-$tRNA^{Phe}$, Tyr-$tRNA^{Tyr}$, and Ser-$tRNA^{Ser}$) were prepared using a flexizyme (Non-Patent Document 13) catalyst, that is, an artificial aminoacylation ribozyme and their EF-Tu affinities were quantified by RNase A protection assay. As reported previously, they showed similar affinity in a range of -8 to -9.4 kcal/mol (Fig. 2a). The affinity values observed were slightly lower than those previously reported (-9.5 to -10.5 kcal/mol) (Reference Literature 5). The difference was thought to occur due to a difference in the composition of a buffer or the presence of a His6 tag at the C terminal of the EF-Tu used in the present study. For the determination of the EF-Tu affinity of $^{Me}$AA-tRNA, 14 kinds of $^{Me}$AAs were charged to orthogonal $tRNA^{AsnE2}_{GAC}$ and the body sequence of it was engineered to avoid aminoacylation by endogenous AARS (Non-Patent Document 14 and Reference Literatures 2 and 6) by using flexizyme-catalyzed aminoacylation. In contrast to the standard pAA-tRNAs, many $^{Me}$AA-$tRNA^{AsnE2}_{GAC}$s other than $^{Me}$G-$tRNA^{AsnE2}_{GAC}$, $^{Me}$S-$tRNA^{AsnE2}_{GAC}$ and $^{Me}$A-$tRNA^{AsnE2}_{GAC}$ showed an undetectably weak EF-Tu affinity level (Fig. 2b). Considering that the α-amino group of the charged amino acid is housed in a pocket composed of an EF-Tu residue (Fig. 2c), the N-methyl modification is presumed to cause steric hindrance between the $^{Me}$AA and the pocket, leading to deterioration in EF-Tu affinity. The three exceptions, that is, $^{Me}$G-$tRNA^{AsnE2}_{GAC}$, $^{Me}$S-$tRNA^{AsnE2}_{GAC}$, and $^{Me}$A-$tRNA^{AsnE2}_{GAC}$C have a possibility of alleviating the steric hindrance by locating their side chains (-H, $-CH_3$, and -OH groups) in the pocket.

Next, the ribosomal synthesis of a model peptide (Fig. 2d) containing a single $^{Me}$AA was performed using six kinds of $^{Me}$AA-$tRNA^{AsnE2}_{GAC}$s by an improved in vitro translation system called FIT (Flexible In vitro Translation) system (Non-Patent Documents 13 to 16 and Reference Literatures 2 and 3). The system depends on a reconstituted E. coli cell-free translation system (Reference Literature 4) supplemented with a synthetic tRNA pre-charged with npAA by the catalytic action of flexizyme. The mRNA template was designed to determine the translation accuracy by MALDI-TOF-MS after purification by an anti-FLAG M2 agarose. The genetic code of a GUC codon was reprogrammed by excluding the corresponding Val from the FIT system and supplementing one of $^{Me}$AA-$tRNA^{AsnE2}_{GAC}$s instead. When $^{Me}$G-$tRNA^{AsnE2}_{GAC}$ and $^{Me}$S-$tRNA^{AsnE2}_{GAC}$ showing strong EF-Tu affinity were used, an correct peptide was observed as a single major peak in MALDI-TOF-MS. On the other hand, when $^{Me}$AA-$tRNA^{AsnE2}_{GAC}$ having weak EF-Tu affinity was used, an undesirable byproduct containing either one of Leu or Ile instead of $^{Me}$AA was detected in addition to the correct peptide product in MALDI-TOF-MS (Fig. 2d). As an exception, $^{Me}$F-$tRNA^{AsnE2}_{GAC}$ did not show a detectable level of

EF-Tu affinity, but a single major peak was observed for $^{Me}$F. In a supplementary experiment, a byproduct was observed only when Ile was supplied in the FIT system, which has revealed that the misincorporated amino acid was not Leu but Ile (Fig. 7). Similar weak EF-Tu affinity and translation inaccuracy were observed in another type npAA, ε-N-acetyllysine ($^{Ac}$K), which was examined to confirm the universality of the present study (Figs. 2b and 2d). In conclusion, the present experiment indicated that the EF-Tu affinities of many $^{Me}$AA-tRNA$^{AsnE2}$$_{GAC}$s were significantly weaker than the mean value of standard pAA-tRNAs and this weak EF-Tu affinity often caused translation inaccuracy during ribosomal polypeptide synthesis.

Development of a method for reinforcing the weak EF-Tu affinity of $^{Me}$AA-tRNA

Since the fact that many $^{Me}$AA-tRNAs did not bind to EF-Tu with sufficient intensity was discovered, development of a novel method for reinforcing the weak EF-Tu affinity of $^{Me}$AA-tRNA was encouraged. It has been reported that binding affinity between EF-Tu and Phe-tRNA$^{Phe}$ changes depending on its T-stem sequence (Reference Literature 7), so that the present inventors have thought that the EF-Tu affinity of $^{Me}$AA-tRNA can be changed by using a different T-stem sequence. Based on this concept, three T-stem sequences (T-stems No. 1, No. 3, and No.4) were selected (Fig. 3a, Reference Literature 7). The original T-stem shown in Fig. 3a was renamed as T-stem No. 2. Based on the previous studies, the present inventors considered that the EF-Tu affinity of those four $^{Me}$AA-tRNAs showed an increase with an increase in the number of the T-stems from No. 1, 2 to 4.

The feasibility of the affinity reinforcement by T-stem substitution was confirmed using Phe-tRNA$^{AsnE2}$$_{GAC}$ and $^{Me}$F-tRNA$^{AsnE2}$$_{GAC}$. When the T-stem sequence of Phe-tRNA$^{snE2}$$_{GAC}$ was substituted by another T-stem sequence, the EF-Tu affinity changed in an expected order (Fig. 3b). In $^{Me}$F-TRNA$^{AsnE2}$$_{GAC}$, a similar change was also observed in the EF-Tu affinity of T-stem sequences No. 2, No. 3, and No. 4 (Fig. 3c). A control test using an uncharged tRNA$^{AsnE2}$$_{GAc}$ has revealed that the aforesaid tRNAs do not bind to EF-Tu insofar as they are not charged with an amino acid (Fig. 8).

The effect of EF-Tu affinity reinforcement on the translation accuracy and efficiency was studied using Phe-tRNA$^{AsnE2}$$_{GAC}$ and $^{Me}$F-tRNA$^{AsnE2}$$_{GAC}$. An mRNA template was designed to determine the peptide expression level by tricine-SDS-PAGE (autoradiographic detection of [$^{14}$C]-Asp in a C-terminal FLAG peptide region) and decoding accuracy was evaluated by MALDI-TOF-MS after purification using an anti-FLAG M2 agarose (Figs. 4a and 4b). When the T-stem No. 1 was used, a byproduct containing Ile instead of Phe and $^{Me}$F was observed in MALDI-TOF-MS, but only an expected peptide was obtained when the tRNAs having the relatively strong T-stem were used (Fig. 4a and Fig. 4b). When the translation efficiency of a Phe-containing peptide was quantified using an Ile-free FIT system, a synthesis rate decreased with an increase in the EF-Tu affinity (Fig. 4c). The decrease in synthesis rate is presumed to occur because (1) Phe-tRNA$^{AsnE2}$$_{GAC}$ having a stronger T-stem might bind to EF-Tu in preference and disturb the binding of canonical pAA-tRNA and thereby decrease the synthesis rate of a full-length peptide; or (2) the too strong interaction between EF-Tu and the substituted T-stem might disturb the rapid delivery of aminoacyl-tRNA from EF-Tu to a ribosome as observed in the previous study (Reference Literatures 8 and 9). To specify a correct reason, further research is necessary. In the case of $^{Me}$F-tRNA$^{AsnE2}$$_{GAC}$, the synthesis rate was highest when the T-stems No. 2 and No. 3 were used, compared with the use of T-stem No. 1 or No. 4 (Fig. 4d). This suggests that the T-stem No. 1 is too weak for $^{Me}$F-tRNA$^{AsnE2}$$_{GAC}$. In conclusion, the present results have proved that the EF-Tu affinity of the aminoacyl-tRNA can be drastically changed by the T-stem substitution strategy and selection of the best T-stem from four candidates enables improvement in translation accuracy and efficiency. The influence of the EF-Tu affinity on the translation accuracy was also studied in the other four $^{Me}$AA-tRNA$^{AsnE2}$$_{GAC}$ and $^{Ac}$K-tRNA$^{AsnE2}$$_{GAC}$ (Fig. 9). In all the examples, the reinforcement of the EF-Tu affinity was achieved by the T-stem substitution, and therefore, the translation accuracy was improved. Particularly, when $^{Me}$L and $^{Ac}$K were incorporated, a byproduct was observed as a major peak when T-stem No. 2 was used, but production of an undesirable byproduct was suppressed by substituting the T-stem sequence by No. 4 sequence. The reinforcement of the EF-Tu affinity was confirmed also in the other eight kinds of $^{Me}$AA-tRNA$^{AsnE2}$$_{GAC}$s (Fig. 10).

Expression of highly N-methylated peptide via tuning of EF-Tu affinity of $^{Me}$AA-tRNA

Next, to prove the improved the N-methyl peptide synthesis by the EF-Tu affinity tuning strategy, expression of highly N-methylated peptides respectively containing 6 different $^{Me}$AAs was attempted. In the present experiment, two improvements were performed. First, the EF-Tu affinity of $^{Me}$AA-tRNAs was adjusted to fall in a range of that of pAA-tRNAs by selecting the T-stem sequence suited for individual $^{Me}$AA-tRNAs (Fig. 5a). Under the conditions, EF-Tu can bind to any of pAA-tRNAs and $^{Me}$AA-tRNAs with equivalent affinity. Secondly, the concentration of each of the $^{Me}$AA-tRNAs was adjusted based on the flexizyme catalyzed acylation efficiency (typically, 20 to 50%) and the concentration of the pre-charged $^{Me}$AA-tRNAs was adjusted to 10 μM, while in a conventional method, 50 μM of each of the pre-charged tRNAs with $^{Me}$AA was added without considering the acylation efficiency (Fig. 5b). In the present experiment, a model peptide composed of 14 pAAs and 6 $^{Me}$AAs was expressed in the FIT system, and 6 $^{Me}$AA-tRNAs ($^{Me}$Y-tRNA$^{AsnE2TS3}$$_{GAA}$, $^{Me}$A-tRNA$^{AsnE2TS3}$$_{GAU}$, $^{Me}$F-tRNA$^{AsnE2TS3}$$_{GGC}$, $^{Me}$V-tRNA$^{AsnE2TS1}$$_{GAC}$, $^{Me}$G-tRNA$^{AsnE2TS5}$$_{CAG}$, and

$^{Me}$S-tRNA$^{AsnE2TS5}$$_{GAG}$) were used instead of the excluded 5 pAAs (Phe, Leu, Ile, Val, and Ala) (Fig. 5c and Fig. 5d). When the conventional system (Reference Literature 6) was used for the translation, a mixture of a desired peptide containing pAA instead of $^{Me}$AA with various byproducts was synthesized, and an expression level of the mixture was 0.7 μM in total (Fig. 5e and Fig. 5f). On the other hand, the optimized system developed in the present study produced only a desired peptide having an improved expression level 1.8 μM. The results have shown that a translation disorder may be caused by poor EF-Tu affinity of $^{Me}$AA-tRNA and that the affinity tuning strategy drastically improves the synthesis efficiency and accuracy of N-methyl peptides.

Next, the number of the $^{Me}$AAs was increased further to 9, and a 32-mer model peptide P14 composed of 9 respectively different $^{Me}$AAs and 14 pAAs were expressed (Fig. 6a to Fig. 6c). In the present experiment, 3 kinds of $^{Me}$AAs having an unnatural side chain were added to the repertoire. Compared with control translation using 20 pAAs, the P14 peptide having 9 N-methyl modifications was expressed at 34% efficiency relative to the control having maintained translation accuracy, as confirmed by MALDI-TOF-MS (Fig. 6d and Fig. 6e). The results have proved that the EF-Tu affinity tuning strategy drastically increases the number of simultaneously usable $^{Me}$AAs to at most 9 and thereby enables the expression of a highly N-methylated peptide.

As shown in the results of the present Examples, the present invention can provide a novel method which achieves ribosomal synthesis of a non-standard peptide containing many kinds of $^{Me}$AAs.

First, the present Example has quantitatively proved that the binding affinity between EF-Tu and many $^{Me}$AA-tRNAs was weak (Fig. 2). This has supported our assumption that inefficient $^{Me}$AA incorporation is due to the disorder in EF-Tu mediated delivery of $^{Me}$AA-tRNA to a ribosome. The present Example has also indicated that the weak EF-Tu affinity of $^{Me}$AA-tRNA can be reinforced by the T-stem substitution strategy (Fig. 3, Fig. 9, and Fig. 10). Particularly when the EF-Tu binding affinity was changed to a too intense value, a synthesis efficiency of a corresponding peptide decreased (Fig. 4), which was consistent with the previous report on the study of pAAs and fluorescent npAA (Reference Literatures 8 and 9).

Tuning of the affinity between EF-Tu and each $^{Me}$AA-tRNA has successfully improved the synthesis efficiency and accuracy of model peptides containing 6 respectively-different $^{Me}$AAs (Fig. 5). Further, the translation system developed herein has achieved the synthesis of a highly N-methylated peptide containing even 9 respectively-different $^{Me}$AAs (Fig. 6). This has increased the number of simultaneously usable $^{Me}$AAs from 3 to 9 (Reference Literatures 6 and 10). Particularly, 9 $^{Me}$AAs include $^{Me}$V which is reported as a substrate unsuited for ribosome polypeptide synthesis (Reference Literature 3). The diversity of 24 constitution blocks (15 pAAs and 9 $^{Me}$AAs) achieved by the present Example has overcome the previous restriction of 23 constitution blocks (20 pAAs and 3 npAAs) (Reference Literatures 11 and 12). These facts shown by the present Examples have clearly proved the improvement in synthesis efficiency and accuracy of a highly N-methylated peptide.

The npAA incorporation inefficiency is presumed to occur due to (1) the disorder in the EF-Tu-mediated delivery of a corresponding npAA-tRNA, (2) a slow peptidyl transfer reaction with which npAA is involved, and (3) the hindered ejection of a newly-formed npAA-containing polypeptide through an exit tunnel. In the related art, which process is a real cause for inefficient incorporation has not been identified, but the present invention has shown that the synthesis efficiency and accuracy of N-methylated peptide can be improved drastically by tuning the insufficient EF-Tu affinity of $^{Me}$AA-tRNA. This fact means that the same strategy is useful for promoting the efficient incorporation of abundant and multiple npAAs as well as $^{Me}$AAs. In the present Example, such an example is proved for $^{Ac}$K (Fig. 9).

The peptide translation system according to the present invention enables the synthesis of a highly N-methylated peptide library with sufficient accuracy and efficiency. Such integration of a peptide library and a peptide screening technique is expected to promote the discovery of practical non-standard peptide drugs having desirable pharmacological properties in future.

Reference Literature 1: Nissen, P. et al. Crystal structure of the ternary complex of Phe-tRNAPhe, EF-Tu, and a GTP analog. Science 270, 1464-1472 (1995).

Reference Literature 2: Goto, Y., Katoh, T. & Suga, H. Flexizymes for genetic code reprogramming. Nat Protoc 6, 779-790, doi:10.1038/nprot.2011.331 (2011).

Reference Literature 3: Kawakami, T., Murakami, H. & Suga, H. Messenger RNA-programmed incorporation of multiple N-methyl amino acids into linear and cyclic peptides. Chem. Biol. 15, 32-42, doi:10.1016/j.chembiol.2007.12.008 (2008).

Reference Literature 4: Shimizu, Y. et al. Cell-free translation reconstituted with purified components. Nat. Biotechnol. 19, 751-755, doi:10.1038/90802 (2001).

Reference Literature 5: LaRiviere, F. J., Wolfson, A. D. & Uhlenbeck, O. C. Uniform binding of aminoacyl-tRNAs to elongation factor Tu by thermodynamic compensation. Science 294, 165-168, doi:10.1126/science.1064242 (2001).

Reference Literature 6: Yamagishi, Y. et al. Natural product-like macrocyclic N-methyl-peptide inhibitors against a ubiquitin ligase uncovered from a ribosome-expressed de novo library. Chem. Biol. 18, 1562-1570, doi:10.1016/j.chembiol.2011.09.013 (2011).

Reference Literature 7: Schrader, J. M., Chapman, S. J. & Uhlenbeck, O. C. Understanding the sequence specificity of tRNA binding to elongation factor Tu using tRNA mutagenesis. J. Mol. Biol. 386, 1255-1264 (2009).
Reference Literature 8: Schrader, J. M., Chapman, S. J. & Uhlenbeck, O. C. Tuning the affinity of aminoacyl-tRNA to elongation factor Tu for optimal decoding. Proceedings of the National Academy of Sciences of the United States of America 108, 5215-5220, doi:10.1073/pnas.1102128108 (2011).
Reference Literature 9: Mittelstaet, J., Konevega, A. L. & Rodnina, M. V. A kinetic safety gate controlling the delivery of unnatural amino acids to the ribosome. J. Am. Chem. Soc. 135, 17031-17038, doi:10.1021/ja407511q (2013).
Reference Literature 10: Subtelny, A. O., Hartman, M. C. & Szostak, J. W. Ribosomal synthesis of N-methyl peptides. J. Am. Chem. Soc. 130, 6131-6136, doi:10.1021/ja710016v (2008).
Reference Literature 11: Ohtsuki, T., Manabe, T. & Sisido, M. Multiple incorporation of non-natural amino acids into a single protein using tRNAs with non-standard structures. FEBS Lett. 579, 6769-6774, doi:10.1016/j.febslet.2005.11.010 (2005).
Reference Literature 12: Iwane, Y. et al. Expanding the amino acid repertoire of ribosomal polypeptide synthesis via the artificial division of codon boxes. Nat Chem 8, 317-325, doi:10.1038/nchem.2446 (2016).

Sequence Listing Free Text

SEQ ID NOS: 1, 2 and 4 to 6 represent the base sequences of a T-arm.
SEQ ID NO: 3 represents the base sequences of a T-loop.
SEQ ID NOS: 123 to 125 represent the base sequences of mRNAs listed in Table 6.
SEQ ID NOS: 126 to 128 represent the base sequences of mRNAs in a translation region described in the drawing.
SEQ ID NOS: 129 to 133 represent the amino acid sequences of translated peptides.
SEQ ID NOS: 134 to 136 represent the base sequences of a D-arm.

## Claims

1. A tRNA having a T-stem in which $N_1N_2GGN_3$ and $N_4CCN_5U$ form a pair and encoding an N-methyl amino acid. ($N_1$ is G or A; one of $N_2$ and $N_5$ is G and the other one is C; and $N_3$ and $N_4$ are any amino acids forming a pair).

2. A translation system, comprising the tRNA as claimed in Claim 1 and an EF-Tu protein.

3. A method of producing a peptide library, comprising a step of translating with a cell-free translation system by using the tRNA as claimed in Claim 1 or the translation system as claimed in Claim 2.

4. A method of producing a complex library of a peptide and an mRNA encoding the peptide, comprising a step of translating with a cell-free translation system by using the tRNA as claimed in Claim 1 or the translation system as claimed in Claim 2.

5. The production method according to Claim 3 or 4, further comprising a step of preparing the tRNA as claimed in Claim 1 or the translation system as claimed in Claim 2, wherein in the preparation step, which tRNA is charged, a tRNA having a T-stem in which $N_1N_2GGN_3$ and $N_4CCN_5U$ form a pair or a tRNA other than the tRNA having a T-stem in which $N_1N_2GGN_3$ and $N_4CCN_5U$ form a pair is determined prior to charging with an N-methyl amino acid.

6. The production method according to Claim 5, comprising a step of preparing the tRNA as claimed in Claim 1 or the translation system as claimed in Claim 2, wherein in the preparation step, which tRNA is charged, a tRNA having a T-stem in which $AGGGN_3$ and $N_4CCCU$ form a pair and a tRNA having a T-stem in which $GCGGN_3$ and $N_4CCGU$ form a pair, or a tRNA other than the said tRNAs is determined prior to charging with an N-methyl amino acid.

7. The production method according to Claim 5, further comprising a step of preparing the tRNA as claimed in Claim 1 or the translation system as claimed in Claim 2, wherein in the preparation step, which tRNA is charged, a tRNA having $AGGGG(N_4)_mCCCCU$ (SEQ ID NO: 1) and a tRNA having $GCGGG(N_4)_mCCCGU$ (SEQ ID NO: 2), or a tRNA other than the said tRNAs is determined prior to charging with an N-methyl amino acid.

8. The production method according to any one of Claims 3 to 7, wherein a tRNA having, as an affinity between a tRNA charged with an N-methyl amino acid and EF-Tu, $\Delta G$ (kcal/mol) of -7.5 to -10 is selected and the tRNA thus selected is charged with the N-methyl amino acid.

9. A peptide library produced by the method as claimed in any one of Claims 3 and 5 to 8 or a peptide-mRNA complex library produced by the method as claimed in any one of Claims 4 to 8.

10. The peptide library or peptide-mRNA complex library according to Claim 9, comprising a peptide containing two or more N-methyl amino acids in one peptide structure.

## Figures

[Fig.1]

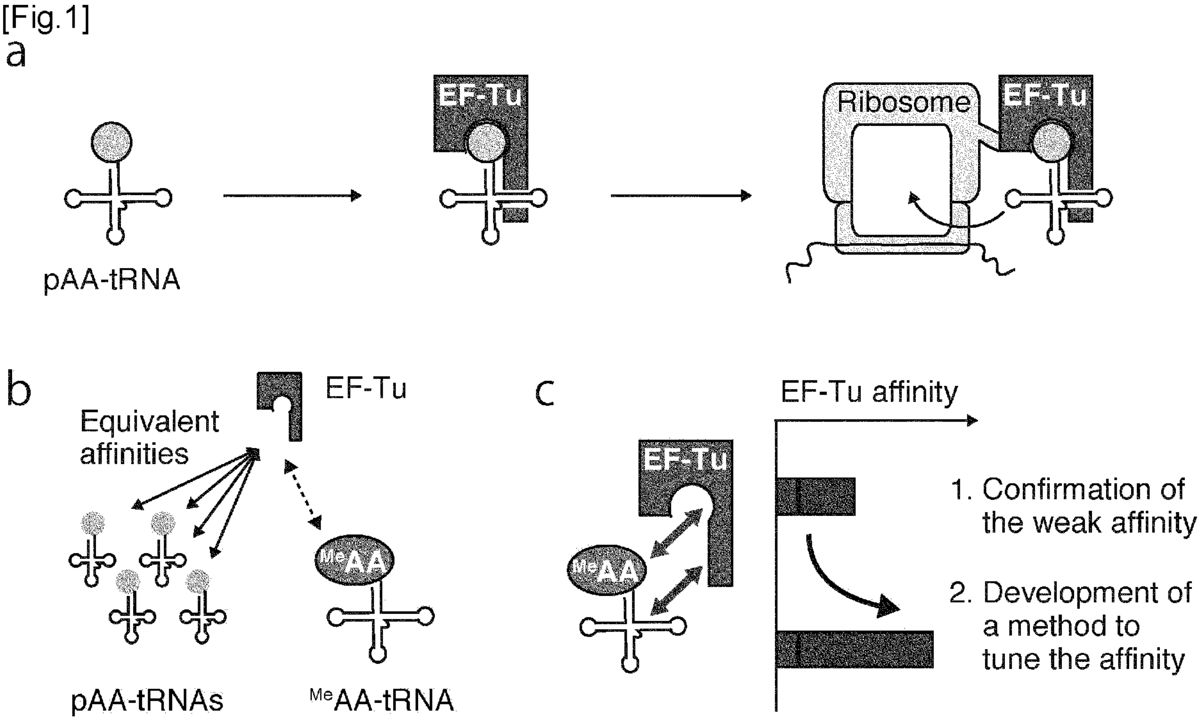


d

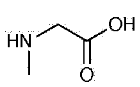
MeG

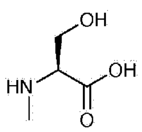
MeS

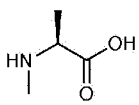
MeA

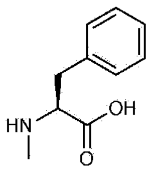
MeF

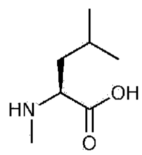
MeL

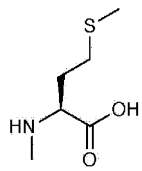
MeM

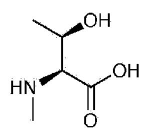
MeT

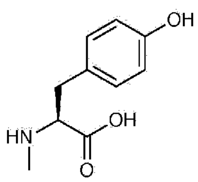
MeY

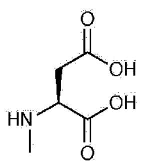
MeD

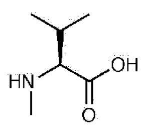
MeV

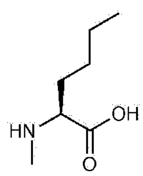
MeNl

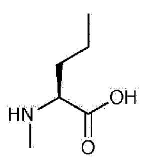
MeNv

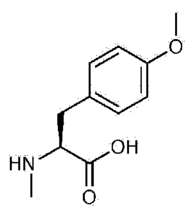
MeYm

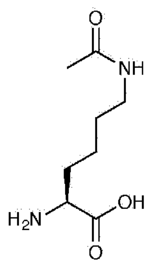
AcK

[Fig.2]

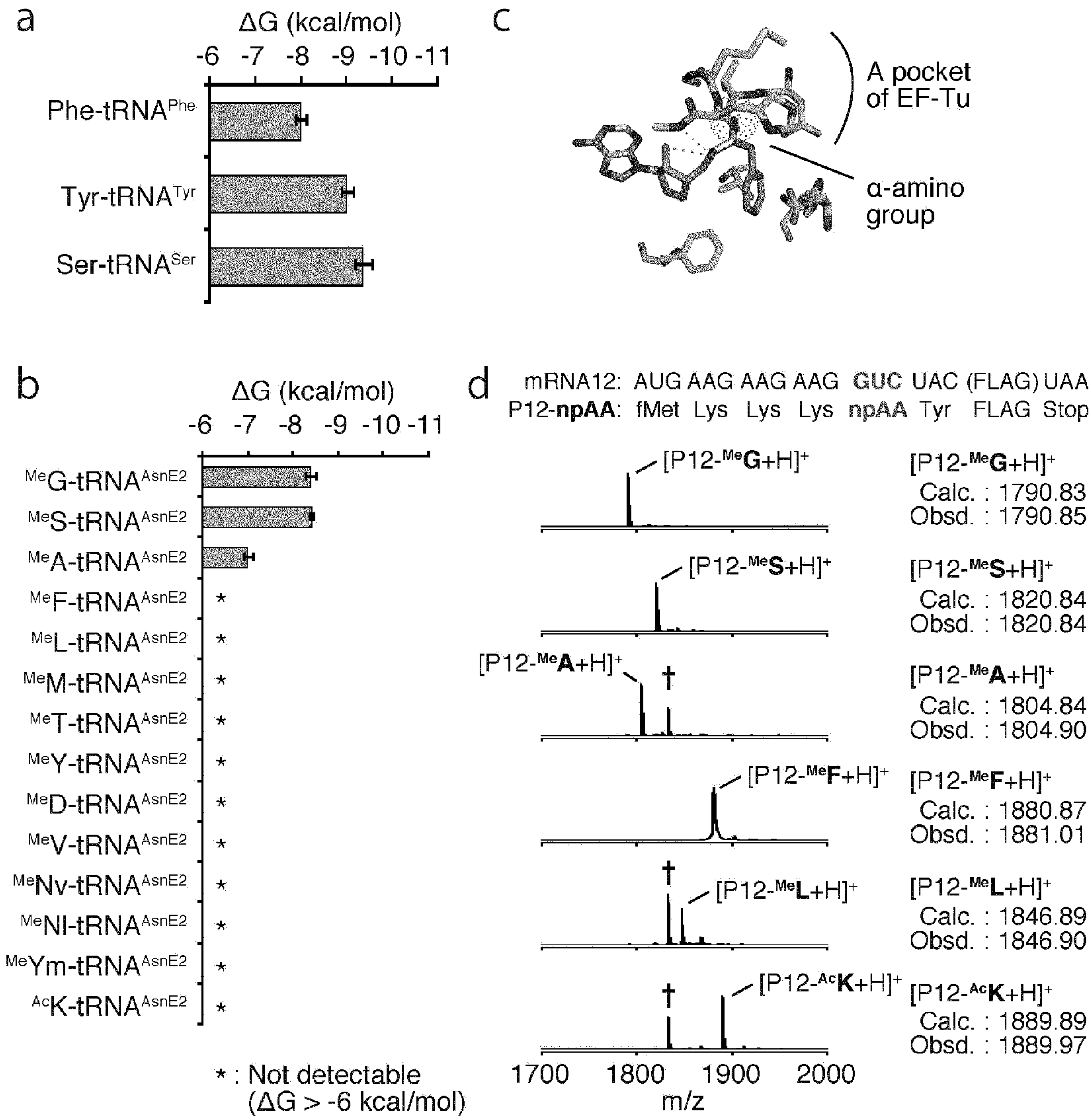
a
ΔG (kcal/mol)
-6 -7 -8 -9 -10 -11
Phe-tRNAPhe
Tyr-tRNATyr
Ser-tRNASer
c
A pocket of EF-Tu
α-amino group
b
ΔG (kcal/mol)
-6 -7 -8 -9 -10 -11
MeG-tRNAAsnE2
MeS-tRNAAsnE2
MeA-tRNAAsnE2
MeF-tRNAAsnE2
MeL-tRNAAsnE2
MeM-tRNAAsnE2
MeT-tRNAAsnE2
MeY-tRNAAsnE2
MeD-tRNAAsnE2
MeV-tRNAAsnE2
MeNv-tRNAAsnE2
MeNl-tRNAAsnE2
MeYm-tRNAAsnE2
AcK-tRNAAsnE2
* : Not detectable (ΔG > -6 kcal/mol)
d
mRNA12: AUG AAG AAG AAG GUC UAC (FLAG) UAA
P12-npAA: fMet Lys Lys Lys npAA Tyr FLAG Stop
[P12-MeG+H]+
Calc. : 1790.83
Obsd. : 1790.85
[P12-MeS+H]+
Calc. : 1820.84
Obsd. : 1820.84
[P12-MeA+H]+
Calc. : 1804.84
Obsd. : 1804.90
[P12-MeF+H]+
Calc. : 1880.87
Obsd. : 1881.01
[P12-MeL+H]+
Calc. : 1846.89
Obsd. : 1846.90
[P12-AcK+H]+
Calc. : 1889.89
Obsd. : 1889.97
†
1700 1800 1900 2000
m/z

[Fig.3]

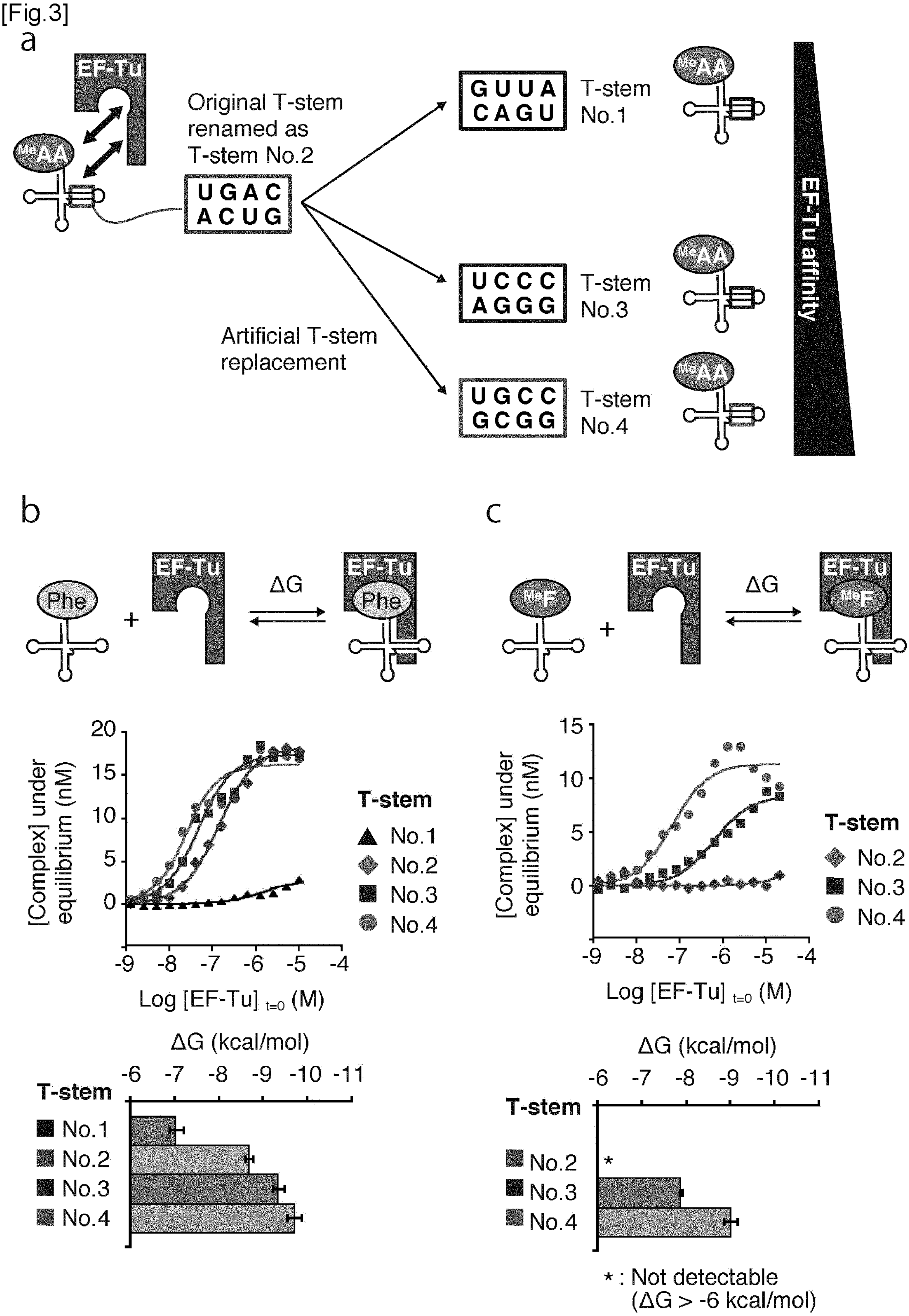
a
EF-Tu
MeAA
Original T-stem
renamed as
T-stem No.2
UGAC
ACUG
GUUA
CAGU
T-stem No.1
UCCC
AGGG
T-stem No.3
Artificial T-stem
replacement
UGCC
GCGG
T-stem No.4
EF-Tu affinity
b
Phe
ΔG
[Complex] under equilibrium (nM)
Log [EF-Tu] t=0 (M)
T-stem
No.1
No.2
No.3
No.4
ΔG (kcal/mol)
c
MeF
*
* : Not detectable
(ΔG > -6 kcal/mol)

[Fig.4]

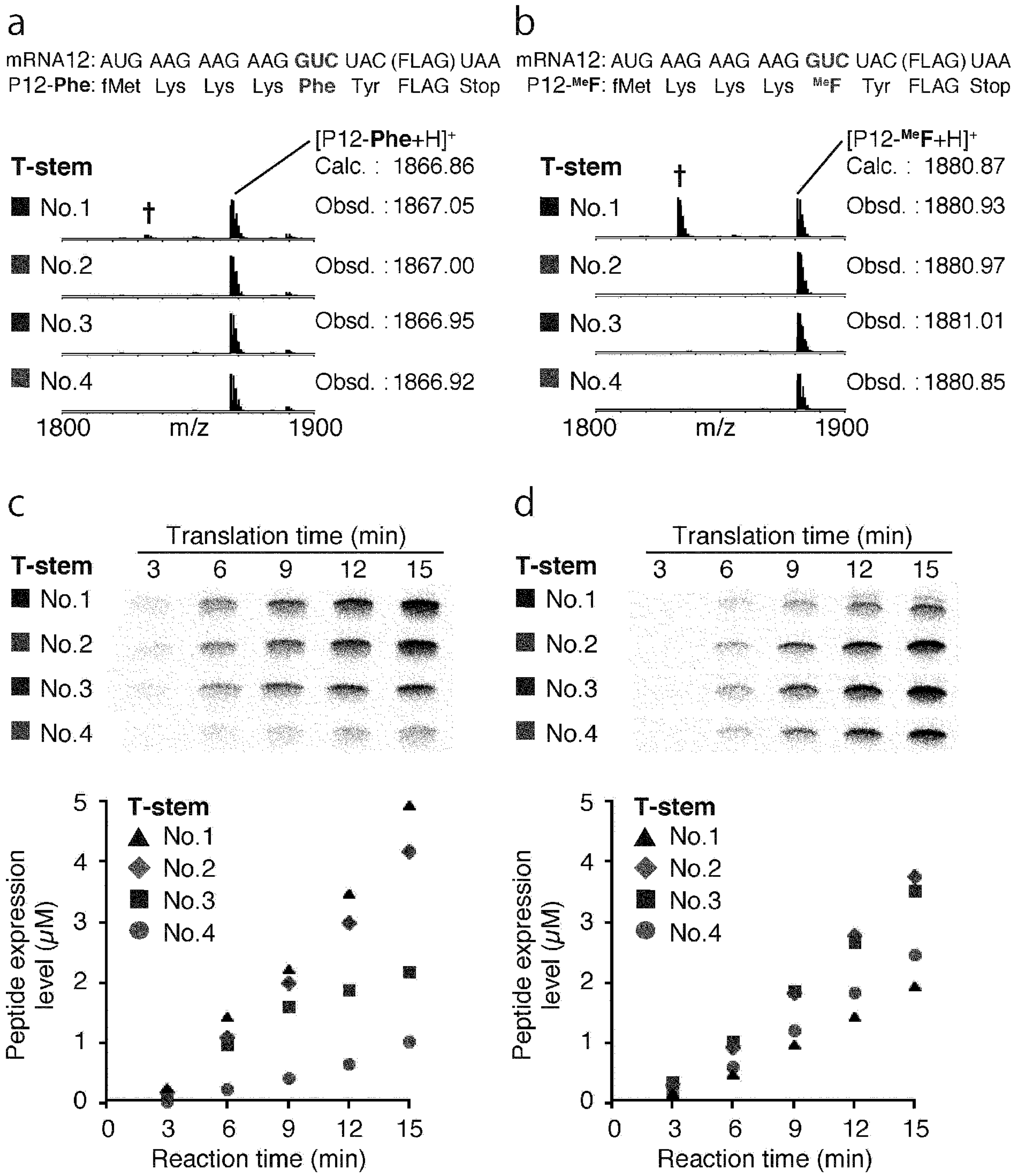
a
mRNA12: AUG AAG AAG AAG GUC UAC (FLAG) UAA
P12-Phe: fMet Lys Lys Lys Phe Tyr FLAG Stop
[P12-Phe+H]+
Calc. : 1866.86
T-stem
No.1
Obsd. : 1867.05
No.2
Obsd. : 1867.00
No.3
Obsd. : 1866.95
No.4
Obsd. : 1866.92
1800
m/z
1900
b
mRNA12: AUG AAG AAG AAG GUC UAC (FLAG) UAA
P12-MeF: fMet Lys Lys Lys MeF Tyr FLAG Stop
[P12-MeF+H]+
Calc. : 1880.87
Obsd. : 1880.93
Obsd. : 1880.97
Obsd. : 1881.01
Obsd. : 1880.85
c
Translation time (min)
3 6 9 12 15
d
Peptide expression level (μM)
Reaction time (min)

[Fig.5]

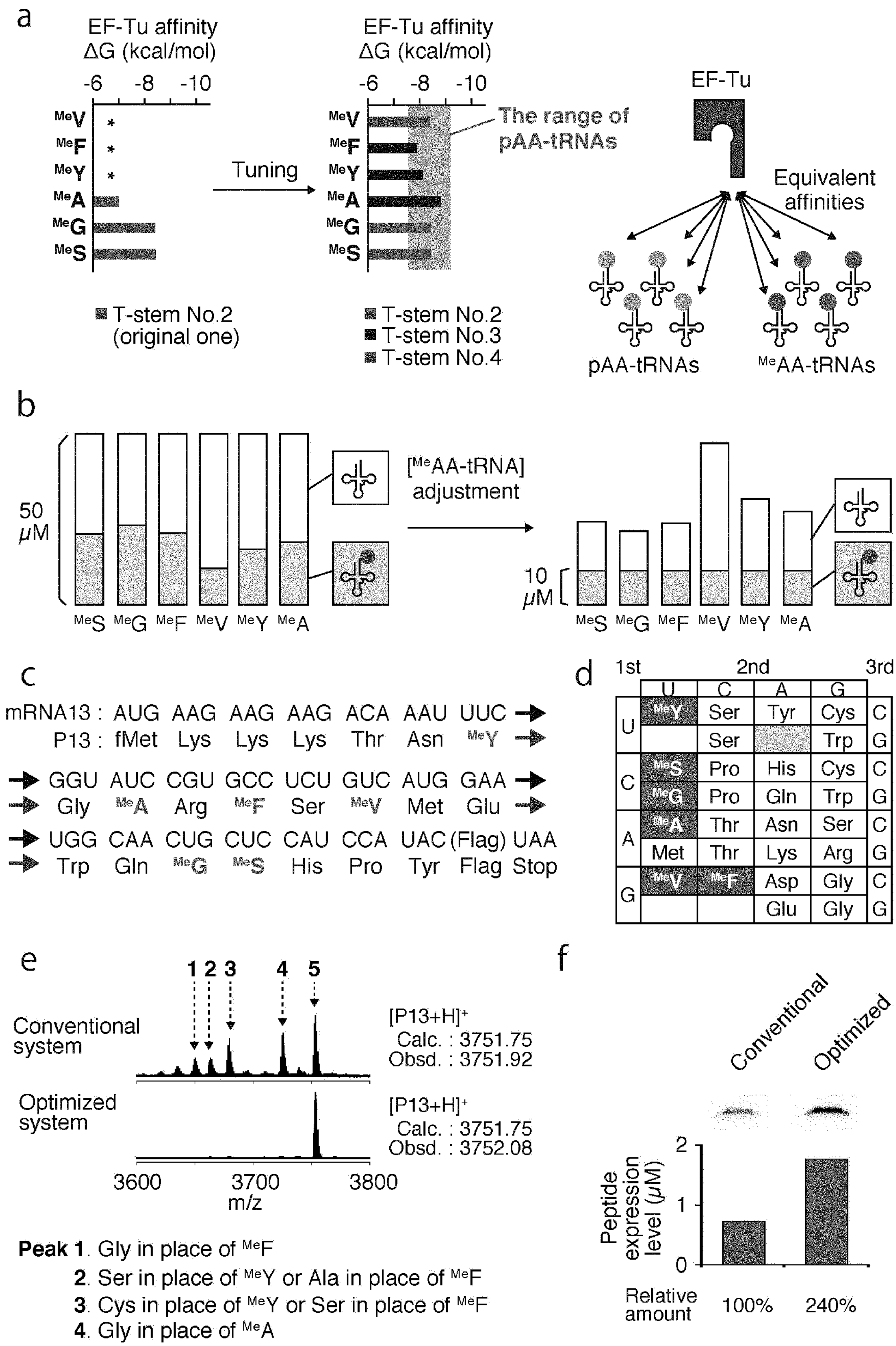


**Peak 1**. Gly in place of MeF

**2**. Ser in place of MeY or Ala in place of MeF

**3**. Cys in place of MeY or Ser in place of MeF

**4**. Gly in place of MeA

**5**. Desired P13 peptide

[Fig.6]

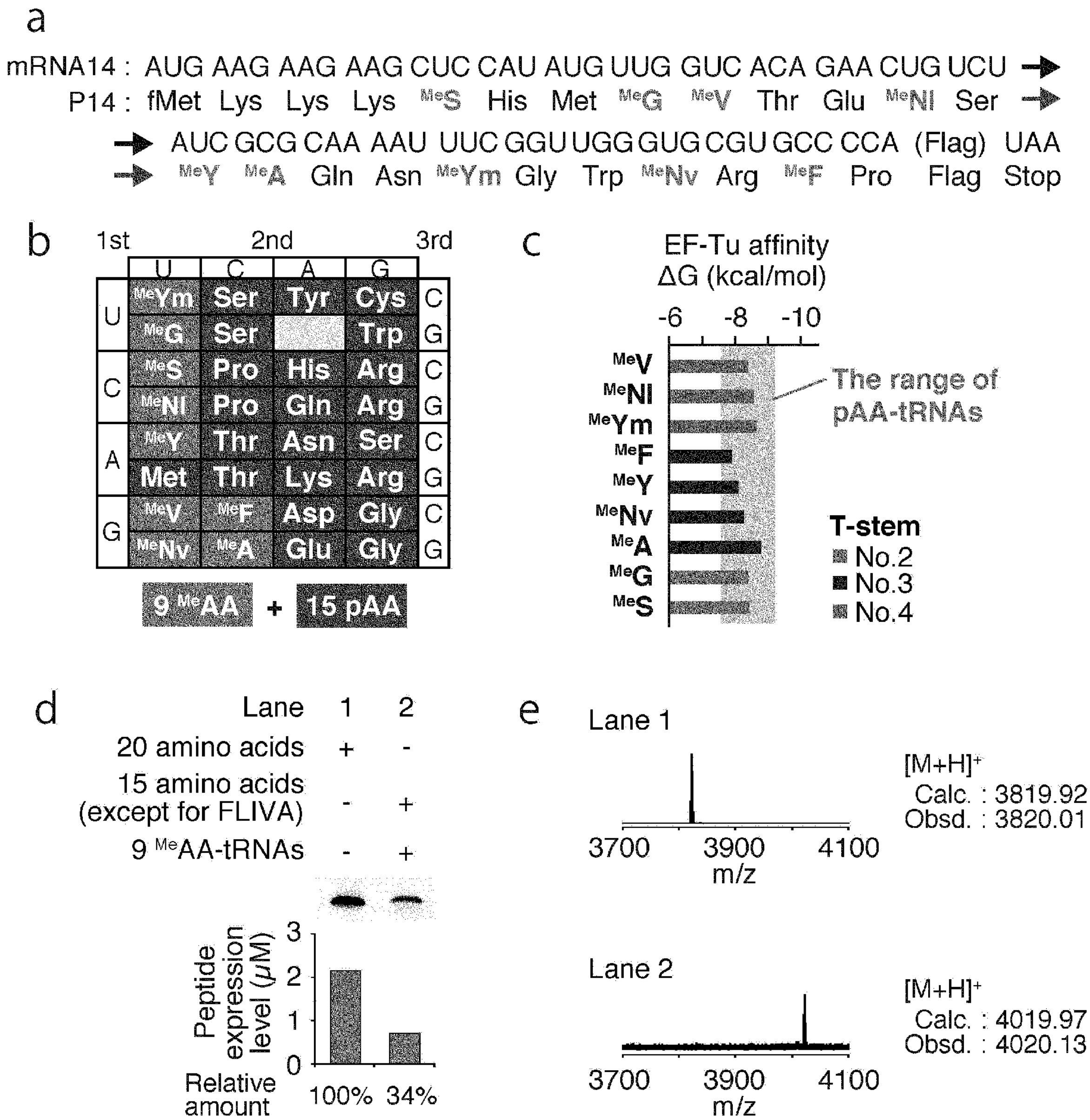
a
mRNA14 : AUG AAG AAG AAG CUC CAU AUG UUG GUC ACA GAA CUG UCU
P14 : fMet Lys Lys Lys MeS His Met MeG MeV Thr Glu MeNl Ser
AUC GCG CAA AAU UUC GGU UGG GUG CGU GCC CCA (Flag) UAA
MeY MeA Gln Asn MeYm Gly Trp MeNv Arg MeF Pro Flag Stop
b
1st
2nd
3rd
U C A G
MeYm Ser Tyr Cys
MeG Ser Trp
MeS Pro His Arg
MeNl Pro Gln Arg
MeY Thr Asn Ser
Met Thr Lys Arg
MeV MeF Asp Gly
MeNv MeA Glu Gly
9 MeAA + 15 pAA
c
EF-Tu affinity
ΔG (kcal/mol)
-6 -8 -10
MeV MeNl MeYm MeF MeY MeNv MeA MeG MeS
The range of pAA-tRNAs
T-stem
No.2
No.3
No.4
d
Lane 1 2
20 amino acids + -
15 amino acids (except for FLIVA) - +
9 MeAA-tRNAs - +
Peptide expression level (μM)
0 1 2 3
Relative amount 100% 34%
e
Lane 1
[M+H]+
Calc. : 3819.92
Obsd. : 3820.01
3700 3900 4100
m/z
Lane 2
[M+H]+
Calc. : 4019.97
Obsd. : 4020.13
3700 3900 4100
m/z

[Fig.7]

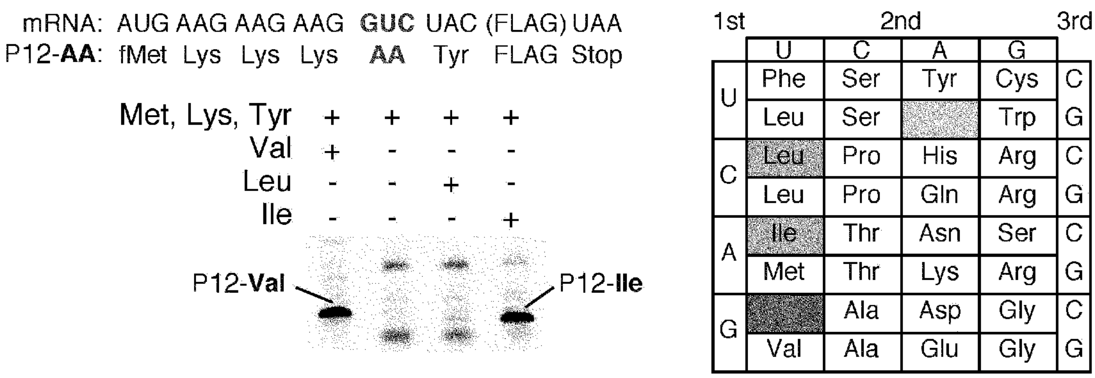
mRNA: AUG AAG AAG AAG GUC UAC (FLAG) UAA
P12-AA: fMet Lys Lys Lys AA Tyr FLAG Stop
Met, Lys, Tyr + + + +
Val + - - -
Leu - - + -
Ile - - - +
P12-Val
P12-Ile
1st
2nd
3rd
U C A G
Phe Ser Tyr Cys
Leu Ser Trp
Leu Pro His Arg
Leu Pro Gln Arg
Ile Thr Asn Ser
Met Thr Lys Arg
Ala Asp Gly
Val Ala Glu Gly

[Fig.8]

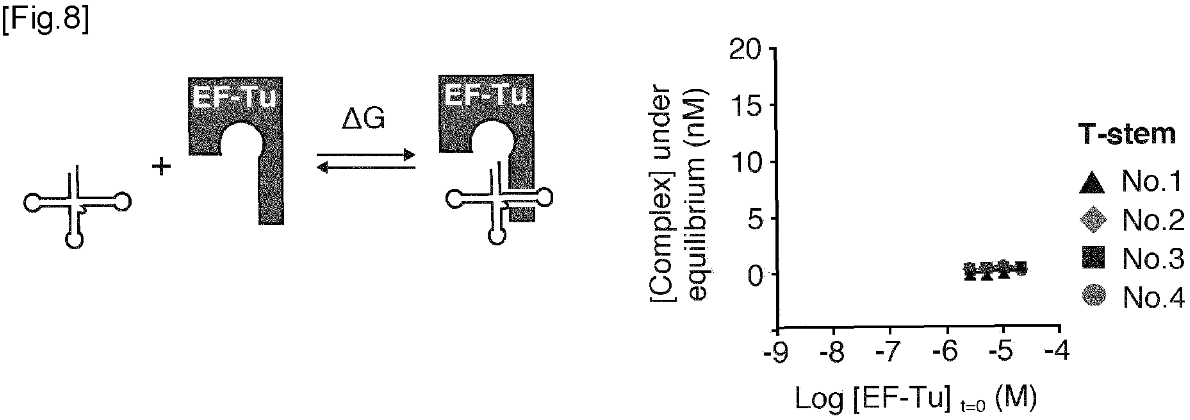
EF-Tu
+
ΔG
EF-Tu
20
15
10
5
0
[Complex] under equilibrium (nM)
-9
-8
-7
-6
-5
-4
Log [EF-Tu] t=0 (M)
T-stem
No.1
No.2
No.3
No.4

[Fig.9]

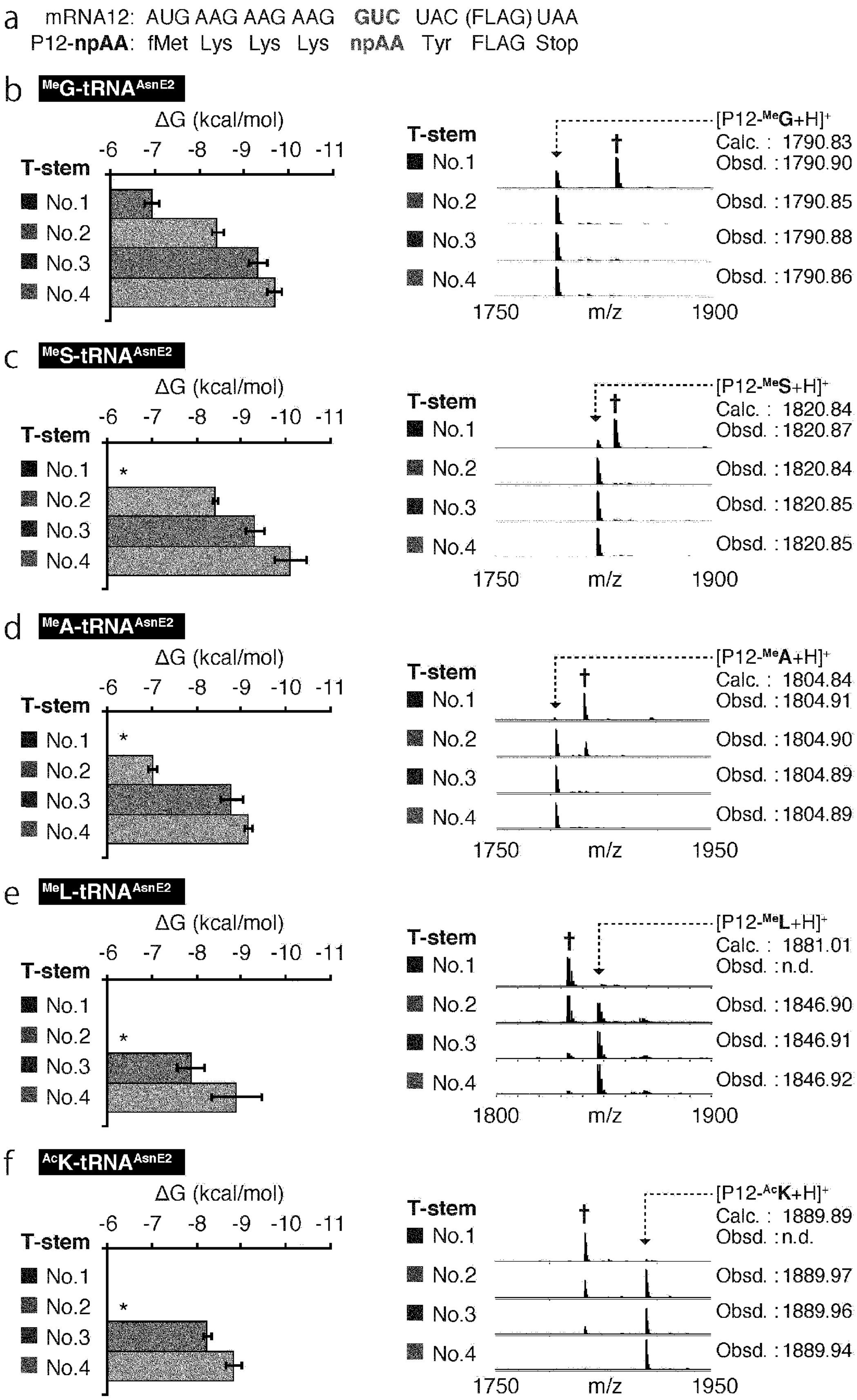
a
mRNA12: AUG AAG AAG AAG GUC UAC (FLAG) UAA
P12-npAA: fMet Lys Lys Lys npAA Tyr FLAG Stop
b MeG-tRNAAsnE2
ΔG (kcal/mol)
-6 -7 -8 -9 -10 -11
T-stem
No.1
No.2
No.3
No.4
[P12-MeG+H]+
Calc. : 1790.83
Obsd. : 1790.90
Obsd. : 1790.85
Obsd. : 1790.88
Obsd. : 1790.86
1750 m/z 1900
c MeS-tRNAAsnE2
[P12-MeS+H]+
Calc. : 1820.84
Obsd. : 1820.87
Obsd. : 1820.84
Obsd. : 1820.85
Obsd. : 1820.85
1750 m/z 1900
d MeA-tRNAAsnE2
[P12-MeA+H]+
Calc. : 1804.84
Obsd. : 1804.91
Obsd. : 1804.90
Obsd. : 1804.89
Obsd. : 1804.89
1750 m/z 1950
e MeL-tRNAAsnE2
[P12-MeL+H]+
Calc. : 1881.01
Obsd. : n.d.
Obsd. : 1846.90
Obsd. : 1846.91
Obsd. : 1846.92
1800 m/z 1900
f AcK-tRNAAsnE2
[P12-AcK+H]+
Calc. : 1889.89
Obsd. : n.d.
Obsd. : 1889.97
Obsd. : 1889.96
Obsd. : 1889.94
1750 m/z 1950

[Fig.10]

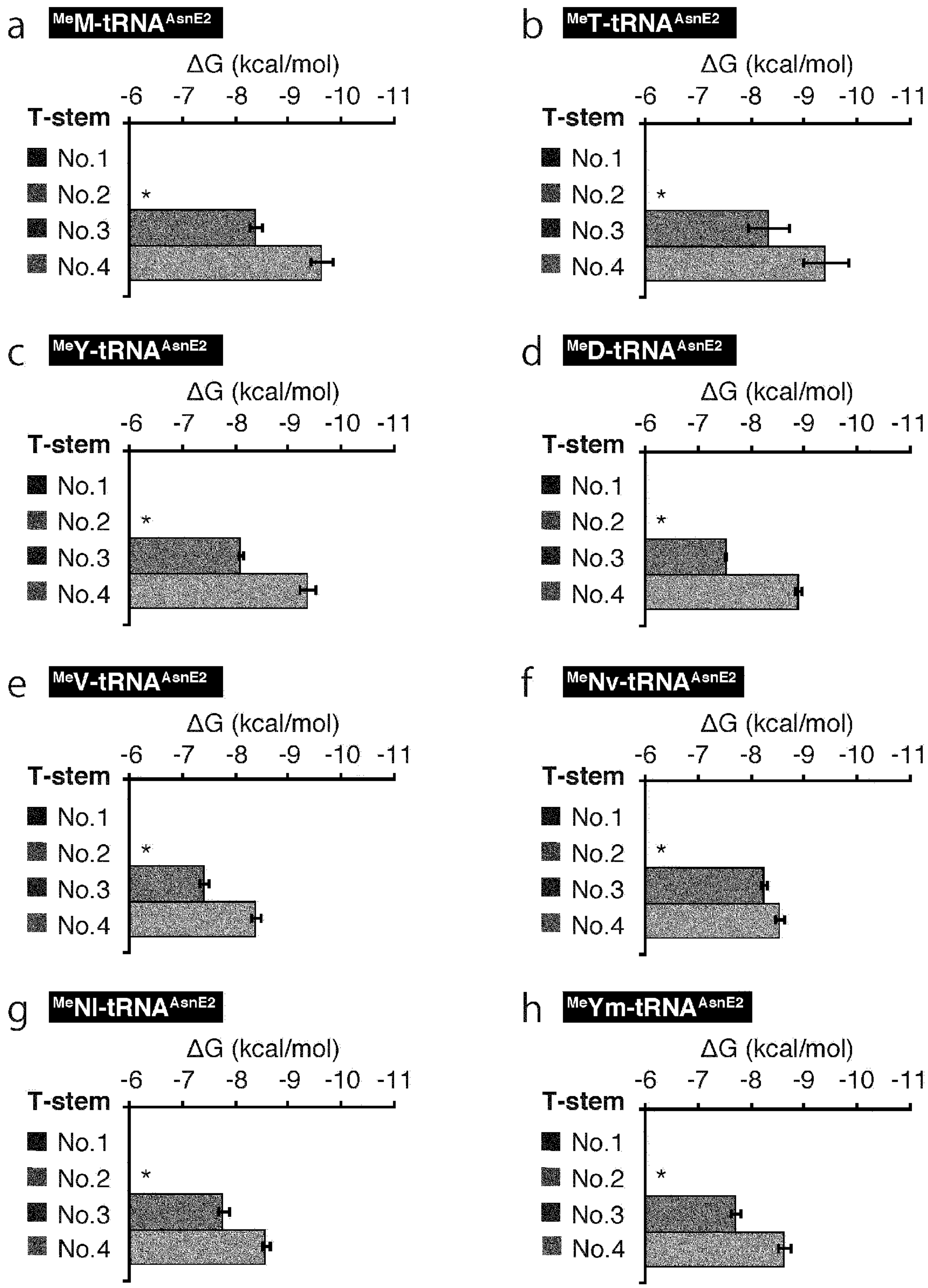
a
MeM-tRNA AsnE2
b
MeT-tRNA AsnE2
c
MeY-tRNA AsnE2
d
MeD-tRNA AsnE2
e
MeV-tRNA AsnE2
f
MeNv-tRNA AsnE2
g
MeNl-tRNA AsnE2
h
MeYm-tRNA AsnE2
ΔG (kcal/mol)
-6 -7 -8 -9 -10 -11
T-stem
No.1
No.2
No.3
No.4
*

## INTERNATIONAL SEARCH REPORT

International application No.
PCT/JP2020/043289

A. CLASSIFICATION OF SUBJECT MATTER
C07K 1/00(2006.01)i; C07K 7/00(2006.01)i; C40B 40/08(2006.01)i; C40B 40/10(2006.01)i; C12M 1/00(2006.01)i; C12N 15/11(2006.01)i; C12P 21/02(2006.01)i
FI: C12N15/11 Z ZNA; C12M1/00 A; C12P21/02 C; C40B40/08; C40B40/10; C07K1/00; C07K7/00

According to International Patent Classification (IPC) or to both national classification and IPC

B. FIELDS SEARCHED

Minimum documentation searched (classification system followed by classification symbols)
C07K1/00; C07K7/00; C40B40/08; C40B40/10; C12M1/00; C12N15/11; C12P21/02

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
CAplus/MEDLINE/EMBASE/BIOSIS/WPIDS (STN), JSTPlus/JMEDPlus/JST7580 (JDreamIII), GenBank/EMBL/DDBJ/GeneSeq, PubMed, Japio-GPG/FX

C. DOCUMENTS CONSIDERED TO BE RELEVANT

| Category* | Citation of document, with indication, where appropriate, of the relevant passages | Relevant to claim No. |
|---|---|---|
| Y | KATOH, T., et al., "Logical engineering of D-arm and T-stem of tRNA that enhances D-amino acid incorporation.", Nucleic Acids Research, 2017, vol. 45, no. 22, pp. 12601-12610 page 12605, right column, paragraph [0003] to page 12606, left column, paragraph [0001], page 12609, left column, paragraph [0002], page 12609, right column, paragraph [0002], fig. 1, 4, 5, abstract | 1-10 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 January 2021 (15.01.2021) | 26 January 2021 (26.01.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2020/043289

C (Continuation). DOCUMENTS CONSIDERED TO BE RELEVANT

| Category* | Citation of document, with indication, where appropriate, of the relevant passages | Relevant to claim No. |
|---|---|---|
| Y | KATOH, T., et al., "Ribosomal Incorporation of Consecutive ß-Amino Acids.", Journal of the American Chemical Society, 2018, vol. 140, pp. 12159-12167 page 12162, left column, paragraph [0003] to page 12163, left column, paragraph [0001], page 12165, right column, paragraph [0003], fig. 2, 4, abstract | 1-10 |
| A | WO 2012/033154 A1 (THE UNIVERSITY OF TOKYO) 15 March 2012 (2012-03-15) entire text, all drawings | 1-10 |

Form PCT/ISA/210 (continuation of second sheet) (January 2015)

**INTERNATIONAL SEARCH REPORT**
Information on patent family members

International application No.
PCT/JP2020/043289

| Patent Documents referred in the Report | Publication Date | Patent Family | Publication Date |
|---|---|---|---|
| WO 2012/033154 A1 | 13 Mar. 2012 | US 2013/0178394 A1 entire text, all drawings<br>EP 2615455 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008117833 A
- WO 2012074129 A


### Non-patent literature cited in the description


- **PASSIOURA, T. ; KATOH, T. ; GOTO, Y. ; SUGA, H.** Selection-based discovery of druglike macrocyclic peptides. *Annu. Rev. Biochem.,* 2014, vol. 83, 727-752
- **DRIGGERS, E. M. ; HALE, S. P. ; LEE, J. ; TERRETT, N. K.** The exploration of macrocycles for drug discovery-an underexploited structural class. *Nat. Rev. Drug Discov.,* 2008, vol. 7, 608-624
- **KOEHN, F. E. ; CARTER, G. T.** The evolving role of natural products in drug discovery. *Nat. Rev. Drug Discov.,* 2005, vol. 4, 206-220
- **HANDSCHUMACHER, R. E. ; HARDING, M. W. ; RICE, J. ; DRUGGE, R. J. ; SPEICHER, D. W.** Cyclophilin: a specific cytosolic binding protein for cyclosporin A. *Science,* 1984, vol. 226, 544-547
- **TAKAHASHI, N. ; HAYANO, T. ; SUZUKI, M.** Peptidyl-Prolyl Cis-Trans Isomerase Is the Cyclosporin-a-Binding Protein Cyclophilin. *Nature,* 1989, vol. 337, 473-475
- **WALSH, C. T. ; ZYDOWSKY, L. D. ; MCKEON, F. D.** Cyclosporin A, the cyclophilin class of peptidylprolyl isomerases, and blockade of T cell signal transduction. *J. Biol. Chem.,* 1992, vol. 267, 13115-13118
- **ALTSCHUH, D. ; VIX, O. ; REES, B. ; THIERRY, J. C.** A conformation of cyclosporin A in aqueous environment revealed by the X-ray structure of a cyclosporin-Fab complex. *Science,* 1992, vol. 256, 92-94
- **CONRADI, R. A. ; HILGERS, A. R. ; HO, N. F. ; BURTON, P. S.** The influence of peptide structure on transport across Caco-2 cells. II. Peptide bond modification which results in improved permeability. *Pharm. Res.,* 1992, vol. 9, 435-439
- **HAVIV, F. et al.** Effect of N-methyl substitution of the peptide bonds in luteinizing hormone-releasing hormone agonists. *J. Med. Chem.,* 1993, vol. 36, 363-369
- **CHIKHALE, E. G. ; NG, K. Y. ; BURTON, P. S. ; BORCHARDT, R. T.** Hydrogen bonding potential as a determinant of the in vitro and in situ blood-brain barrier permeability of peptides. *Pharm. Res.,* 1994, vol. 11, 412-419
- **MILLER, S. M. et al.** Comparison of the Proteolytic Susceptibilities of Homologous L-Amino-Acid, D-Amino-Acid, and N-Substituted Glycine Peptide and Peptoid Oligomers. *Drug Dev. Res.,* 1995, vol. 35, 20-32
- **ROBERTS, R. W. ; SZOSTAK, J. W.** RNA-peptide fusions for the in vitro selection of peptides and proteins. *Proceedings of the National Academy of Sciences of the United States of America,* 1997, vol. 94, 12297-12302
- **MURAKAMI, H. ; OHTA, A. ; ASHIGAI, H. ; SUGA, H.** A highly flexible tRNA acylation method for non-natural polypeptide synthesis. *Nat. Methods,* 2006, vol. 3, 357-359
- **OHTA, A. ; MURAKAMI, H. ; HIGASHIMURA, E. ; SUGA, H.** Synthesis of polyester by means of genetic code reprogramming. *Chem. Biol.,* 2007, vol. 14, 1315-1322
- **GOTO, Y. et al.** Reprogramming the translation initiation for the synthesis of physiologically stable cyclic peptides. *ACS chemical biology,* 2008, vol. 3, 120-129
- **GOTO, Y. ; MURAKAMI, H. ; SUGA, H.** Initiating translation with D-amino acids. *RNA,* 2008, vol. 14, 1390-1398
- **H. F. KUNG et al.** *Journal of Biological Chemistry,* 1977, vol. 252 (19), 6889-6894
- **M. C. GONZA et al.** *Proceeding of National Academy of Sciences of the United States of America,* 1985, vol. 82, 1648-1652
- **M. Y. PAVLOV ; M. EHRENBERG.** *Archives of Biochemistry and Biophysics,* 1996, vol. 328 (1), 9-16
- **Y. SHIMIZU et al.** *Nature Biotechnology,* 2001, vol. 19 (8), 751-755
- **H. OHASHI et al.** *Biochemical and Biophysical Research Communications,* 2007, vol. 352 (1), 270-276
- **KAWAKAMI, T. et al.** *Nature Chemical Biology,* 2009, vol. 5, 888-890
- **YAMAGISHI, Y. et al.** *ChemBioChem,* 2009, vol. 10, 1469-1472
- **SAKO, Y. et al.** *Journal of American Chemical Society,* 2008, vol. 130, 7932-7934

- **GOTO, Y. et al.** *ACS Chemical Biology,* 2008, vol. 3, 120-129
- **KAWAKAMI T. et al.** *Chemistry & Biology,* 2008, vol. 15, 32-42
- **NISSEN, P. et al.** Crystal structure of the ternary complex of Phe-tRNAPhe, EF-Tu, and a GTP analog. *Science,* 1995, vol. 270, 1464-1472
- **GOTO, Y. ; KATOH, T. ; SUGA, H.** Flexizymes for genetic code reprogramming. *Nat Protoc,* 2011, vol. 6, 779-790
- **KAWAKAMI, T. ; MURAKAMI, H. ; SUGA, H.** Messenger RNA-programmed incorporation of multiple N-methyl amino acids into linear and cyclic peptides. *Chem. Biol.,* 2008, vol. 15, 32-42
- **SHIMIZU, Y. et al.** Cell-free translation reconstituted with purified components. *Nat. Biotechnol.,* 2001, vol. 19, 751-755
- **LARIVIERE, F. J. ; WOLFSON, A. D. ; UHLENBECK, O. C.** Uniform binding of aminoacyl-tRNAs to elongation factor Tu by thermodynamic compensation. *Science,* 2001, vol. 294, 165-168
- **YAMAGISHI, Y. et al.** Natural product-like macrocyclic N-methyl-peptide inhibitors against a ubiquitin ligase uncovered from a ribosome-expressed de novo library. *Chem. Biol.,* 2011, vol. 18, 1562-1570
- **SCHRADER, J. M. ; CHAPMAN, S. J. ; UHLENBECK, O. C.** Understanding the sequence specificity of tRNA binding to elongation factor Tu using tRNA mutagenesis. *J. Mol. Biol.,* 2009, vol. 386, 1255-1264
- **SCHRADER, J. M. ; CHAPMAN, S. J. ; UHLENBECK, O. C.** Tuning the affinity of aminoacyl-tRNA to elongation factor Tu for optimal decoding. *Proceedings of the National Academy of Sciences of the United States of America,* 2011, vol. 108, 5215-5220
- **MITTELSTAET, J. ; KONEVEGA, A. L. ; RODNINA, M. V.** A kinetic safety gate controlling the delivery of unnatural amino acids to the ribosome. *J. Am. Chem. Soc.,* 2013, vol. 135, 17031-17038
- **SUBTELNY, A. O. ; HARTMAN, M. C. ; SZOSTAK, J. W.** Ribosomal synthesis of N-methyl peptides. *J. Am. Chem. Soc.,* 2008, vol. 130, 6131-6136
- **OHTSUKI, T. ; MANABE, T. ; SISIDO, M.** Multiple incorporation of non-natural amino acids into a single protein using tRNAs with non-standard structures. *FEBS Lett.,* 2005, vol. 579, 6769-6774
- **IWANE, Y. et al.** Expanding the amino acid repertoire of ribosomal polypeptide synthesis via the artificial division of codon boxes. *Nat Chem,* 2016, vol. 8, 317-325

SEQUENCE LISTING

<110> The University of Tokyo

<120> Logical engineering of T-stem of tRNA that enhances D-amino acid incorporation

<130> T0529AJP0015

<150> JP2019-209114
<151> 2019-11-19

<160> 136

<170> PatentIn version 3.5

<210> 1
<211> 11
<212> RNA
<213> Artificial Sequence

<220>
<223> T-arm of tRNA

<220>
<221> misc_feature
<222> (6)..(6)
<223> n is a, c, g, or u

<400> 1
aggggncccc u 11

<210> 2
<211> 11
<212> RNA
<213> Artificial Sequence

<220>
<223> T-arm of tRNA

<220>
<221> misc_feature
<222> (6)..(6)
<223> n is a, c, g, or u

<400> 2
gcgggncccg u 11

<210> 3
<211> 7
<212> RNA
<213> Artificial Sequence

<220>
<223> T-loop of tRNA

<400> 3
uucgaau 7

<210> 4
<211> 11
<212> RNA
<213> Artificial Sequence

<220>
<223> T-arm of tRNA

<220>
<221> misc_feature
<222> (1)..(2)
<223> n is a, c, g, or u

<220>
<221> misc_feature
<222> (5)..(7)
<223> n is a, c, g, or u

<220>
<221> misc_feature
<222> (10)..(10)
<223> n is a, c, g, or u

<400> 4
nnggnnnccn u 11

<210> 5
<211> 17
<212> RNA
<213> Artificial Sequence

<220>
<223> T-arm of tRNA

<400> 5
aggggvucga auccccu 17

<210> 6
<211> 17
<212> RNA
<213> Artificial Sequence

<220>
<223> T-arm of tRNA

<400> 6
gcgguucga aucccgu 17

<210> 7
<211> 66
<212> DNA
<213> Artificial Sequence

<220>
<223> O-197

<400> 7
taatacgact cactatagcc cggatagctc agtcggtaga gcaggggact gaaaatcccc 60

gtgtcc 66

<210> 8
<211> 42
<212> DNA
<213> Artificial Sequence

<220>
<223> O-198

<400> 8
gtgcccggac tcggaatcga accaaggaca cggggatttt ca 42

<210> 9
<211> 22
<212> DNA
<213> Artificial Sequence

<220>
<223> O-199

<400> 9
ggcgtaatac gactcactat ag 22

<210> 10
<211> 15
<212> DNA
<213> Artificial Sequence

<220>
<223> O-200

<220>
<221> misc_feature
<222> (2)..(2)
<223> n is gm

<400> 10
gntgcccgga ctcgg 15

<210> 11
<211> 16
<212> DNA
<213> Artificial Sequence

<220>
<223> O-201

<220>
<221> misc_feature
<222> (2)..(2)
<223> n is gm

<400> 11
tngtgcccgg actcgg 16

<210> 12
<211> 67
<212> DNA
<213> Artificial Sequence

<220>
<223> O-202

<400> 12
ggcgtaatac gactcactat aggtggggtt cccgagcggc caaagggagc agactgtaaa 60

tctgccg 67

<210> 13
<211> 56
<212> DNA
<213> Artificial Sequence

<220>
<223> O-203

<400> 13
ggtggtgggg gaaggattcg aaccttcgaa gtctgtgacg gcagatttac agtctg 56

<210> 14
<211> 17
<212> DNA
<213> Artificial Sequence

<220>
<223> O-204

<220>
<221> misc_feature
<222> (2)..(2)
<223> n is gm

<400> 14
gntggtgggg gaaggat 17

<210> 15
<211> 18
<212> DNA
<213> Artificial Sequence

<220>
<223> O-205

<220>
<221> misc_feature
<222> (2)..(2)
<223> n is gm

<400> 15
tngtggtggg ggaaggat 18

<210> 16
<211> 67
<212> DNA
<213> Artificial Sequence

<220>
<223> O-206

<400> 16
ggcgtaatac gactcactat aggagagatg ccggagcggc tgaacggacc ggtctcgaaa 60

accggag 67

<210> 17
<211> 60
<212> DNA
<213> Artificial Sequence

<220>
<223> O-207

<400> 17
ggcggagaga gggggatttg aaccccccggt agagttgccc ctactccggt tttcgagacc 60

<210> 18
<211> 16
<212> DNA
<213> Artificial Sequence

<220>
<223> O-208

<220>
<221> misc_feature
<222> (2)..(2)
<223> n is gm

<400> 18
gncggagaga gggggga 16

<210> 19
<211> 17
<212> DNA
<213> Artificial Sequence

<220>
<223> O-209

<220>
<221> misc_feature
<222> (2)..(2)
<223> n is gm

<400> 19
tngcggagag aggggga 17

<210> 20

<211>  48
<212>  DNA
<213>  Artificial Sequence

<220>
<223>  O-210

<400>  20
gtaatacgac tcactatagg ctctgtagtt cagtcggtag aacggcgg                48

<210>  21
<211>  58
<212>  DNA
<213>  Artificial Sequence

<220>
<223>  O-211

<400>  21
gcggctctgc aatgactcga acactggaca tacggattgt cagtccgccg ttctaccg    58

<210>  22
<211>  15
<212>  DNA
<213>  Artificial Sequence

<220>
<223>  O-212

<220>
<221>  misc_feature
<222>  (2)..(2)
<223>  n is gm

<400>  22
gncggctctg caatg                                                    15

<210>  23
<211>  58
<212>  DNA
<213>  Artificial Sequence

<220>
<223>  O-213

<400>  23
gcggctctga ctggactcga accagtgaca tacggattgt cagtccgccg ttctaccg    58

<210>  24
<211>  15
<212>  DNA
<213>  Artificial Sequence

<220>
<223>  O-214

<220>

<221> misc_feature
<222> (2)..(2)
<223> n is gm

<400> 24
gncggctctg actgg 15

<210> 25
<211> 58
<212> DNA
<213> Artificial Sequence

<220>
<223> O-215

<400> 25
gcggctctga ggggactcga acccctgaca tacggattgt cagtccgccg ttctaccg 58

<210> 26
<211> 15
<212> DNA
<213> Artificial Sequence

<220>
<223> O-216

<220>
<221> misc_feature
<222> (2)..(2)
<223> n is gm

<400> 26
gncggctctg agggg 15

<210> 27
<211> 58
<212> DNA
<213> Artificial Sequence

<220>
<223> O-217

<400> 27
gcggctctga cgggactcga acccgcgaca tacggattgt cagtccgccg ttctaccg 58

<210> 28
<211> 15
<212> DNA
<213> Artificial Sequence

<220>
<223> O-218

<220>
<221> misc_feature
<222> (2)..(2)
<223> n is gm

<400> 28
gncggctctg acggg 15

<210> 29
<211> 16
<212> DNA
<213> Artificial Sequence

<220>
<223> O-219

<220>
<221> misc_feature
<222> (2)..(2)
<223> n is gm

<400> 29
tngcggctct gcaatg 16

<210> 30
<211> 16
<212> DNA
<213> Artificial Sequence

<220>
<223> O-220

<220>
<221> misc_feature
<222> (2)..(2)
<223> n is gm

<400> 30
tngcggctct gactgg 16

<210> 31
<211> 16
<212> DNA
<213> Artificial Sequence

<220>
<223> O-221

<220>
<221> misc_feature
<222> (2)..(2)
<223> n is gm

<400> 31
tngcggctct gagggg 16

<210> 32
<211> 16
<212> DNA
<213> Artificial Sequence

<220>
<223> O-222

<220>
<221> misc_feature
<222> (2)..(2)
<223> n is gm

<400> 32
tngcggctct gacggg 16

<210> 33
<211> 48
<212> DNA
<213> Artificial Sequence

<220>
<223> O-223

<400> 33
gtaatacgac tcactatagg ctctgtagtt cagtcggaag aacggcgg 48

<210> 34
<211> 58
<212> DNA
<213> Artificial Sequence

<220>
<223> O-224

<400> 34
gcggctctgc aatgactcga acactggaca tacggatttt cagtccgccg ttcttccg 58

<210> 35
<211> 58
<212> DNA
<213> Artificial Sequence

<220>
<223> O-225

<400> 35
gcggctctga ctggactcga accagtgaca tacggatttt cagtccgccg ttcttccg 58

<210> 36
<211> 58
<212> DNA
<213> Artificial Sequence

<220>
<223> O-226

<400> 36
gcggctctga ggggactcga acccctgaca tacggatttt cagtccgccg ttcttccg 58

<210> 37

<211> 58
<212> DNA
<213> Artificial Sequence

<220>
<223> O-227

<400> 37
gcggctctga cgggactcga acccgcgaca tacggatttt cagtccgccg ttctaccg 58

<210> 38
<211> 58
<212> DNA
<213> Artificial Sequence

<220>
<223> O-228

<400> 38
gcggctctgc aatgactcga acactggaca tacggatttt gagtccgccg ttctaccg 58

<210> 39
<211> 58
<212> DNA
<213> Artificial Sequence

<220>
<223> O-229

<400> 39
gcggctctga ctggactcga accagtgaca tacggatttt gagtccgccg ttctaccg 58

<210> 40
<211> 58
<212> DNA
<213> Artificial Sequence

<220>
<223> O-230

<400> 40
gcggctctga ggggactcga acccctgaca tacggatttt gagtccgccg ttctaccg 58

<210> 41
<211> 58
<212> DNA
<213> Artificial Sequence

<220>
<223> O-231

<400> 41
gcggctctga cgggactcga acccgcgaca tacggatttt gagtccgccg ttctaccg 58

<210> 42
<211> 58
<212> DNA
<213> Artificial Sequence

<220>
<223>  O-232

<400>  42
gcggctctgc aatgactcga acactggaca tacggaacct caatccgccg ttctaccg          58

<210>  43
<211>  58
<212>  DNA
<213>  Artificial Sequence

<220>
<223>  O-233

<400>  43
gcggctctga ctggactcga accagtgaca tacggaacct caatccgccg ttctaccg          58

<210>  44
<211>  58
<212>  DNA
<213>  Artificial Sequence

<220>
<223>  O-234

<400>  44
gcggctctga ggggactcga acccctgaca tacggaacct caatccgccg ttctaccg          58

<210>  45
<211>  58
<212>  DNA
<213>  Artificial Sequence

<220>
<223>  O-235

<400>  45
gcggctctga cgggactcga acccgcgaca tacggaacct caatccgccg ttctaccg          58

<210>  46
<211>  58
<212>  DNA
<213>  Artificial Sequence

<220>
<223>  O-236

<400>  46
gcggctctgc aatgactcga acactggaca tacggaacct gaatccgccg ttctaccg          58

<210>  47
<211>  58
<212>  DNA
<213>  Artificial Sequence

<220>
<223>  O-237

<400> 47
gcggctctga ctggactcga accagtgaca tacggaacct gaatccgccg ttctaccg 58

<210> 48
<211> 58
<212> DNA
<213> Artificial Sequence

<220>
<223> O-238

<400> 48
gcggctctga ggggactcga acccctgaca tacggaacct gaatccgccg ttctaccg 58

<210> 49
<211> 58
<212> DNA
<213> Artificial Sequence

<220>
<223> O-239

<400> 49
gcggctctga cgggactcga acccgcgaca tacggaacct gaatccgccg ttctaccg 58

<210> 50
<211> 58
<212> DNA
<213> Artificial Sequence

<220>
<223> O-240

<400> 50
gcggctctgc aatgactcga acactggaca tacggattat cagtccgccg ttctaccg 58

<210> 51
<211> 58
<212> DNA
<213> Artificial Sequence

<220>
<223> O-241

<400> 51
gcggctctga ctggactcga accagtgaca tacggattat cagtccgccg ttctaccg 58

<210> 52
<211> 58
<212> DNA
<213> Artificial Sequence

<220>
<223> O-242

<400> 52
gcggctctga ggggactcga acccctgaca tacggattat cagtccgccg ttctaccg 58

<210> 53
<211> 58
<212> DNA
<213> Artificial Sequence

<220>
<223> O-243

<400> 53
gcggctctga cgggactcga acccgcgaca tacggattat cagtccgccg ttctaccg 58

<210> 54
<211> 58
<212> DNA
<213> Artificial Sequence

<220>
<223> O-244

<400> 54
gcggctctgc aatgactcga acactggaca tacggagtgt gagtccgccg ttctaccg 58

<210> 55
<211> 58
<212> DNA
<213> Artificial Sequence

<220>
<223> O-245

<400> 55
gcggctctga ctggactcga accagtgaca tacggagtgt gagtccgccg ttctaccg 58

<210> 56
<211> 58
<212> DNA
<213> Artificial Sequence

<220>
<223> O-246

<400> 56
gcggctctga ggggactcga acccctgaca tacggagtgt gagtccgccg ttctaccg 58

<210> 57
<211> 58
<212> DNA
<213> Artificial Sequence

<220>
<223> O-247

<400> 57
gcggctctga cgggactcga acccgcgaca tacggagtgt gagtccgccg ttctaccg 58

<210> 58

<211> 58
<212> DNA
<213> Artificial Sequence

<220>
<223> O-248

<400> 58
gcggctctgc aatgactcga acactggaca tacggactgc cagtccgccg ttctaccg 58

<210> 59
<211> 58
<212> DNA
<213> Artificial Sequence

<220>
<223> O-249

<400> 59
gcggctctga ctggactcga accagtgaca tacggactgc cagtccgccg ttctaccg 58

<210> 60
<211> 58
<212> DNA
<213> Artificial Sequence

<220>
<223> O-250

<400> 60
gcggctctga ggggactcga acccctgaca tacggactgc cagtccgccg ttctaccg 58

<210> 61
<211> 58
<212> DNA
<213> Artificial Sequence

<220>
<223> O-251

<400> 61
gcggctctga cgggactcga acccgcgaca tacggactgc cagtccgccg ttctaccg 58

<210> 62
<211> 58
<212> DNA
<213> Artificial Sequence

<220>
<223> O-252

<400> 62
gcggctctgc aatgactcga acactggaca tacggattgc gaatccgccg ttctaccg 58

<210> 63
<211> 58
<212> DNA
<213> Artificial Sequence

<220>
<223>  O-253

<400>  63
gcggctctga ctggactcga accagtgaca tacggattgc gaatccgccg ttctaccg          58

<210>  64
<211>  58
<212>  DNA
<213>  Artificial Sequence

<220>
<223>  O-254

<400>  64
gcggctctga ggggactcga acccctgaca tacggattgc gaatccgccg ttctaccg          58

<210>  65
<211>  58
<212>  DNA
<213>  Artificial Sequence

<220>
<223>  O-255

<400>  65
gcggctctga cgggactcga acccgcgaca tacggattgc gaatccgccg ttctaccg          58

<210>  66
<211>  58
<212>  DNA
<213>  Artificial Sequence

<220>
<223>  O-256

<400>  66
gcggctctgc aatgactcga acaccggaca tacggattgt cagtccgccg ttctaccg          58

<210>  67
<211>  58
<212>  DNA
<213>  Artificial Sequence

<220>
<223>  O-257

<400>  67
gcggctctgc aatgactcga acaccggaca tacggattta gagtccgccg ttctaccg          58

<210>  68
<211>  55
<212>  DNA
<213>  Artificial Sequence

<220>
<223>  O-258

<400> 68
taatacgact cactataggg ttaactttaa caaggagaaa aacatgaaga agaag 55

<210> 69
<211> 60
<212> DNA
<213> Artificial Sequence

<220>
<223> O-259

<400> 69
cgaagcttac ttgtcgtcgt cgtccttgta gtcgtagacc ttcttcttca tgtttttctc 60

<210> 70
<211> 18
<212> DNA
<213> Artificial Sequence

<220>
<223> O-260

<400> 70
cgaagcttac ttgtcgtc 18

<210> 71
<211> 53
<212> DNA
<213> Artificial Sequence

<220>
<223> O-261

<400> 71
gagaaaaaca tgaagaagaa gacaaatttc ggtatccgtg cctctgtcat gga 53

<210> 72
<211> 54
<212> DNA
<213> Artificial Sequence

<220>
<223> O-262

<400> 72
gtcgtccttg tagtcgtatg gatggagcag ttgccattcc atgacagagg cacg 54

<210> 73
<211> 33
<212> DNA
<213> Artificial Sequence

<220>
<223> O-263

<400> 73
cgaagcttac ttgtcgtcgt cgtccttgta gtc 33

<210> 74
<211> 59
<212> DNA
<213> Artificial Sequence

<220>
<223> O-264

<400> 74
gagaaaaaca tgaagaagaa gctccatatg ttggtcacag aactgtctat cgcgcaaaa 59

<210> 75
<211> 55
<212> DNA
<213> Artificial Sequence

<220>
<223> O-265

<400> 75
gtcgtccttg tagtctgggg cacgcaccca accgaaattt tgcgcgatag acagt 55

<210> 76
<211> 70
<212> DNA
<213> Artificial Sequence

<220>
<223> O-266

<400> 76
taatacgact cactataggg ttaactttaa caaggagaaa aacatggact acaaggacga 60

cgacgacaag 70

<210> 77
<211> 53
<212> DNA
<213> Artificial Sequence

<220>
<223> O-267

<400> 77
cgaagcttac ttcttcttga tgaaggcgac caggagcttg tcgtcgtcgt cct 53

<210> 78
<211> 18
<212> DNA
<213> Artificial Sequence

<220>
<223> O-268

<400> 78
cgaagcttac ttcttctt 18

<210> 79
<211> 75
<212> RNA
<213> Artificial Sequence

<220>
<223> Phe without 3'-A

<400> 79
gcccggauag cucagucggu agagcagggg acugaaaauc cccguguccu ugguucgauu 60

ccgaguccgg gcacc 75

<210> 80
<211> 84
<212> RNA
<213> Artificial Sequence

<220>
<223> Tyr without 3'-A

<400> 80
ggugggguuc ccgagcggcc aaagggagca gacuguaaau cugccgucac agacuucgaa 60

gguucgaauc cuucccccac cacc 84

<210> 81
<211> 89
<212> RNA
<213> Artificial Sequence

<220>
<223> Ser without 3'-A

<400> 81
ggagagaugc cggagcggcu gaacggaccg gucucgaaaa ccggaguagg ggcaacucua 60

ccgggggguuc aaauccccccu cucuccgcc 89

<210> 82
<211> 75
<212> RNA
<213> Artificial Sequence

<220>
<223> AsnE2 without 3'-A

<400> 82
ggcucuguag uucagucggu agaacggcgg auugacauuc cguaugucca guguucgagu 60

cauugcagag ccgcc 75

<210> 83
<211> 75
<212> RNA
<213> Artificial Sequence

<220>

<223>  AsnE2 without 3′-A

<400>  83
ggcucuguag uucagucggu agaacggcgg auugacauuc cguaugucac ugguucgagu    60

ccagucagag ccgcc    75

<210>  84
<211>  75
<212>  RNA
<213>  Artificial Sequence

<220>
<223>  AsnE2 without 3′-A

<400>  84
ggcucuguag uucagucggu agaacggcgg auugacauuc cguaugucag ggguucgagu    60

ccccucagag ccgcc    75

<210>  85
<211>  75
<212>  RNA
<213>  Artificial Sequence

<220>
<223>  AsnE2 without 3′-A

<400>  85
ggcucuguag uucagucggu agaacggcgg auugacauuc cguaugucgc ggguucgagu    60

cccgucagag ccgcc    75

<210>  86
<211>  76
<212>  RNA
<213>  Artificial Sequence

<220>
<223>  AsnE2

<400>  86
ggcucuguag uucagucggu agaacggcgg auugacauuc cguaugucca guguucgagu    60

cauugcagag ccgcca    76

<210>  87
<211>  76
<212>  RNA
<213>  Artificial Sequence

<220>
<223>  AsnE2

<400>  87
ggcucuguag uucagucggu agaacggcgg auugacauuc cguaugucac ugguucgagu    60

ccagucagag ccgcca    76

<210> 88
<211> 76
<212> RNA
<213> Artificial Sequence

<220>
<223> AsnE2

<400> 88
ggcucuguag uucagucggu agaacggcgg auugacauuc cguaugucag ggguucgagu 60

ccccucagag ccgcca 76

<210> 89
<211> 76
<212> RNA
<213> Artificial Sequence

<220>
<223> AsnE2

<400> 89
ggcucuguag uucagucggu agaacggcgg auugacauuc cguaugucgc ggguucgagu 60

cccgucagag ccgcca 76

<210> 90
<211> 76
<212> RNA
<213> Artificial Sequence

<220>
<223> AsnE2

<400> 90
ggcucuguag uucagucgga agaacggcgg acugaaaauc cguaugucca guguucgagu 60

cauugcagag ccgcca 76

<210> 91
<211> 76
<212> RNA
<213> Artificial Sequence

<220>
<223> AsnE2

<400> 91
ggcucuguag uucagucgga agaacggcgg acugaaaauc cguaugucac ugguucgagu 60

ccagucagag ccgcca 76

<210> 92
<211> 76
<212> RNA
<213> Artificial Sequence

<220>

<223> AsnE2

<400> 92
ggcucuguag uucagucgga agaacggcgg acugaaaauc cguaugucag ggguucgagu 60

ccccucagag ccgcca 76

<210> 93
<211> 76
<212> RNA
<213> Artificial Sequence

<220>
<223> AsnE2

<400> 93
ggcucuguag uucagucgga agaacggcgg acugaaaauc cguaugucgc ggguucgagu 60

cccgucagag ccgcca 76

<210> 94
<211> 76
<212> RNA
<213> Artificial Sequence

<220>
<223> AsnE2

<400> 94
ggcucuguag uucagucggu agaacggcgg acucaaaauc cguaugucca guguucgagu 60

cauugcagag ccgcca 76

<210> 95
<211> 76
<212> RNA
<213> Artificial Sequence

<220>
<223> AsnE2

<400> 95
ggcucuguag uucagucggu agaacggcgg acucaaaauc cguaugucac ugguucgagu 60

ccagucagag ccgcca 76

<210> 96
<211> 76
<212> RNA
<213> Artificial Sequence

<220>
<223> AsnE2

<400> 96
ggcucuguag uucagucggu agaacggcgg acucaaaauc cguaugucag ggguucgagu 60

ccccucagag ccgcca 76

<210> 97
<211> 76
<212> RNA
<213> Artificial Sequence

<220>
<223> AsnE2

<400> 97
ggcucuguag uucagucggu agaacggcgg acucaaaauc cguaugucgc ggguucgagu 60

cccgucagag ccgcca 76

<210> 98
<211> 76
<212> RNA
<213> Artificial Sequence

<220>
<223> AsnE2

<400> 98
ggcucuguag uucagucggu agaacggcgg auugagguuc cguaugucca guguucgagu 60

cauugcagag ccgcca 76

<210> 99
<211> 76
<212> RNA
<213> Artificial Sequence

<220>
<223> AsnE2

<400> 99
ggcucuguag uucagucggu agaacggcgg auugagguuc cguaugucac ugguucgagu 60

ccagucagag ccgcca 76

<210> 100
<211> 76
<212> RNA
<213> Artificial Sequence

<220>
<223> AsnE2

<400> 100
ggcucuguag uucagucggu agaacggcgg auugagguuc cguaugucag ggguucgagu 60

ccccucagag ccgcca 76

<210> 101
<211> 76
<212> RNA
<213> Artificial Sequence

<220>

<223> AsnE2

<400> 101
ggcucuguag uucagucggu agaacggcgg auugagguuc cguaugucgc ggguucgagu 60

cccgucagag ccgcca 76

<210> 102
<211> 76
<212> RNA
<213> Artificial Sequence

<220>
<223> AsnE2

<400> 102
ggcucuguag uucagucggu agaacggcgg auucagguuc cguaugucca guguucgagu 60

cauugcagag ccgcca 76

<210> 103
<211> 76
<212> RNA
<213> Artificial Sequence

<220>
<223> AsnE2

<400> 103
ggcucuguag uucagucggu agaacggcgg auucagguuc cguaugucac ugguucgagu 60

ccagucagag ccgcca 76

<210> 104
<211> 76
<212> RNA
<213> Artificial Sequence

<220>
<223> AsnE2

<400> 104
ggcucuguag uucagucggu agaacggcgg auucagguuc cguaugucag ggguucgagu 60

ccccucagag ccgcca 76

<210> 105
<211> 76
<212> RNA
<213> Artificial Sequence

<220>
<223> AsnE2

<400> 105
ggcucuguag uucagucggu agaacggcgg auucagguuc cguaugucgc ggguucgagu 60

cccgucagag ccgcca 76

<210> 106
<211> 76
<212> RNA
<213> Artificial Sequence

<220>
<223> AsnE2

<400> 106
ggcucuguag uucagucggu agaacggcgg acucauaauc cguaugucca guguucgagu 60

cauugcagag ccgcca 76

<210> 107
<211> 76
<212> RNA
<213> Artificial Sequence

<220>
<223> AsnE2

<400> 107
ggcucuguag uucagucggu agaacggcgg acucauaauc cguaugucac ugguucgagu 60

ccagucagag ccgcca 76

<210> 108
<211> 76
<212> RNA
<213> Artificial Sequence

<220>
<223> AsnE2

<400> 108
ggcucuguag uucagucggu agaacggcgg acucauaauc cguaugucag ggguucgagu 60

ccccucagag ccgcca 76

<210> 109
<211> 76
<212> RNA
<213> Artificial Sequence

<220>
<223> AsnE2

<400> 109
ggcucuguag uucagucggu agaacggcgg acucauaauc cguaugucgc ggguucgagu 60

cccgucagag ccgcca 76

<210> 110
<211> 76
<212> RNA
<213> Artificial Sequence

<220>

<223> AsnE2

<400> 110
ggcucuguag uucagucggu agaacggcgg acucacacuc cguaugucca guguucgagu 60

cauugcagag ccgcca 76

<210> 111
<211> 76
<212> RNA
<213> Artificial Sequence

<220>
<223> AsnE2

<400> 111
ggcucuguag uucagucggu agaacggcgg acucacacuc cguaugucac ugguucgagu 60

ccagucagag ccgcca 76

<210> 112
<211> 76
<212> RNA
<213> Artificial Sequence

<220>
<223> AsnE2

<400> 112
ggcucuguag uucagucggu agaacggcgg acucacacuc cguaugucag ggguucgagu 60

ccccucagag ccgcca 76

<210> 113
<211> 76
<212> RNA
<213> Artificial Sequence

<220>
<223> AsnE2

<400> 113
ggcucuguag uucagucggu agaacggcgg acucacacuc cguaugucgc ggguucgagu 60

cccgucagag ccgcca 76

<210> 114
<211> 76
<212> RNA
<213> Artificial Sequence

<220>
<223> AsnE2

<400> 114
ggcucuguag uucagucggu agaacggcgg acuggcaguc cguaugucca guguucgagu 60

cauugcagag ccgcca 76

<210> 115
<211> 76
<212> RNA
<213> Artificial Sequence

<220>
<223> AsnE2

<400> 115
ggcucuguag uucagucggu agaacggcgg acuggcaguc cguaugucac ugguucgagu 60

ccagucagag ccgcca 76

<210> 116
<211> 76
<212> RNA
<213> Artificial Sequence

<220>
<223> AsnE2

<400> 116
ggcucuguag uucagucggu agaacggcgg acuggcaguc cguaugucag ggguucgagu 60

ccccucagag ccgcca 76

<210> 117
<211> 76
<212> RNA
<213> Artificial Sequence

<220>
<223> AsnE2

<400> 117
ggcucuguag uucagucggu agaacggcgg acuggcaguc cguaugucgc ggguucgagu 60

cccgucagag ccgcca 76

<210> 118
<211> 76
<212> RNA
<213> Artificial Sequence

<220>
<223> AsnE2

<400> 118
ggcucuguag uucagucggu agaacggcgg auucgcaauc cguaugucca guguucgagu 60

cauugcagag ccgcca 76

<210> 119
<211> 76
<212> RNA
<213> Artificial Sequence

<220>

<223> AsnE2

<400> 119
ggcucuguag uucagucggu agaacggcgg auucgcaauc cguaugucac ugguucgagu 60

ccagucagag ccgcca 76

<210> 120
<211> 76
<212> RNA
<213> Artificial Sequence

<220>
<223> AsnE2

<400> 120
ggcucuguag uucagucggu agaacggcgg auucgcaauc cguaugucag ggguucgagu 60

ccccucagag ccgcca 76

<210> 121
<211> 76
<212> RNA
<213> Artificial Sequence

<220>
<223> AsnE2

<400> 121
ggcucuguag uucagucggu agaacggcgg auucgcaauc cguaugucgc ggguucgagu 60

cccgucagag ccgcca 76

<210> 122
<211> 76
<212> RNA
<213> Artificial Sequence

<220>
<223> AsnE2

<400> 122
ggcucuguag uucagucggu agaacggcgg auugacauuc cguauguccg guguucgagu 60

cauugcagag ccgcca 76

<210> 123
<211> 73
<212> RNA
<213> Artificial Sequence

<220>
<223> mRNA12

<400> 123
gggcuuuaau aaggagaaaa acaugaagaa gaaggucuac gacuacaagg acgacgacga 60

caaguaagcu ucg 73

<210> 124
<211> 121
<212> RNA
<213> Artificial Sequence

<220>
<223> mRNA13

<400> 124
gggcuuuaau aaggagaaaa acaugaagaa gaagacaaau uucgguaucc gugccucugu 60

cauggaaugg caacugcucc auccauacga cuacaaggac gacgacgaca aguaagcuuc 120

g 121

<210> 125
<211> 127
<212> RNA
<213> Artificial Sequence

<220>
<223> mRNA14

<400> 125
gggcuuuaau aaggagaaaa acaugaagaa gaagcuccau auguugguca cagaacuguc 60

uaucgcgcaa aauuucgguu gggugcgugc cccagacuac aaggacgacg acgacaagua 120

agcuucg 127

<210> 126
<211> 45
<212> RNA
<213> Artificial Sequence

<220>
<223> mRNA12 in Fig.2d, Fig.4a, 4b, Fig.7 and Fig.9

<400> 126
augaagaaga aggucuacga cuacaaggac gacgacgaca aguaa 45

<210> 127
<211> 93
<212> RNA
<213> Artificial Sequence

<220>
<223> mRNA13 in Fig.5c

<400> 127
augaagaaga agacaaauuu cgguauccgu gccucuguca uggaauggca acugcuccau 60

ccauacgacu acaaggacga cgacgacaag uaa 93

<210> 128
<211> 99
<212> RNA
<213> Artificial Sequence

<220>
<223> mRNA14 in Fig.6a

<400> 128
augaagaaga agcuccauau guuggucaca gaacugucua ucgcgcaaaa uuucgguugg 60

gugcgugccc cagacuacaa ggacgacgac gacaaguaa 99

<210> 129
<211> 14
<212> PRT
<213> Artificial Sequence

<220>
<223> P12-npAA

<220>
<221> MISC_FEATURE
<222> (1)..(1)
<223> N-Formylmethionine

<220>
<221> MISC_FEATURE
<222> (5)..(5)
<223> nonproteinogenic amino acid

<400> 129

Met Lys Lys Lys Xaa Tyr Asp Tyr Lys Asp Asp Asp Asp Lys
1 5 10

<210> 130
<211> 14
<212> PRT
<213> Artificial Sequence

<220>
<223> P12-Phe

<220>
<221> MISC_FEATURE
<222> (1)..(1)
<223> N-Formylmethionine

<400> 130

Met Lys Lys Lys Phe Tyr Asp Tyr Lys Asp Asp Asp Asp Lys
1 5 10

<210> 131
<211> 14
<212> PRT
<213> Artificial Sequence

<220>
<223> P12-MeF

<220>
<221> MISC_FEATURE
<222> (1)..(1)
<223> N-Formylmethionine

<220>
<221> MISC_FEATURE
<222> (5)..(5)
<223> MeF

<400> 131

Met Lys Lys Lys Xaa Tyr Asp Tyr Lys Asp Asp Asp Asp Lys
1               5                   10

<210> 132
<211> 30
<212> PRT
<213> Artificial Sequence

<220>
<223> P13

<220>
<221> MISC_FEATURE
<222> (1)..(1)
<223> N-Formylmethionine

<220>
<221> MISC_FEATURE
<222> (7)..(7)
<223> MeY

<220>
<221> MISC_FEATURE
<222> (9)..(9)
<223> MeA

<220>
<221> MISC_FEATURE
<222> (11)..(11)
<223> MeF

<220>
<221> MISC_FEATURE
<222> (13)..(13)
<223> MeV

<220>
<221> MISC_FEATURE
<222> (18)..(18)
<223> MeG

<220>
<221> MISC_FEATURE
<222> (19)..(19)
<223> MeS

<400> 132

Met Lys Lys Lys Thr Asn Xaa Gly Xaa Arg Xaa Ser Xaa Met Glu Trp
1               5                   10                  15

Gln Xaa Xaa His Pro Tyr Asp Tyr Lys Asp Asp Asp Asp Lys
            20                  25                  30

<210>  133
<211>  32
<212>  PRT
<213>  Artificial Sequence

<220>
<223>  P14

<220>
<221>  MISC_FEATURE
<222>  (1)..(1)
<223>  N-Formylmethionine

<220>
<221>  MISC_FEATURE
<222>  (5)..(5)
<223>  MeS

<220>
<221>  MISC_FEATURE
<222>  (8)..(8)
<223>  MeG

<220>
<221>  MISC_FEATURE
<222>  (9)..(9)
<223>  MeV

<220>
<221>  MISC_FEATURE
<222>  (12)..(12)
<223>  MeNl

<220>
<221>  MISC_FEATURE
<222>  (14)..(14)
<223>  MeY

<220>
<221>  MISC_FEATURE
<222>  (15)..(15)
<223>  MeA

<220>
<221>  MISC_FEATURE
<222>  (18)..(18)
<223>  MeYm

<220>
<221>  MISC_FEATURE
<222>  (21)..(21)
<223>  MeNv

<220>

<221> MISC_FEATURE
<222> (23)..(23)
<223> MeF

<400> 133

Met Lys Lys Lys Xaa His Met Xaa Xaa Thr Glu Xaa Ser Xaa Xaa Gln
1 5 10 15

Asn Xaa Gly Trp Xaa Arg Xaa Pro Asp Tyr Lys Asp Asp Asp Asp Lys
20 25 30

<210> 134
<211> 17
<212> RNA
<213> Artificial Sequence

<220>
<223> D-arm of tRNA

<220>
<221> misc_feature
<222> (1)..(2)
<223> n is a, c, g, or u

<220>
<221> misc_feature
<222> (5)..(13)
<223> n is a, c, g, or u

<220>
<221> misc_feature
<222> (16)..(17)
<223> n is a, c, g, or u

<400> 134
nngcnnnnnn nnngcnn 17

<210> 135
<211> 21
<212> RNA
<213> Artificial Sequence

<220>
<223> D-arm of tRNA

<220>
<221> misc_feature
<222> (1)..(2)
<223> n is a, c, g, or u

<220>
<221> misc_feature
<222> (20)..(21)
<223> n is a, c, g, or u

<400> 135
nngcgcagcc ugguagcgcn n 21

<210> 136
<211> 21
<212> RNA
<213> Artificial Sequence

<220>
<223> D-arm of tRNA

<400> 136
gcgcgcagcc ugguagcgcg c 21